(19) Europäisches Patentamt
European Patent Office
Office européen des brevets

(11) EP 4 678 627 A1

(12) EUROPEAN PATENT APPLICATION

(43) Date of publication:
**14.01.2026 Bulletin 2026/03**

(21) Application number: **24187620.0**

(22) Date of filing: **10.07.2024**

(51) International Patent Classification (IPC):
***C07C 211/02*** *(2006.01)* ***C07C 211/03*** *(2006.01)*
***C07D 295/125*** *(2006.01)* ***C07D 295/135*** *(2006.01)*
***A61P 9/10*** *(2006.01)* ***A61P 11/00*** *(2006.01)*
***A61K 31/495*** *(2006.01)*

(52) Cooperative Patent Classification (CPC):
**A61P 3/10; A61K 31/495; A61P 9/10; A61P 11/00; A61P 25/00; C07C 237/34; C07D 295/13; C07D 295/135;** C07C 2601/08; C07C 2601/16

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universiteit Antwerpen**
**2000 Antwerpen (BE)**

(72) Inventors:
- **Augustyns, Koen**
**2020 Antwerpen (BE)**
- **Van der Veken, Pieter**
**2020 Antwerpen (BE)**
- **Vanden Berghe, Tom**
**2020 Antwerpen (BE)**
- **Hassannia, Behrouz**
**2020 Antwerpen (BE)**
- **Lanthier, Caroline**
**2020 Antwerpen (BE)**
- **Scarpellini, Camilla**
**2020 Antwerpen (BE)**
- **Klejborowska, Greta**
**2020 Antwerpen (BE)**

(74) Representative: **De Clercq & Partners**
**Edgard Gevaertdreef 10a**
**9830 Sint-Martens-Latem (BE)**

(54) **NOVEL FERROPTOSIS INHIBITORS**

(57) The present invention relates to a compound of formula (I) or a stereoisomer, or tautomer,

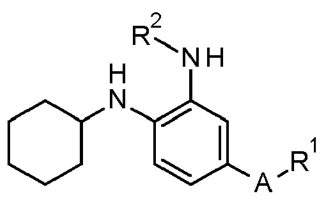

(I)

wherein moiety A, $R^1$, and $R^2$ have the same meaning as that defined in the claims and the description. The present invention also relates to the use of such compounds for the prevention and/or treatment of a disease associated with ferroptosis and/or oxytosis such as liver disease, chronic kidney disease, lung disease, ocular surface diseases, wound healing, multiple organ dysfunction syndrome, neurological disease, acute renal failure, ischemia-reperfusion injury, sepsis, prevention of transplant rejection, iron toxicity, iron metabolism-related disease and genetic disorder of GPX4. The present invention also provides pharmaceutical compositions comprising such compounds, as well as the use of the compounds as a medicament and methods of prevention and/or treatment of such diseases.

EP 4 678 627 A1

## Description

### Field of the invention

The present invention relates to ferrostatin-1 derivative compounds, or pharmaceutically acceptable salts thereof, as defined herein, that are useful for inhibiting undesired cell death occurring in diseases associated with ferroptosis and/or oxytosis such as liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease, and genetic disorders of GPX4. The invention also provides compositions containing a pharmaceutically acceptable carrier and one or more compounds from the invention methods of prevention and/or treatment of such diseases.

### Background of the invention

Cell death is crucial for normal development, homeostasis, and the prevention of hyperproliferative diseases such as cancer. It was once thought that almost all regulated cell death in mammalian cells resulted from the activation of caspase-dependent apoptosis. This view has been challenged by the discovery of several regulated non-apoptotic cell death pathways activated in specific disease states. Regulated necrosis is defined as a genetically controlled cell death process that eventually results in cellular leakage, and is morphologically characterized by cytoplasmic granulation, as well as organelle and/or cellular swelling. Ferroptosis is one recognized form of regulated necrosis and its hallmark is the production of iron-dependent lipophilic reactive oxygen species (ROS). Cell membrane rupture during ferroptotic cell death is characterized by hydrogen abstraction and oxygenation of poly unsaturated fatty acids (PUFAs) of phospholipids (PLs), which is catalysed by redox-active iron. This subsequently leads to cell death due to disruption of membrane stability and the accumulation of lipid hydroperoxides to lethal levels. Although the process of lipid peroxidation has been linked to several regulated cell death modalities, ferroptosis is exclusively driven by excessive lipid peroxidation. Oxidative damage of PUFA-PLs can be initiated either through non-enzymatic free-radical chain reactions involving Fenton chemistry or enzyme-mediated processes catalysed by iron-dependent lipoxygenases (LOXs) or cytochrome P450 oxidoreductase (POR). In addition to ferroptosis, glutamine- and oxidative stress induced cell death are inhibited by iron chelation. In line with this, iron-dependent neuronal cell death is blocked by metal protein-attenuating compounds (e.g. clioquinol) and iron chelators (e.g. deferoxamine), which are being explored for the treatment of neurodegenerative diseases. Another type of regulated necrosis is oxytosis which is also induced when the $Xc^-$ Cys/Glu antiporter is inhibited through an excess of the neurotransmitter glutamine, the latter process is often designated as excitotoxicity in neuronal cells. Because of the clear mechanistic overlaps between oxytosis and ferroptosis it is likely that the use of modulators of ferroptosis in disease will target the same disease processes which are associated with oxytosis. Disease processes where undesired ferroptosis and/or oxytosis occur are typically disorders where an oxidative stress factor is involved such as for example in several neurodegenerative diseases, liver-, cardiac- and kidney-ischaemia - reperfusion injury, stroke, sepsis, diabetes and epilepsy. Oxidative stress due to iron overload is for example highly relevant in organs accumulating iron such as the brain, kidney and liver. Several compounds have been described in the art which are able to inhibit ferroptosis such as for example WO2013/152039, Skouta R. et al (2014) J. Am. Chem. Soc. 136, 4551-4556). The prior art highlights the importance of the ethyl-ester in the maintenance of the potency of the first-in-class compound ferroptosis inhibitor molecule (designated as Ferrostatin-1) and there have been suggestions for chain modifications of the ester for generating improved molecules. Indeed, the latter reference also teaches that esters modified to amides and sulfonamides at the same position have a lower EC50. It has also been suggested that improved pharmacokinetic variants of Ferrostatin-1 could be ester analogues. It would be desirable to generate additional compounds that can inhibit or reduce ferroptosis, in particular compounds with an improved physicochemical and/or pharmacokinetic profile.

There is a need for small molecules that use ferroptosis and/or oxytosis as their target, have strong selectivity, have properties that are amenable to develop them as pharmaceuticals, such as high solubility, and have significant efficacy for the treatment of the ferroptosis and/or oxytosis related diseases.

### Summary of the invention

The present invention is based on the unexpected finding that at least one of the above-mentioned objectives can be attained by small molecules.

The present invention provides compounds which have surprisingly found to be potent inhibitors of ferroptosis and/or oxytosis, while also displaying excellent solubility; in addition, they show very potent *in vitro* anti-ferroptotic activity in the low nanomolar range, good radical-trapping ability, and medium to high human microsomal stability. In view thereof, these compounds can be used to treat diseases where an excess of ferroptosis and/or oxytosis occurs. Further, the inventors have found that for some compounds show very good plasma availability after IV administration, making them

particularly suitable for therapeutic use by parenteral administration.

A first aspect of the present invention provides a compound of formula (I) or a stereoisomer, or tautomer thereof,

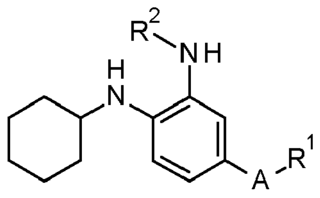

(I)

wherein,

A is selected from

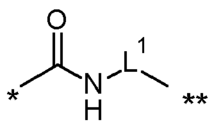

or

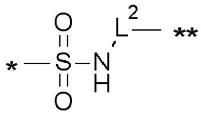

;

wherein * represents where moiety A is bound to the rest of the molecule; and ** represents where moiety A is bound to $R^1$;

$L^1$ is selected from $-(CR^3R^4)_{n}-$ or

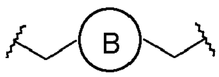

;

$L^2$ is selected from $-(CR^5R^6)_{m}-$ or

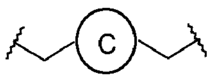

;

n is an integer selected from 1, 2, 3, 4, 5, 6, 7, 8, 9 or 10;

m is an integer selected from 1, 2, 3, 4, 5, 6, 7, 8, 9 or 10;

cycle B is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^B$;

cycle C is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^C$;

$R^1$ is selected from the group consisting of heterocyclyl, $-NR^8R^9$, halogen, halo$C_{1-6}$alkyl, halo$C_{1-6}$alkyloxy, $C_{3-10}$cycloalkyl, $C_{6-12}$aryl, $C_{6-12}$aryl$C_{1-6}$alkyl, heteroaryl, cyano, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said heterocyclyl $C_{3-10}$cycloalkyl, $C_{6-12}$aryl, $C_{6-12}$aryl$C_{1-6}$alkyl, or heteroaryl can be unsubstituted or substituted with one or more $Z^1$;

$R^2$ is selected from the group consisting of $C_{3-10}$cycloalkyl, hydrogen, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl-

$C_{1-6}$alkyl, or heteroaryl$C_{1-6}$alkyl can be unsubstituted or substituted with one or more $Z^2$;

$R^3$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^3$;

$R^4$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^4$;

$R^5$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^5$;

$R^6$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or $Z^6$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro;

or a solvate, hydrate, pharmaceutically acceptable salt, or prodrug thereof;

wherein when moiety A is $-S(O)_2-(CH_2)_2-$ then $R^2$ is not hydrogen, benzyl or pyridyl;

wherein when moiety A is $-C(O)-(CH_2)_2-$ then $R^2$ is not hydrogen or benzyl;

with the proviso that said compound is not

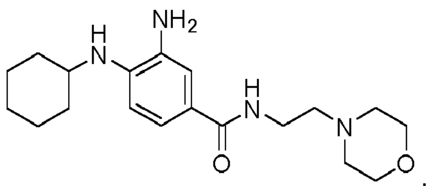

A second, related aspect of the present invention provides a pharmaceutical composition comprising a compound of formula (I) as described in the first aspect of the invention, and a pharmaceutically acceptable carrier.

A third aspect of the present invention provides a compound of formula (I) as described in the first aspect of the invention or a pharmaceutical composition as described in the second aspect of the invention for use as a medicament.

According to a fourth, related aspect, the present invention also encompasses a compound according to the first aspect of the invention, or a pharmaceutical composition according to the second aspect of the invention for use in the prevention or treatment of a disease associated with ferroptosis and/or oxytosis.

A filth aspect of the invention relates to a compound or its stereoisomer, or tautomer, or solvate, hydrate, pharmaceutically acceptable salt, or prodrug thereof, wherein said compound is

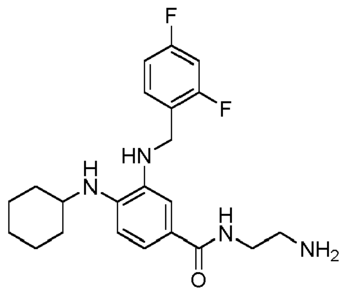

,

for use as a medicament for oral or parenteral, preferably parenteral administration.

A sixth of the invention relates to a compound, or its stereoisomer, or tautomer, or solvate, hydrate, pharmaceutically acceptable salt, or prodrug thereof, wherein said compound is

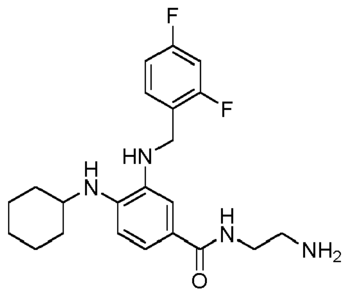

,

for use as a medicament for parenteral administration in the prevention or treatment of a disease associated with ferroptosis and/or oxytosis.

According to a seventh, related aspect, the present invention also encompasses a compound according to the first aspect of the invention or a or a pharmaceutical composition according to the second aspect of the invention or a compound according to the sixth or seventh aspect of the invention for use in the prevention or treatment of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The independent and dependent claims set out particular and preferred features of the invention. Features from the dependent claims may be combined with features of the independent or other dependent claims as appropriate.

## Detailed description of the invention

Before the present invention is described, it is to be understood that this invention is not limited to particular processes, methods, and compounds described, as such processes, methods, and compounds may, of course, vary. It is also to be understood that the terminology used herein is not intended to be limiting, since the scope of the present invention will be limited only by the appended claims.

When describing the compounds and processes of the invention, the terms used are to be construed in accordance with the following definitions, unless a context dictates otherwise.

As used in the specification and the appended claims, the singular forms "a", "an," and "the" include both singular and plural referents unless the context clearly dictates otherwise. By way of example, "a compound" means one compound or more than one compound.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations

are appropriate to perform in the disclosed invention. It is to be understood that the value to which the modifier "about" refers is itself also specifically, and preferably, disclosed.

As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiments but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments combinations of features of different embodiments are meant to be within the scope of the invention, and form different embodiments as would be understood by those in the art. For example, in the following claims, any of the claimed embodiments can be used in any combination.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

When describing the present invention, the terms used are to be construed in accordance with the following definitions, unless a context dictates otherwise.

The terms described above and others used in the specification are well understood to those in the art.

Whenever the term "substituted" is used herein, it is meant to indicate that one or more hydrogen atoms on the atom indicated in the expression using "substituted" is replaced with a selection from the indicated group, provided that the indicated atom's normal valence is not exceeded, and that the substitution results in a chemically stable compound, i.e. a compound that is sufficiently robust to survive isolation from a reaction mixture.

Where groups can be substituted, such groups may be substituted with one or more, and preferably one, two or three substituents. Preferred substituents may be selected from but not limited to, for example, the group comprising halo, hydroxyl, alkyl, alkoxy, trifluoromethyl, trifluoromethoxy, cycloalkyl, aryl, arylalkyl, heterocyclyl, heteroaryl, cyano, amino, nitro, carboxyl, and mono- or dialkylamino.

The term "halo" or "halogen" as a group or part of a group is generic for fluoro, chloro, bromo, iodo.

The term "hydroxyl" or "hydroxy" as used herein refers to the group -OH.

The term "cyano" as used herein refers to the group -C=N.

The term "amino" as used herein refers to the $-NH_2$ group.

The term "nitro" as used herein refers to the $-NO_2$ group.

The term "carboxy" or "carboxyl" or "hydroxycarbonyl" as used herein refers to the group $-CO_2H$.

The term "aminocarbonyl" as used herein refers to the group $-CONH_2$.

The term "alkyl", as a group or part of a group, refers to a hydrocarbyl group of formula $-C_nH_{2n+1}$ wherein n is a number greater than or equal to 1. Alkyl groups may be linear or branched and may be substituted as indicated herein. Generally, alkyl groups of this invention comprise from 1 to 6 carbon atoms, preferably from 1 to 5 carbon atoms, preferably from 1 to 4 carbon atoms, more preferably from 1 to 3 carbon atoms, still more preferably 1 to 2 carbon atoms. When a subscript is used herein following a carbon atom, the subscript refers to the number of carbon atoms that the named group may contain. For example, "$C_{1-6}$alkyl" includes all linear or branched alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers. For example, "$C_{1-5}$alkyl" includes all includes all linear or branched alkyl groups with between 1 and 5 carbon atoms, and thus includes methyl, ethyl, n-propyl, i-propyl, butyl and its isomers (e.g. n-butyl, i-butyl and t-butyl); pentyl and its isomers. For example, "$C_{1-4}$alkyl" includes all linear or branched alkyl groups with between 1 and 4 carbon atoms, and thus includes methyl, ethyl, n-propyl, i-propyl, butyl and its isomers (e.g. n-butyl, i-butyl and t-butyl). For example "$C_{1-3}$alkyl" includes all linear or branched alkyl groups with between 1 and 3 carbon atoms, and thus includes methyl, ethyl, n-propyl, i-propyl.

When the term "alkyl" is used as a suffix following another term, as in "hydroxyalkyl," this is intended to refer to an alkyl group, as defined above, being substituted with one or two (preferably one) substituent(s) selected from the other, specifically-named group, also as defined herein. The term "hydroxyalkyl" therefore refers to a $-R^a-OH$ group wherein $R^a$ is

alkylene as defined herein.

The term "haloalkyl" as a group or part of a group, refers to an alkyl group having the meaning as defined above wherein one, two, or three hydrogen atoms are each replaced with a halogen as defined herein. Non-limiting examples of such haloalkyl groups include chloromethyl, 1-bromoethyl, fluoromethyl, difluoromethyl, trifluoromethyl, 1,1,1-trifluoroethyl, trichloromethyl, tribromomethyl, and the like.

The term "alkoxy" or "alkyloxy", as a group or part of a group, refers to a group having the formula $-OR^b$ wherein $R^b$ is alkyl as defined herein above. Non-limiting examples of suitable alkoxy include methoxy, ethoxy, propoxy, isopropoxy, butoxy, isobutoxy, sec-butoxy, tert-butoxy, pentyloxy and hexyloxy.

The term "cycloalkyl", as a group or part of a group, refers to a cyclic alkyl group, that is a monovalent, saturated, hydrocarbyl group having 1 or more cyclic structure, and comprising from 3 to 10 carbon atoms, more preferably from 3 to 9 carbon atoms, more preferably from 3 to 7 carbon atoms; more preferably from 3 to 6 carbon atoms. Cycloalkyl includes all saturated hydrocarbon groups containing 1 or more rings, including monocyclic or bicyclic groups. The further rings of multi-ring cycloalkyls may be either fused, bridged and/or joined through one or more spiro atoms. When a subscript is used herein following a carbon atom, the subscript refers to the number of carbon atoms that the named group may contain. For example, the term "$C_{3-8}$cycloalkyl", a cyclic alkyl group comprising from 3 to 8 carbon atoms. For example, the term "$C_{3-6}$cycloalkyl", a cyclic alkyl group comprising from 3 to 6 carbon atoms. Examples of $C_{3-10}$cycloalkyl groups include but are not limited to cyclopropyl, cyclobutyl, cyclopentyl, cyclohexyl, cycloheptyl, cyclooctyl, bicycle[2.2.1]heptan-2yl, (1S,4R)-norbornan-2-yl, (1R,4R)-norbornan-2-yl, (1S,4S)-norbornan-2-yl, (1R,4S)-norbornan-2-yl, 1-adamantyl.

The term "cycloalkyloxy", as a group or part of a group, refers to a group having the formula - $OR^f$ wherein $R^f$ is cycloalkyl as defined herein above.

The term "cycloalkenyl", as a group or part of a group, refers to a cyclic alkenyl group, that is a monovalent, unsaturated, hydrocarbyl group having 1 or more cyclic structure, comprising one or more carbon-carbon double bonds; preferably 3 double bonds; preferably 2 double bonds; preferably one double bond. Cycloalkenyl groups according to the present invention comprise from 3 to 10 carbon atoms, more preferably from 3 to 9 carbon atoms, more preferably from 3 to 7 carbon atoms; more preferably from 3 to 6 carbon atoms. Cycloalkenyl includes all unsaturated hydrocarbon groups containing 1 or more rings, including monocyclic or bicyclic groups comprising at least one double bond. The further rings of multi-ring cycloalkenyls may be either fused, bridged and/or joined through one or more spiro atoms.

When a subscript is used herein following a carbon atom, the subscript refers to the number of carbon atoms that the named group may contain. For example, the term "$C_{3-8}$cycloalkenyl", a cyclic alkenyl group comprising from 3 to 8 carbon atoms. For example, the term "$C_{3-6}$cycloalkenyl", a cyclic alkenyl group comprising from 3 to 6 carbon atoms. Examples of $C_{3-10}$cycloalkeyl groups include but are not limited to cyclopropenyl, cyclobutenyl, cyclopentenyl, cyclohexenyl, cycloheptenyl, cyclooctenyl.

The term "cycloalkynyl", as a group or part of a group, refers to a cyclic alkynyl group, that is a monovalent, unsaturated, hydrocarbyl group having 1 or more cyclic structure, comprising one or more carbon-carbon triple bonds; preferably 3 triple bonds; preferably 2 triple bonds; preferably one double bond. Cycloalkynyl groups according to the present invention comprise from 3 to 10 carbon atoms, more preferably from 3 to 9 carbon atoms, more preferably from 3 to 7 carbon atoms; more preferably from 3 to 6 carbon atoms. Cycloalkynyl includes all unsaturated hydrocarbon groups containing 1 or more rings, including monocyclic or bicyclic groups, comprising at least one triple bond. The further rings of multi-ring cycloalkynyls may be either fused, bridged and/or joined through one or more spiro atoms. When a subscript is used herein following a carbon atom, the subscript refers to the number of carbon atoms that the named group may contain. For example, the term "Ca-scycloalkynyl", a cyclic alkynyl group comprising from 3 to 8 carbon atoms. For example, the term "$C_{3-6}$cycloalkynyl", a cyclic alkynnyl group comprising from 3 to 6 carbon atoms. Examples of $C_{3-10}$cycloalkynyl groups include but are not limited to cyclopropynyl, cyclobutynyl, cyclopentynyl, cyclohexynyl, cycloheptynyl, cyclooctynyl.

The term "aryl", as a group or part of a group, refers to a polyunsaturated, aromatic hydrocarbyl group having a single ring (i.e. phenyl) or multiple aromatic rings fused together (e.g. naphthyl), or linked covalently, typically comprising 6 to 12 carbon atoms; wherein at least one ring is aromatic, preferably comprising 6 to 10 carbon atoms, wherein at least one ring is aromatic. The aromatic ring may optionally include one to two additional rings (either cycloalkyl, heterocyclyl or heteroaryl) fused thereto. Examples of suitable aryl include $C_{6-12}$aryl, preferably $C_{6-10}$aryl, more preferably $C_{6-8}$aryl. Non-limiting examples of aryl comprise phenyl, biphenylyl, biphenylenyl, or 1-or 2-naphthanelyl; 5- or 6-tetralinyl, 1-, 2-, 3-, 4-, 5-, 6-, 7- or 8-azulenyl, 4-, 5-, 6 or 7-indenyl, 4- or 5-indanyl, 5-, 6-, 7- or 8-tetrahydronaphthyl, 1,2,3,4-tetrahydronaphthyl, and 1,4-dihydronaphthyl; 1-, 2-, 3-, 4- or 5-pyrenyl. A "substituted aryl" refers to an aryl group having one or more substituent(s) (for example 1, 2 or 3 substituent(s), or 1 to 2 substituent(s)), at any available point of attachment.

The term "aryloxy", as a group or part of a group, refers to a group having the formula $-OR^g$ wherein $R^g$ is aryl as defined herein above.

The term "arylalkyl", as a group or part of a group, means a alkyl as defined herein, wherein at least one hydrogen atom is replaced by at least one aryl as defined herein. Non-limiting examples of arylalkyl group include benzyl, phenethyl, dibenzylmethyl, methylphenylmethyl, 3-(2-naphthyl)-butyl, and the like.

The terms "heterocyclyl" or "heterocycloakyl" or "heterocyclo", as a group or part of a group, refer to non-aromatic, fully saturated or partially unsaturated cyclic groups (for example, 3 to 7 member monocyclic, 7 to 11 member bicyclic, or comprising a total of 3 to 10 ring atoms) which have at least one heteroatom in at least one carbon atom-containing ring; wherein said ring may be fused to an aryl, cycloalkyl, heteroaryl or heterocyclyl ring. Each ring of the heterocyclyl group containing a heteroatom may have 1, 2, 3 or 4 heteroatoms selected from N, O and/or S, where the N and S heteroatoms may optionally be oxidized and the N heteroatoms may optionally be quaternized, and wherein at least one carbon atom of heterocyclyl can be oxidized to form at least one C=O. The heterocyclic group may be attached at any heteroatom or carbon atom of the ring or ring system, where valence allows. The rings of multi-ring heterocycles may be fused, bridged and/or joined through one or more spiro atoms.

Non limiting exemplary heterocyclic groups include aziridinyl, oxiranyl, thiiranyl, piperidinyl, azetidinyl, oxetanyl, pyrrolidinyl, thietanyl, 2-imidazolinyl, pyrazolidinyl imidazolidinyl, isoxazolinyl, oxazolidinyl, isoxazolidinyl, thiazolidinyl, isothiazolidinyl, piperidinyl, succinimidyl, 3H-indolyl, indolinyl, chromanyl (also known as 3,4-dihydrobenzo[b]pyranyl), isoindolinyl, 2H-pyrrolyl, 1-pyrrolinyl, 2-pyrrolinyl, 3-pyrrolinyl, 4H-quinolizinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydrothiophenyl, tetrahydroquinolinyl, tetrahydroisoquinolin-1-yl, tetrahydroisoquinolin-2-yl, tetrahydroisoquinolin-3-yl, tetrahydroisoquinolin-4-yl, thiomorpholin-4-yl, thiomorpholin-4-ylsulfoxide, thiomorpholin-4-ylsulfone, 1,3-dioxolanyl, 1,4-oxathianyl, 1,4-dithianyl, 1,3,5-trioxanyl, 1H-pyrrolizinyl, tetrahydro-1,1-dioxothiophenyl, N- formylpiperazinyl, and morpholin-4-yl. The term "aziridinyl" as used herein includes aziridin-1-yl and aziridin-2-yl. The term "oxyranyl" as used herein includes oxyranyl-2-yl. The term "thiiranyl" as used herein includes thiiran-2-yl. The term "azetidinyl" as used herein includes azetidin-1-yl, azetidin-2-yl and azetidin-3-yl. The term "oxetanyl" as used herein includes oxetan-2-yl and oxetan-3-yl. The term "thietanyl" as used herein includes thietan-2-yl and thietan-3-yl. The term "pyrrolidinyl" as used herein includes pyrrolidin-1-yl, pyrrolidin-2-yl and pyrrolidin-3-yl. The term "tetrahydrofuranyl" as used herein includes tetrahydrofuran-2-yl and tetrahydrofuran-3-yl. The term "tetrahydrothiophenyl" as used herein includes tetrahydrothiophen-2-yl and tetrahydrothiophen-3-yl. The term "succinimidyl" as used herein includes succinimid-1-yl and succininmid-3-yl. The term "dihydropyrrolyl" as used herein includes 2,3-dihydropyrrol-1-yl, 2,3-dihydro-1H-pyrrol-2-yl, 2,3-dihydro-1H-pyrrol-3-yl, 2,5-dihydropyrrol-1-yl, 2,5-dihydro-1H-pyrrol-3-yl and 2,5-dihydropyrrol-5-yl. The term "2H-pyrrolyl" as used herein includes 2H-pyrrol-2-yl, 2H-pyrrol-3-yl, 2H-pyrrol-4-yl and 2H-pyrrol-5-yl. The term "3H-pyrrolyl" as used herein includes 3H-pyrrol-2-yl, 3H-pyrrol-3-yl, 3H-pyrrol-4-yl and 3H-pyrrol-5-yl. The term "dihydrofuranyl" as used herein includes 2,3-dihydrofuran-2-yl, 2,3-dihydrofuran-3-yl, 2,3-dihydrofuran-4-yl, 2,3-dihydrofuran-5-yl, 2,5-dihydrofuran-2-yl, 2,5-dihydrofuran-3-yl, 2,5-dihydrofuran-4-yl and 2,5-dihydrofuran-5-yl. The term "dihydrothiophenyl" as used herein includes 2,3-dihydrothiophen-2-yl, 2,3-dihydrothiophen-3-yl, 2,3-dihydrothiophen-4-yl, 2,3-dihydrothiophen-5-yl, 2,5-dihydrothiophen-2-yl, 2,5-dihydrothiophen-3-yl, 2,5-dihydrothiophen-4-yl and 2,5-dihydrothiophen-5-yl. The term "imidazolidinyl" as used herein includes imidazolidin-1-yl, imidazolidin-2-yl and imidazolidin-4-yl. The term "pyrazolidinyl" as used herein includes pyrazolidin-1-yl, pyrazolidin-3-yl and pyrazolidin-4-yl. The term "imidazolinyl" as used herein includes imidazolin-1-yl, imidazolin-2-yl, imidazolin-4-yl and imidazolin-5-yl. The term "pyrazolinyl" as used herein includes 1-pyrazolin-3-yl, 1-pyrazolin-4-yl, 2-pyrazolin-1-yl, 2-pyrazolin-3-yl, 2-pyrazolin-4-yl, 2-pyrazolin-5-yl, 3-pyrazolin-1-yl, 3-pyrazolin-2-yl, 3-pyrazolin-3-yl, 3-pyrazolin-4-yl and 3-pyrazolin-5-yl. The term "dioxolanyl" also known as "1,3-dioxolanyl" as used herein includes dioxolan-2-yl, dioxolan-4-yl and dioxolan-5-yl. The term "dioxolyl" also known as "1,3-dioxolyl" as used herein includes dioxol-2-yl, dioxol-4-yl and dioxol-5-yl. The term "oxazolidinyl" as used herein includes oxazolidin-2-yl, oxazolidin-3-yl, oxazolidin-4-yl and oxazolidin-5-yl. The term "isoxazolidinyl" as used herein includes isoxazolidin-2-yl, isoxazolidin-3-yl, isoxazolidin-4-yl and isoxazolidin-5-yl. The term "oxazolinyl" as used herein includes 2-oxazolinyl-2-yl, 2-oxazolinyl-4-yl, 2-oxazolinyl-5-yl, 3-oxazolinyl-2-yl, 3-oxazolinyl-4-yl, 3-oxazolinyl-5-yl, 4-oxazolinyl-2-yl, 4-oxazolinyl-3-yl, 4-oxazolinyl-4-yl and 4-oxazolinyl-5-yl. The term "isoxazolinyl" as used herein includes 2-isoxazolinyl-3-yl, 2-isoxazolinyl-4-yl, 2-isoxazolinyl-5-yl, 3-isoxazolinyl-3-yl, 3-isoxazolinyl-4-yl, 3-isoxazolinyl-5-yl, 4-isoxazolinyl-2-yl, 4-isoxazolinyl-3-yl, 4-isoxazolinyl-4-yl and 4-isoxazolinyl-5-yl. The term "thiazolidinyl" as used herein includes thiazolidin-2-yl, thiazolidin-3-yl, thiazolidin-4-yl and thiazolidin-5-yl. The term "isothiazolidinyl" as used herein includes isothiazolidin-2-yl, isothiazolidin-3-yl, isothiazolidin-4-yl and isothiazolidin-5-yl. The term "chromanyl" as used herein includes chroman-2-yl, chroman-3-yl, chroman-4-yl, chroman-5-yl, chroman-6-yl, chroman-7-yl and chroman-8-yl. The term "thiazolinyl" as used herein includes 2-thiazolinyl-2-yl, 2-thiazolinyl-4-yl, 2-thiazolinyl-5-yl, 3-thiazolinyl-2-yl, 3-thiazolinyl-4-yl, 3-thiazolinyl-5-yl, 4-thiazolinyl-2-yl, 4-thiazolinyl-3-yl, 4-thiazolinyl-4-yl and 4-thiazolinyl-5-yl. The term "isothiazolinyl" as used herein includes 2-isothiazolinyl-3-yl, 2-isothiazolinyl-4-yl, 2-isothiazolinyl-5-yl, 3-isothiazolinyl-3-yl, 3-isothiazolinyl-4-yl, 3-isothiazolinyl-5-yl, 4-isothiazolinyl-2-yl, 4-isothiazolinyl-3-yl, 4-isothiazolinyl-4-yl and 4-isothiazolinyl-5-yl. The term "piperidyl" also known as "piperidinyl" as used herein includes piperid-1-yl, piperid-2-yl, piperid-3-yl and piperid-4-yl. The term "dihydropyridinyl" as used herein includes 1,2-dihydropyridin-1-yl, 1,2-dihydropyridin-2-yl, 1,2-dihydropyridin-3-yl, 1,2-dihydropyridin-4-yl, 1,2-dihydropyridin-5-yl, 1,2-dihydropyridin-6-yl, 1,4-dihydropyridin-1-yl, 1,4-dihydropyridin-2-yl, 1,4-dihydropyridin-3-yl, 1,4-dihydropyridin-4-yl, 2,3-dihydropyridin-2-yl, 2,3-dihydropyridin-3-yl, 2,3-dihydropyridin-4-yl, 2,3-dihydropyridin-5-yl, 2,3-dihydropyridin-6-yl, 2,5-dihydropyridin-2-yl, 2,5-dihydropyridin-3-yl, 2,5-dihydropyridin-4-yl, 2,5-dihydropyridin-5-yl, 2,5-dihydropyridin-6-yl, 3,4-dihydropyridin-2-yl, 3,4-dihydropyridin-3-yl, 3,4-dihydropyridin-4-yl, 3,4-dihydropyridin-5-yl and 3,4-dihydropyridin-6-yl. The term "tetrahydropyridinyl" as used herein includes 1,2,3,4-tetrahydropyridin-1-yl, 1,2,3,4-tetrahydropyridin-2-yl, 1,2,3,4-tetrahydropyridin-3-yl, 1,2,3,4-tetrahydropyridin-4-yl, 1,2,3,4-tetrahydropyridin-5-yl, 1,2,3,4-tetrahydropyridin-6-yl, 1,2,3,6-tetrahydropyridin-1-yl, 1,2,3,6-tetrahydropyridin-2-yl, 1,2,3,6-tetrahydropyridin-3-yl, 1,2,3,6-tetrahydropyridin-4-yl, 1,2,3,6-tetrahydropyridin-5-yl, 1,2,3,6-tetrahydropyridin-6-yl, 2,3,4,5-tetrahydropyridin-2-yl, 2,3,4,5-tetrahydropyridin-3-yl, 2,3,4,5-tetrahydropyridin-3-yl, 2,3,4,5-tetrahydropyridin-4-yl, 2,3,4,5-tetrahydropyridin-5-yl and 2,3,4,5-tetrahydropyridin-6-yl. The term "tetrahydropyranyl" also known as "oxanyl" or "tetrahydro-2H-pyranyl", as used herein includes tetrahydropyran-2-yl, tetrahydropyran-3-yl and tetrahydropyran-4-yl. The term "2H-pyranyl" as used herein includes 2H-pyran-2-yl, 2H-pyran-3-yl, 2H-pyran-4-yl, 2H-pyran-5-yl and 2H-pyran-6-yl. The term "4H-pyranyl" as used herein includes 4H-pyran-2-yl, 4H-pyran-3-yl and 4H-pyran-4-yl. The term "3,4-dihydro-2H-pyranyl" as used herein includes 3,4-dihydro-2H-pyran-2-yl, 3,4-dihydro-2H-pyran-3-yl, 3,4-dihydro-2H-pyran-4-yl, 3,4-dihydro-2H-pyran-5-yl and 3,4-dihydro-2H-pyran-6-yl. The term "3,6-dihydro-2H-pyranyl" as used herein includes 3,6-dihydro-2H-pyran-2-yl, 3,6-dihydro-2H-pyran-3-yl, 3,6-dihydro-2H-pyran-4-yl, 3,6-dihydro-2H-pyran-5-yl and 3,6-dihydro-2H-pyran-6-yl. The term "tetrahydrothiophenyl", as used herein includes tetrahydrothiophen-2-yl, tetrahydrothiophenyl -3-yl and tetrahydrothiophenyl -4-yl. The term "2H-thiopyranyl" as used herein includes 2H-thiopyran-2-yl, 2H-thiopyran-3-yl, 2H-thiopyran-4-yl, 2H-thiopyran-5-yl and 2H-thiopyran-6-yl. The term "4H-thiopyranyl" as used herein includes 4H-thiopyran-2-yl, 4H-thiopyran-3-yl and 4H-thiopyran-4-yl. The term "3,4-dihydro-2H-thiopyranyl" as used herein includes 3,4-dihydro-2H-thiopyran-2-yl, 3,4-dihydro-2H-thiopyran-3-yl, 3,4-dihydro-2H-thiopyran-4-yl, 3,4-dihydro-2H-thiopyran-5-yl and 3,4-dihydro-2H-thiopyran-6-yl. The term "3,6-dihydro-2H-thiopyranyl" as used herein includes 3,6-dihydro-2H-thiopyran-2-yl, 3,6-dihydro-2H-thiopyran-3-yl, 3,6-dihydro-2H-thiopyran-4-yl, 3,6-dihydro-2H-thiopyran-5-yl and 3,6-dihydro-2H-thiopyran-6-yl. The term "piperazinyl" also known as "piperazidinyl" as used herein includes piperazin-1-yl and piperazin-2-yl. The term "morpholinyl" as used herein includes morpholin-2-yl, morpholin-3-yl and morpholin-4-yl. The term "thiomorpholinyl" as used herein includes thiomorpholin-2-yl, thiomorpholin-3-yl and thiomorpholin-4-yl. The term "dioxanyl" as used herein includes 1,2-dioxan-3-yl, 1,2-dioxan-4-yl, 1,3-dioxan-2-yl, 1,3-dioxan-4-yl, 1,3-dioxan-5-yl and 1,4-dioxan-2-yl. The term "dithianyl" as used herein includes 1,2-dithian-3-yl, 1,2-dithian-4-yl, 1,3-dithian-2-yl, 1,3-dithian-4-yl, 1,3-dithian-5-yl and 1,4-dithian-2-yl. The term "oxathianyl" as used herein includes oxathian-2-yl and oxathian-3-yl. The term "trioxanyl" as used herein includes 1,2,3-trioxan-4-yl, 1,2,3-trioxay-5-yl, 1,2,4-trioxay-3-yl, 1,2,4-trioxay-5-yl, 1,2,4-trioxay-6-yl and 1,3,4-trioxay-2-yl. The term "azepanyl" as used herein includes azepan-1-yl, azepan-2-yl, azepan-1-yl, azepan-3-yl and azepan-4-yl. The term "homopiperazinyl" as used herein includes homopiperazin-1-yl, homopiperazin-2-yl, homopiperazin-3-yl and homopiperazin-4-yl. The term "indolinyl" as used herein includes indolin-1-yl, indolin-2-yl, indolin-3-yl, indolin-4-yl, indolin-5-yl, indolin-6-yl, and indolin-7-yl. The term "quinolizinyl" as used herein includes quinolizidin-1-yl, quinolizidin-2-yl, quinolizidin-3-yl and quinolizidin-4-yl. The term "isoindolinyl" as used herein includes isoindolin-1-yl, isoindolin-2-yl, isoindolin-3-yl, isoindolin-4-yl, isoindolin-5-yl, isoindolin-6-yl, and isoindolin-7-yl. The term "3H-indolyl" as used herein includes 3H-indol-2-yl, 3H-indol-3-yl, 3H-indol-4-yl, 3H-indol-5-yl, 3H-indol-6-yl, and 3H-indol-7-yl. The term "quinolizinyl" as used herein includes quinolizidin-1-yl, quinolizidin-2-yl, quinolizidin-3-yl and quinolizidin-4-yl. The term "quinolizinyl" as used herein includes quinolizidin-1-yl, quinolizidin-2-yl, quinolizidin-3-yl and quinolizidin-4-yl. The term "tetrahydroquinolinyl" as used herein includes tetrahydroquinolin-1-yl, tetrahydroquinolin-2-yl, tetrahydroquinolin-3-yl, tetrahydroquinolin-4-yl, tetrahydroquinolin-5-yl, tetrahydroquinolin-6-yl, tetrahydroquinolin-7-yl and tetrahydroquinolin-8-yl. The term "tetrahydroisoquinolinyl" as used herein includes tetrahydroisoquinolin-1-yl, tetrahydroisoquinolin-2-yl, tetrahydroisoquinolin-3-yl, tetrahydroisoquinolin-4-yl, tetrahydroisoquinolin-5-yl, tetrahydroisoquinolin-6-yl, tetrahydroisoquinolin-7-yl and tetrahydroisoquinolin-8-yl. The term "1H-pyrrolizine" as used herein includes 1H-pyrrolizin-1-yl, 1H-pyrrolizin-2-yl, 1H-pyrrolizin-3-yl, 1H-pyrrolizin-5-yl, 1H-pyrrolizin-6-yl and 1H-pyrrolizin-7-yl. The term "3H-pyrrolizine" as used herein includes 3H-pyrrolizin-1-yl, 3H-pyrrolizin-2-yl, 3H-pyrrolizin-3-yl, 3H-pyrrolizin-5-yl, 3H-pyrrolizin-6-yl and 3H-pyrrolizin-7-yl.

The term "heterocyclyloxy", as a group or part of a group, refers to a group having the formula $-O-R^i$ wherein $R^i$ is heterocyclyl as defined herein above.

The term "heterocyclylalkyl", as a group or part of a group, means a alkyl as defined herein, wherein at least one hydrogen atom is replaced by at least one heterocyclyl as defined herein.

The term "heteroaryl" as a group or part of a group, refers but is not limited to 5 to 12 carbon-atom aromatic rings or ring systems containing 1 or 2 rings which can be fused together or linked covalently, typically containing 5 to 6 atoms; at least one of which is aromatic in which one or more carbon atoms in one or more of these rings can be replaced by N, O and/or S atoms where the N and S heteroatoms may optionally be oxidized and the N heteroatoms may optionally be quaternized, and wherein at least one carbon atom of said heteroaryl can be oxidized to form at least one C=O. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl,

isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, benzo[d]oxazol-2(3H)-one, 2,3-dihydro-benzofuranyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1 (2H)-yl, 1,3-benzodioxolyl, quinolinyl, isoquinolinyl, cinnolinyl, quinazolinyl, quinoxalinyl; preferably said heteroaryl group is selected from the group consisting of pyridyl, 1,3-benzodioxolyl, benzo[d]oxazol-2(3H)-one, 2,3-dihydro-benzofuranyl, pyrazinyl, pyrazolyl, pyrrolyl, isoxazolyl, thiophenyl, imidazolyl, benzimidazolyl, pyrimidinyl, triazolyl and thiazolyl.

The term "pyrrolyl" (also called azolyl) as used herein includes pyrrol-1-yl, pyrrol-2-yl and pyrrol-3-yl. The term "furanyl" (also called "furyl") as used herein includes furan-2-yl and furan-3-yl (also called furan-2-yl and furan-3-yl). The term "thiophenyl" (also called "thienyl") as used herein includes thiophen-2-yl and thiophen-3-yl (also called thien-2-yl and thien-3-yl). The term "pyrazolyl" (also called 1H-pyrazolyl and 1,2-diazolyl) as used herein includes pyrazol-1-yl, pyrazol-3-yl, pyrazol-4-yl and pyrazol-5-yl. The term "imidazolyl" as used herein includes imidazol-1-yl, imidazol-2-yl, imidazol-4-yl and imidazol-5-yl. The term "oxazolyl" (also called 1,3-oxazolyl) as used herein includes oxazol-2-yl, oxazol-4-yl and oxazol-5-yl. The term "isoxazolyl" (also called 1,2-oxazolyl), as used herein includes isoxazol-3-yl, isoxazol-4-yl, and isoxazol-5-yl. The term "thiazolyl" (also called 1,3-thiazolyl),as used herein includes thiazol-2-yl, thiazol-4-yl and thiazol-5-yl (also called 2-thiazolyl, 4-thiazolyl and 5-thiazolyl). The term "isothiazolyl" (also called 1,2-thiazolyl) as used herein includes isothiazol-3-yl, isothiazol-4-yl, and isothiazol-5-yl. The term "triazolyl" as used herein includes 1H-triazolyl and 4H-1,2,4-triazolyl, "1H-triazolyl" includes 1H-1,2,3-triazol-1-yl, 1H-1,2,3-triazol-4-yl, 1H-1,2,3-triazol-5-yl, 1H-1,2,4-triazol-1-yl, 1H-1,2,4-triazol-3-yl and 1H-1,2,4-triazol-5-yl. "4H-1,2,4-triazolyl" includes 4H-1,2,4-triazol-4-yl, and 4H-1,2,4-triazol-3-yl. The term "oxadiazolyl" as used herein includes 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl and 1,3,4-oxadiazol-2-yl. The term "thiadiazolyl" as used herein includes 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl (also called furazan-3-yl) and 1,3,4-thiadiazol-2-yl. The term "tetrazolyl" as used herein includes 1H-tetrazol-1-yl, 1H-tetrazol-5-yl, 2H-tetrazol-2-yl, and 2H-tetrazol-5-yl. The term "oxatriazolyl" as used herein includes 1,2,3,4-oxatriazol-5-yl and 1,2,3,5-oxatriazol-4-yl. The term "thiatriazolyl" as used herein includes 1,2,3,4-thiatriazol-5-yl and 1,2,3,5-thiatriazol-4-yl. The term "pyridinyl" (also called "pyridyl") as used herein includes pyridin-2-yl, pyridin-3-yl and pyridin-4-yl (also called 2-pyridyl, 3-pyridyl and 4-pyridyl). The term "pyrimidyl" as used herein includes pyrimid-2-yl, pyrimid-4-yl, pyrimid-5-yl and pyrimid-6-yl. The term "pyrazinyl" as used herein includes pyrazin-2-yl and pyrazin-3-yl. The term "pyridazinyl as used herein includes pyridazin-3-yl and pyridazin-4-yl. The term "oxazinyl" (also called "1,4-oxazinyl") as used herein includes 1,4-oxazin-4-yl and 1,4-oxazin-5-yl. The term "dioxinyl" (also called "1,4-dioxinyl") as used herein includes 1,4-dioxin-2-yl and 1,4-dioxin-3-yl. The term "thiazinyl" (also called "1,4-thiazinyl") as used herein includes 1,4-thiazin-2-yl, 1,4-thiazin-3-yl, 1,4-thiazin-4-yl, 1,4-thiazin-5-yl and 1,4-thiazin-6-yl. The term "triazinyl" as used herein includes 1,3,5-triazin-2-yl, 1,2,4-triazin-3-yl, 1,2,4-triazin-5-yl, 1,2,4-triazin-6-yl, 1,2,3-triazin-4-yl and 1,2,3-triazin-5-yl. The term "imidazo[2,1-b][1,3]thiazolyl" as used herein includes imidazo[2,1-b][1,3]thiazoi-2-yl, imidazo[2,1-b][1,3]thiazol-3-yl, imidazo[2,1-b][1,3]thiazol-5-yl and imidazo[2,1-b][1,3]thiazol-6-yl. The term "thieno[3,2-b]furanyl" as used herein includes thieno[3,2-b]furan-2-yl, thieno[3,2-b]furan-3-yl, thieno[3,2-b]furan-4-yl, and thieno[3,2-b]furan-5-yl. The term "thieno[3,2-b]thiophenyl" as used herein includes thieno[3,2-b]thien-2-yl, thieno[3,2-b]thien-3-yl, thieno[3,2-b]thien-5-yl and thieno[3,2-b]thien-6-yl. The term "thieno[2,3-d][1,3]thiazolyl" as used herein includes thieno[2,3-d][1,3]thiazol-2-yl, thieno[2,3-d][1,3]thiazol-5-yl and thieno[2,3-d][1,3]thiazol-6-yl. The term "thieno[2,3-d]imidazolyl" as used herein includes thieno[2,3-d]imidazol-2-yl, thieno[2,3-d]imidazol-4-yl and thieno[2,3-d]imidazol-5-yl. The term "tetrazolo[1,5-a]pyridinyl" as used herein includes tetrazolo[1,5-a]pyridine-5-yl, tetrazolo[1,5-a]pyridine-6-yl, tetrazolo[1,5-a]pyridine-7-yl, and tetrazolo[1,5-a]pyridine-8-yl. The term "indolyl" as used herein includes indol-1-yl, indol-2-yl, indol-3-yl,-indol-4-yl, indol-5-yl, indol-6-yl and indol-7-yl. The term "indolizinyl" as used herein includes indolizin-1-yl, indolizin-2-yl, indolizin-3-yl, indolizin-5-yl, indolizin-6-yl, indolizin-7-yl, and indolizin-8-yl. The term "isoindolyl" as used herein includes isoindol-1-yl, isoindol-2-yl, isoindol-3-yl, isoindol-4-yl, isoindol-5-yl, isoindol-6-yl and isoindol-7-yl. The term "benzofuranyl" (also called benzo[b]furanyl) as used herein includes benzofuran-2-yl, benzofuran-3-yl, benzofuran-4-yl, benzofuran-5-yl, benzofuran-6-yl and benzofuran-7-yl. The term "isobenzofuranyl" (also called benzo[c]furanyl) as used herein includes isobenzofuran-1-yl, isobenzofuran-3-yl, isobenzofuran-4-yl, isobenzofuran-5-yl, isobenzofuran-6-yl and isobenzofuran-7-yl. The term "benzothiophenyl" (also called benzo[b]thienyl) as used herein includes 2-benzo[b]thiophenyl, 3-benzo[b]thiophenyl, 4-benzo[b]thiophenyl, 5-benzo[b]thiophenyl, 6-benzo[b]thiophenyl and -7-benzo[b]thiophenyl (also called benzothien-2-yl, benzothien-3-yl, benzothien-4-yl, benzothien-5-yl, benzothien-6-yl and benzothien-7-yl). The term "isobenzothiophenyl" (also called benzo[c]thienyl) as used herein includes isobenzothien-1-yl, isobenzothien-3-yl, isobenzothien-4-yl, isobenzothien-5-yl, isobenzothien-6-yl and isobenzothien-7-yl. The term "indazolyl" (also called 1H-indazolyl or 2-azaindolyl) as used herein includes 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, and 2H-indazol-7-yl. The term "benzimidazolyl" as used herein includes benzimidazol-1-yl, benzimidazol-2-yl, benzimidazol-4-yl, benzimidazol-5-yl, benzimidazol-6-yl and benzimidazol-7-yl. The term "1,3-benzoxazolyl" as used herein includes 1,3-benzoxazol-2-yl, 1,3-benzoxazol-4-yl, 1,3-benzoxazol-5-yl, 1,3-benzoxazol-6-yl and 1,3-benzoxazol-7-yl.

The term "1,2-benzisoxazolyl" as used herein includes 1,2-benzisoxazol-3-yl, 1,2-benzisoxazol-4-yl, 1,2-benzisoxazol-5-yl, 1,2-benzisoxazol-6-yl and 1,2-benzisoxazol-7-yl. The term "2,1-benzisoxazolyl" as used herein includes 2,1-benzisoxazol-3-yl, 2,1-benzisoxazol-4-yl, 2,1-benzisoxazol-5-yl, 2,1-benzisoxazol-6-yl and 2,1-benzisoxazol-7-yl. The term "1,3-benzothiazolyl" as used herein includes 1,3-benzothiazol-2-yl, 1,3-benzothiazol-4-yl, 1,3-benzothiazol-5-yl, 1,3-benzothiazol-6-yl and 1,3-benzothiazol-7-yl. The term "1,2-benzoisothiazolyl" as used herein includes 1,2-benzisothiazol-3-yl, 1,2-benzisothiazol-4-yl, 1,2-benzisothiazol-5-yl, 1,2-benzisothiazol-6-yl and 1,2-benzisothiazol-7-yl. The term "2,1-benzoisothiazolyl" as used herein includes 2,1-benzisothiazol-3-yl, 2,1-benzisothiazol-4-yl, 2,1-benzisothiazol-5-yl, 2,1-benzisothiazol-6-yl and 2,1-benzisothiazol-7-yl. The term "benzotriazolyl" as used herein includes benzotriazol-1-yl, benzotriazol-4-yl, benzotriazol-5-yl, benzotriazol-6-yl and benzotriazol-7-yl. The term "1,2,3-benzoxadiazolyl" as used herein includes 1,2,3-benzoxadiazol-4-yl, 1,2,3-benzoxadiazol-5-yl, 1,2,3-benzoxadiazol-6-yl and 1,2,3-benzoxadiazol-7-yl. The term "2,1,3-benzoxadiazolyl" as used herein includes 2,1,3-benzoxadiazol-4-yl, 2,1,3-benzoxadiazol-5-yl, 2,1,3-benzoxadiazol-6-yl and 2,1,3-benzoxadiazol-7-yl. The term "1,2,3-benzothiadiazolyl" as used herein includes 1,2,3-benzothiadiazol-4-yl, 1,2,3-benzothiadiazol-5-yl, 1,2,3-benzothiadiazol-6-yl and 1,2,3-benzothiadiazol-7-yl. The term "2,1,3-benzothiadiazolyl" as used herein includes 2,1,3-benzothiadiazol-4-yl, 2,1,3-benzothiadiazol-5-yl, 2,1,3-benzothiadiazol-6-yl and 2,1,3-benzothiadiazol-7-yl. The term "thienopyridinyl" as used herein includes thieno[2,3-b]pyridinyl, thieno[2,3-c]pyridinyl, thieno[3,2-c]pyridinyl and thieno[3,2-b]pyridinyl. The term "purinyl" as used herein includes purin-2-yl, purin-6-yl, purin-7-yl and purin-8-yl. The term "imidazo[1,2-a]pyridinyl", as used herein includes imidazo[1,2-a]pyridin-2-yl, imidazo[1,2-a]pyridin-3-yl, imidazo[1,2-a]pyridin-4-yl, imidazo[1,2-a]pyridin-5-yl, imidazo[1,2-a]pyridin-6-yl and imidazo[1,2-a]pyridin-7-yl. The term "1,3-benzodioxolyl", as used herein includes 1,3-benzodioxol-4-yl, 1,3-benzodioxol-5-yl, 1,3-benzodioxol-6-yl, and 1,3-benzodioxol-7-yl. The term "quinolinyl" as used herein includes quinolin-2-yl, quinolin-3-yl, quinolin-4-yl, quinolin-5-yl, quinolin-6-yl, quinolin-7-yl and quinolin-8-yl. The term "isoquinolinyl" as used herein includes isoquinolin-1-yl, isoquinolin-3-yl, isoquinolin-4-yl, isoquinolin-5-yl, isoquinolin-6-yl, isoquinolin-7-yl and isoquinolin-8-yl. The term "cinnolinyl" as used herein includes cinnolin-3-yl, cinnolin-4-yl, cinnolin-5-yl, cinnolin-6-yl, cinnolin-7-yl and cinnolin-8-yl. The term "quinazolinyl" as used herein includes quinazolin-2-yl, quinazolin-4-yl, quinazolin-5-yl, quinazolin-6-yl, quinazolin-7-yl and quinazolin-8-yl. The term "quinoxalinyl" as used herein includes quinoxalin-2-yl, quinoxalin-5-yl, and quinoxalin-6-yl.

The term "heteroaryloxy", as a group or part of a group, refers to a group having the formula -O-$R^k$ wherein $R^k$ is heteroaryl as defined herein above.

The term "heteroarylalkyl", as a group or part of a group, means a alkyl as defined herein, wherein at least one hydrogen atom is replaced by at least one heteroaryl as defined herein.

The term "mono- or di-alkylamino", as a group or part of a group, refers to a group of formula -N($R^o$)($R^p$) wherein R ° and $R^p$ are each independently selected from hydrogen, or alkyl, wherein at least one of R° or $R^p$ is alkyl. Thus, alkylamino include mono-alkyl amino group (e.g. mono-$C_{1-6}$alkylamino group such as methylamino and ethylamino), and di-alkylamino group (e.g. di-$C_{1-6}$alkylamino group such as dimethylamino and diethylamino). Non-limiting examples of suitable mono- or di-alkylamino groups include n-propylamino, isopropylamino, *n*-butylamino, *i*-butylamino, *sec*-butylamino, *t*-butylamino, pentylamino, *n*-hexylamino, di-*n*-propylamino, di-*i*-propylamino, ethylmethylamino, methyl-*n*-propylamino, methyl-*i*-propylamino, *n*-butylmethylamino, *i*-butylmethylamino, *t*-butylmethylamino, ethyl-*n*-propylamino, ethyl-*i*-propylamino, *n*-butylethylamino, i-butylethylamino, *t*-butylethylamino, di*n*-butylamino, di-*i*-butylamino, methylpentylamino, methylhexylamino, ethylpentylamino, ethylhexylamino, propylpentylamino, propylhexylamino, and the like.

The term "mono- or di-arylamino", as a group or part of a group, refers to a group of formula -N($R^q$)($R^r$) wherein $R^q$ and $R^r$ are each independently selected from hydrogen, or aryl, wherein at least one of $R^q$ or $R^r$ is aryl.

The term "mono- or di-arylalkylamino", as a group or part of a group, refers to a group of formula -N($R^{q'}$)($R^{r'}$) wherein $R^{q'}$ and $R^{r'}$ are each independently selected from hydrogen, or arylalkyl, wherein at least one of $R^{q'}$ or $R^{r'}$ is arylalkyl.

The term "mono- or di-cycloalkylamino", as a group or part of a group, refers to a group of formula -N($R^s$)($R^t$) wherein $R^s$ and $R^t$ are each independently selected from hydrogen, or cycloalkyl, wherein at least one of $R^s$ or $R^t$ is cycloalkyl.

The term "mono- or di-heteroarylamino", as a group or part of a group, refers to a group of formula -N($R^u$)($R^v$) wherein $R^u$ and $R^v$ are each independently selected from hydrogen, or heteroaryl, wherein at least one of $R^u$ or $R^v$ is heteroaryl as defined herein.

The term "mono- or di-heterocyclylamino", as a group or part of a group, refers to a group of formula -N($R^w$)($R^x$) wherein $R^w$ and $R^x$ are each independently selected from hydrogen, or heterocyclyl, wherein at least one of $R^w$ or $R^x$ is heterocyclyl as defined herein.

The term "alkyloxycarbonyl", as a group or part of a group, refers to a group of formula - COO-$R^b$, wherein $R^b$ is alkyl as defined herein.

The term "cycloakyloxycarbonyl", as a group or part of a group, refers to a group of formula - COO-$R^b$, wherein $R^b$ is cycloalkyl as defined herein.

The term "aryloxycarbonyl", as a group or part of a group, refers to a group of formula - COO-$R^b$, wherein $R^b$ is aryl

as defined herein.

The term "heterocyclyloxycarbonyl", as a group or part of a group, refers to a group of formula -COO-$R^b$, wherein $R^b$ is heterocyclyl as defined herein.

The term "heteroaryloxycarbonyl", as a group or part of a group, refers to a group of formula -COO-$R^b$, wherein $R^b$ is heteroaryl as defined herein.

The term "alkylsulfinyl", as a group or part of a group, refers to a group of formula -SO-$R^b$, wherein $R^b$ is alkyl as defined herein.

The term "alkylsulfonyl", as a group or part of a group, refers to a group of formula -$S(O)_2$-$R^b$, wherein $R^b$ is alkyl as defined herein.

The term "cycloalkylsulfonyl", as a group or part of a group, refers to a group of formula - $S(O)_2$-$R^b$, wherein $R^b$ is cycloalkyl as defined herein.

The term "arylsulfonyl", as a group or part of a group, refers to a group of formula -$S(O)_2$-$R^b$, wherein $R^b$ is aryl as defined herein.

The term "heterocyclylsulfonyl", as a group or part of a group, refers to a group of formula - $S(O)_2$-$R^b$, wherein $R^b$ is heterocyclyl as defined herein.

The term "heteroarylsulfonyl", as a group or part of a group, refers to a group of formula - $S(O)_2$-$R^b$, wherein $R^b$ is heteroaryl as defined herein.

The term "mono- or di-alkylaminosulfonyl", as a group or part of a group, refers to a group of formula -$S(O)_2$-NN$R^oR^p$, wherein $R^oR^p$ are each independently selected from hydrogen, or alkyl, wherein at least one of R° or $R^p$ is alkyl.

The term "mono- or di-cycloalkylaminosulfonyl", as a group or part of a group, refers to a group of formula -$S(O)_2$-NN$R^oR^p$, wherein $R^oR^p$ are each independently selected from hydrogen, or alkyl, wherein at least one of R° or $R^p$ is cycloalkyl.

The term "mono- or di-arylaminosulfonyl", as a group or part of a group, refers to a group of formula -$S(O)_2$-NN$R^oR^p$, wherein $R^oR^p$ are each independently selected from hydrogen, or alkyl, wherein at least one of R° or $R^p$ is aryl.

The term "mono- or di-heterocyclylaminosulfonyl", as a group or part of a group, refers to a group of formula -$S(O)_2$-NN$R^oR^p$, wherein $R^oR^p$ are each independently selected from hydrogen, or alkyl, wherein at least one of R° or $R^p$ is heterocyclyl.

The term "mono- or di-heteroarylaminosulfonyl", as a group or part of a group, refers to a group of formula -$S(O)_2$-NN$R^oR^p$, wherein $R^oR^p$ are each independently selected from hydrogen, or alkyl, wherein at least one of R° or $R^p$ is heteroaryl.

The term "mono- or dialkylaminocarbonyl", as a group or part of a group, refers to a group of formula -CON$R^oR^p$ wherein $R^oR^p$ are each independently selected from hydrogen, or alkyl, wherein at least one of R° or $R^p$ is alkyl.

The term "mono- or dicycloalkylaminocarbonyl", as a group or part of a group, refers to a group of formula -CON$R^oR^p$ wherein $R^oR^p$ are each independently selected from hydrogen, or cycloalkyl, wherein at least one of R° or $R^p$ is cycloalkyl.

The term "alkylcarbonyl", as a group or part of a group, refers to a group of formula -CO-$R^b$, wherein $R^b$ is alkyl as defined herein.

The term "cycloalkylcarbonyl", as a group or part of a group, refers to a group of formula - CO-$R^b$, wherein $R^b$ is cycloalkyl as defined herein.

The term "arylcarbonyl", as a group or part of a group, refers to a group of formula -CO-$R^b$, wherein $R^b$ is aryl as defined herein.

The term "heterocyclylcarbonyl", as a group or part of a group, refers to a group of formula - CO-$R^b$, wherein $R^b$ is heterocyclyl as defined herein.

The term "heteroarylcarbonyl", as a group or part of a group, refers to a group of formula - CO-$R^b$, wherein $R^b$ is heteroaryl as defined herein.

The term "alkylcarbonylamino", as a group or part of a group, refers to a group of formula -N$R^o$-CO-$R^b$, wherein R ° is selected from hydrogen, or alkyl and $R^b$ is alkyl as defined herein.

The term "alkylsulfonylamino", as a group or part of a group, refers to a group of formula -NR°-S(O)z-$R^b$, wherein R° is selected from hydrogen, or alkyl and $R^b$ is alkyl as defined herein.

Whenever used in the present invention the term "compounds of the invention" or a similar term is meant to include the compounds of general formula (I), as defined above, as well as (II), (II), (IV), (IA), (IB), (IC), (ID), (IVA), (IVB), (IVC), (IVD) as detailed below and any subgroup thereof. This term also refers to the compounds as depicted in Table 1 and their derivatives, salts, solvates, hydrates, tautomeric forms, analogues, pro-drugs, esters and metabolites, as well as their quaternized nitrogen analogues.

As used herein and unless otherwise stated, the term "stereoisomer" refers to all possible different isomeric as well as conformational forms which the compounds of structural formula herein may possess, in particular all possible

stereochemically and conformationally isomeric forms, all diastereomers, enantiomers and/or conformers of the basic molecular structure. Some compounds of the present invention may exist in different tautomeric forms, all of the latter being included within the scope of the present invention.

The present invention includes all possible stereoisomers compounds of formula (I) and any subgroup thereof. When a compound is desired as a single enantiomer, such may be obtained by stereospecific synthesis, by resolution of the final product or any convenient intermediate, or by chiral chromatographic methods as each are known in the art. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994), incorporated by reference with regard to stereochemistry. A structural isomer is a type of isomer in which molecules with the same molecular formula have different bonding patterns and atomic organization. Where structural isomers are interconvertible via a low energy barrier, tautomeric isomerism ('tautomerism') can occur. This can take the form of proton tautomerism in compounds of the invention containing, for example, an imino, keto, or oxime group, or so-called valence tautomerism in compounds which contain an aromatic moiety.

The term "prodrug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Prodrugs of the compounds of the invention can be prepared by modifying functional groups present in said component in such a way that the modifications are cleaved, either in routine manipulation or *in vivo,* to the parent component. Typical examples of prodrugs are described for instance in WO 99/33795, WO 99/33815, WO 99/33793 and WO 99/33792 all incorporated herein by reference. Prodrugs are characterized by increased bio-availability and are readily metabolized into the active inhibitors *in vivo.* The term "prodrug", as used herein, means any compound that will be modified to form a drug species, wherein the modification may take place either inside or outside of the body, and either before or after the pre-drug reaches the area of the body where administration of the drug is indicated.

Preferred statements (features) and embodiments of the compounds and processes of this invention are now set forth. Each statement and embodiment of the invention so defined may be combined with any other statement and/or embodiments unless clearly indicated to the contrary. In particular, any feature indicated as being preferred or advantageous may be combined with any other feature or features indicated as being preferred or advantageous.

1. A compound of formula (I) or a stereoisomer, or tautomer thereof,

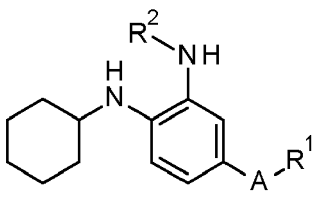

(I)

wherein,

A is selected from

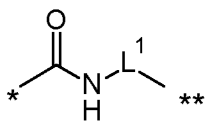

or

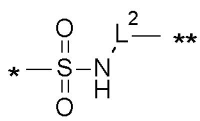

;

wherein * represents where moiety A is bound to the rest of the molecule; and ** represents where moiety A is bound to $R^1$;

$L^1$ is selected from $-(CR^3R^4)_n-$ or

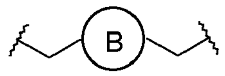

;

$L^2$ is selected from $-(CR^5R^6)_m-$ or

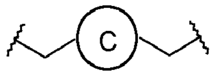

;

n is an integer selected from 1, 2, 3, 4, 5, 6, 7, 8, 9 or 10;

m is an integer selected from 1, 2, 3, 4, 5, 6, 7, 8, 9 or 10;

cycle B is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^B$;

cycle C is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^C$;

$R^1$ is selected from the group consisting of heterocyclyl, $-NR^8R^9$, halogen, halo$C_{1-6}$alkyl, halo$C_{1-6}$alkyloxy, $C_{3-10}$cycloalkyl, $C_{6-12}$aryl, $C_{6-12}$aryl$C_{1-6}$alkyl, heteroaryl, cyano, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said heterocyclyl $C_{3-10}$cycloalkyl, $C_{6-12}$aryl, $C_{6-12}$aryl$C_{1-6}$alkyl, or heteroaryl can be unsubstituted or substituted with one or more $Z^1$;

$R^2$ is selected from the group consisting of $C_{3-10}$cycloalkyl, hydrogen, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;

$R^3$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^3$;

$R^4$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^4$;

$R^5$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^5$;

$R^6$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or $Z^6$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro;

or a solvate, hydrate, pharmaceutically acceptable salt, or prodrug thereof;

wherein when moiety A is $-S(O)_2-(CH_2)_2-$ then $R^2$ is not hydrogen, benzyl or pyridyl;

wherein when moiety A is $-C(O)-(CH_2)_2-$ then $R^2$ is not hydrogen or benzyl;

with the proviso that said compound is not

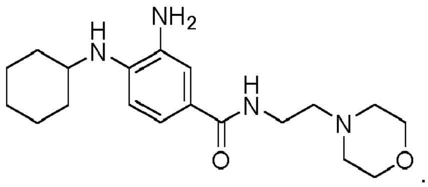

2. The compound according to statement 1, having structural formula (II), (III) or (IV),

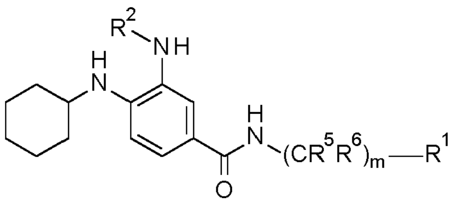

(II)

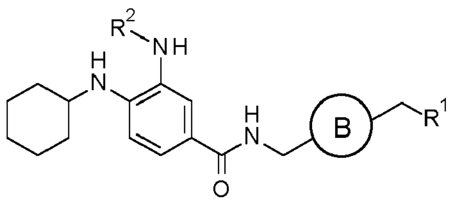

(III)

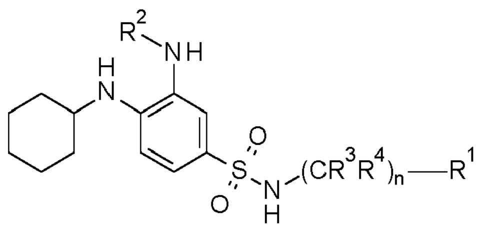

(IV)

wherein n, m, cycle B, $R^1$, $R^2$, $R^3$, $R^4$, $R^5$, $R^6$ have the same meaning as that defined in statement 1.
3. The compound according to statements 1 or 2, having structural formula (IA), (IB), (IC), or (ID)

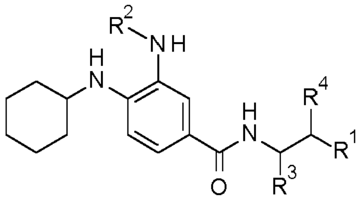

(IA)

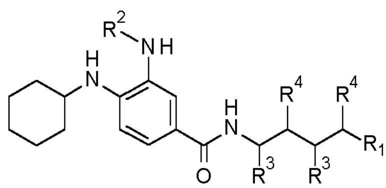

(IB)

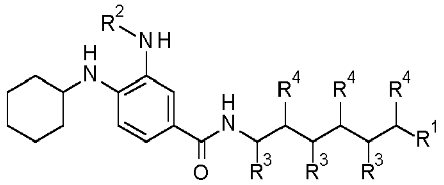 (IC) 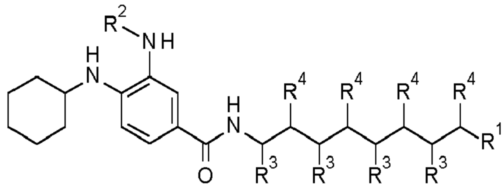 (ID),

wherein $R^1$, $R^2$, $R^3$ and $R^4$ have the same meaning as that defined in statement 1.

4. The compound according to any one of the previous statements, having structural formula (IVA), (IVB), (IVC), or (IVD)

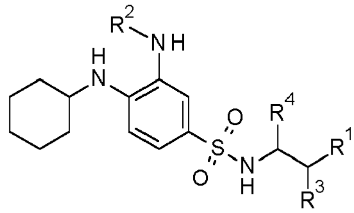 (IVA) 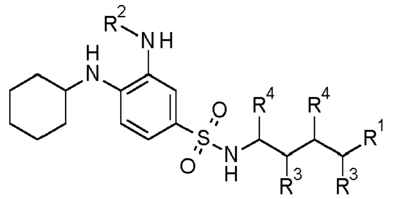 (IVB)

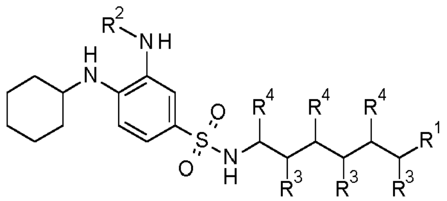 (IVC) 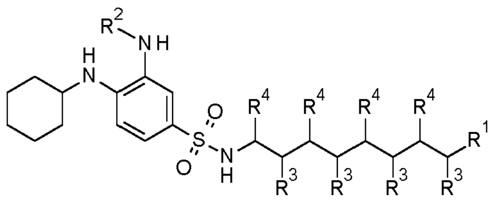 (IVD),

wherein $R^1$, $R^2$, $R^3$ and $R^4$ have the same meaning as that defined in statement 1.

5. The compound according to any one of the previous statements wherein A is

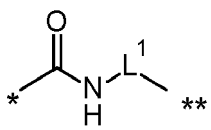

wherein * represents where moiety A is bound to the rest of the molecule; and ** represents where moiety A is bound to $R^1$; and

$L^1$ is-$(CR^3R^4)_n$-.

6. The compound according to any one of the previous statements wherein A is

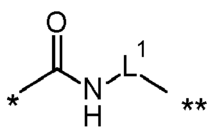

wherein * represents where moiety A is bound to the rest of the molecule and ** represents where moiety A is bound to $R^1$; and

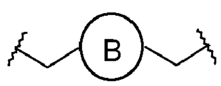.

7. The compound according to any one of the previous statements wherein A is

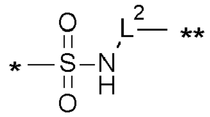

wherein * represents where moiety A is bound to the rest of the molecule; and ** represents where moiety A is bound to $R^1$; and

$L^1$ is $-(CR^5R^6)_m-$.

8. The compound according to any one of the previous statements wherein n is an integer selected from 2, 3, 4, 5, 6, 7, 8 or 9.

9. The compound according to any one of the previous statements wherein m is an integer selected from 2, 3, 4, 5, 6, 7, 8 or 9.

10. The compound according to any one of the previous statements wherein cycle B is selected the group consisting of $C_{6-12}$aryl, $C_{3-10}$cycloalkyl, $C_{3-10}$cycloalkenyl, and heteroaryl, wherein said $C_{6-12}$aryl, $C_{3-10}$cycloalkyl, $C_{3-10}$cycloalkenyl, or heteroaryl can be unsubstituted or substituted with one, two or three $Z^B$.

11. The compound according to any one of the previous statements wherein cycle B is selected the group consisting of $C_{6-12}$aryl and heteroaryl, wherein said $C_{6-12}$aryl or heteroaryl can be unsubstituted or substituted with one, two or three $Z^B$.

12. The compound according to any one of the previous statements wherein cycle C is selected the group consisting of $C_{6-12}$aryl, $C_{3-10}$cycloalkyl, $C_{3-10}$cycloalkenyl, and heteroaryl, wherein said $C_{6-12}$aryl, $C_{3-10}$cycloalkyl, $C_{3-10}$cycloalkenyl, or heteroaryl can be unsubstituted or substituted with one, two or three $Z^C$.

13. The compound according to any one of the previous statements wherein cycle C is selected the group consisting of $C_{6-12}$aryl and heteroaryl, wherein said $C_{6-12}$aryl or heteroaryl can be unsubstituted or substituted with one, two or three $Z^C$.

14. The compound according to any one of the previous statements wherein $R^1$ is selected from the group consisting of heterocyclyl, $-NR^8R^9$, halogen, halo$C_{1-6}$alkyl, halo$C_{1-6}$alkyloxy, $C_{3-10}$cycloalkyl, $C_{6-12}$aryl, $C_{6-12}$aryl$C_{1-6}$alkyl, heteroaryl, cyano, $-NR^8R^9S(O)_2R^{10}$, - $NR^8R^9C(O)_2R^{10}$, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, di-$C_{1-4}$akylamino, di-$C_{3-10}$cycloakylamino, di-$C_{6-12}$arylamino, $C_{1-4}$akylcarbonyl, $C_{3-10}$cycloakylcarbonyl, $C_{6-12}$arylcarbonyl, $-S(O)_2H$ and $C_{1-4}$akylsulfonyl, wherein said heterocyclyl $C_{3-10}$cycloalkyl, $C_{6-12}$aryl, $C_{6-12}$aryl$C_{1-6}$alkyl, or heteroaryl can be unsubstituted or substituted with one, two or three $Z^1$.

15. The compound according to any one of the previous statements wherein $R^1$ is selected from the group consisting of heterocyclyl, $-NR^8R^9$, halogen, halo$C_{1-6}$alkyl, $C_{3-10}$cycloalkyl, $C_{6-12}$aryl, heteroaryl, $-NR^8R^9S(O)_2R^{10}$, $-NR^8R^9C(O)_2R^{10}$, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, $C_{1-4}$akylcarbonyl, $C_{3-10}$cycloakylcarbonyl, $C_{6-12}$arylcarbonyl, wherein said heterocyclyl $C_{3-10}$cycloalkyl, $C_{6-12}$aryl, or heteroaryl can be unsubstituted or substituted with one, two or three $Z^1$.

16. The compound according to any one of the previous statements wherein $R^1$ is selected from the group consisting of heterocyclyl, $-NR^8R^9$, halogen, $C_{3-8}$cycloalkyl, $C_{6-12}$aryl, heteroaryl, $-NR^8R^9C(O)_2R^{10}$, $C_{6-12}$aryloxycarbonyl, mono-$C_{3-10}$cycloakylaminocarbonyl, $C_{3-10}$cycloakylcarbonyl, $C_{6-12}$arylcarbonyl, wherein said heterocyclyl $C_{3-10}$cycloalkyl, $C_{6-12}$aryl, or heteroaryl can be unsubstituted or substituted with one, two or three $Z^1$.

17. The compound according to any one of the previous statements wherein $R^2$ is selected from the group consisting of $C_{3-10}$cycloalkyl, hydrogen, $C_{6-12}$aryl$C_{1-6}$alkyl, $C_{3-10}$cycloalkenyl, $C_{6-12}$aryl, heterocyclyl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{6-12}$aryl, heterocyclyl, heterocyclyl$C_{1-6}$alkyl, or heteroaryl$C_{1-6}$alkyl can be unsubstituted or substituted with one, two or three $Z^2$.

18. The compound according to any one of the previous statements wherein $R^2$ is selected from the group consisting of $C_{3-8}$cycloalkyl, hydrogen, $C_{6-12}$aryl$C_{1-6}$alkyl, $C_{3-8}$cycloalkenyl, $C_{6-12}$aryl, heterocyclyl$C_{1-6}$alkyl, and heteroaryl$C_{1-6}$alkyl, wherein said $C_{3-8}$cycloalkyl, $C_{6-12}$aryl$C_{1-6}$alkyl, $C_{3-8}$cycloalkenyl, $C_{6-12}$aryl, heterocyclyl$C_{1-6}$alkyl, or heteroaryl$C_{1-6}$alkyl can be unsubstituted or substituted with one, two or three $Z^2$.

19. The compound according to any one of the previous statements wherein $R^2$ is selected from the group consisting of $C_{3-8}$cycloalkyl, hydrogen, $C_{6-12}$aryl$C_{1-6}$alkyl, heterocyclyl$C_{1-6}$alkyl, wherein said $C_{3-8}$cycloalkyl, $C_{6-12}$aryl$C_{1-6}$alkyl, heterocyclyl$C_{1-6}$alkyl, can be unsubstituted or substituted with one, two or three $Z^2$.

20. The compound according to any one of the previous statements wherein $R^3$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; wherein said $C_{1-6}$alkyl, $C_{6-12}$aryl$C_{1-6}$alkyl, heterocyclyl$C_{1-6}$alkyl, and heteroaryl$C_{1-6}$alkyl can be unsubstituted or substituted with one, two or three $Z^3$.

21. The compound according to any one of the previous statements wherein $R^3$ is selected from the group consisting of hydrogen, halogen, $C_{1-6}$alkyl, $C_{6-12}$aryl$C_{1-6}$alkyl; wherein said $C_{1-6}$alkyl, $C_{6-12}$aryl$C_{1-6}$alkyl, can be unsubstituted or substituted with one, two or three $Z^3$.

22. The compound according to any one of the previous statements wherein $R^4$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; wherein said $C_{1-6}$alkyl, $C_{6-12}$aryl$C_{1-6}$alkyl, heterocyclyl$C_{1-6}$alkyl, and heteroaryl$C_{1-6}$alkyl can be unsubstituted or substituted with one, two or three $Z^4$.

23. The compound according to any one of the previous statements wherein $R^4$ is selected from the group consisting of hydrogen, halogen, $C_{1-6}$alkyl, $C_{6-12}$aryl$C_{1-6}$alkyl; wherein said $C_{1-6}$alkyl, $C_{6-12}$aryl$C_{1-6}$alkyl, can be unsubstituted or substituted with one, two or three $Z^4$.

24. The compound according to any one of the previous statements wherein $R^5$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; wherein said $C_{1-6}$alkyl, $C_{6-12}$aryl$C_{1-6}$alkyl, heterocyclyl$C_{1-6}$alkyl, and heteroaryl$C_{1-6}$alkyl can be unsubstituted or substituted with one, two or three $Z^5$.

25. The compound according to any one of the previous statements wherein $R^5$ is selected from the group consisting of hydrogen, halogen, $C_{1-6}$alkyl, $C_{6-12}$aryl$C_{1-6}$alkyl; wherein said $C_{1-6}$alkyl, $C_{6-12}$aryl$C_{1-6}$alkyl, can be unsubstituted or substituted with one, two or three $Z^5$.

26. The compound according to any one of the previous statements wherein $R^6$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; wherein said $C_{1-6}$alkyl, $C_{6-12}$aryl$C_{1-6}$alkyl, heterocyclyl$C_{1-6}$alkyl, and heteroaryl$C_{1-6}$alkyl can be unsubstituted or substituted with one, two or three $Z^6$.

26. The compound according to any one of the previous statements wherein $R^6$ is selected from the group consisting of hydrogen, halogen, $C_{1-6}$alkyl, $C_{6-12}$aryl$C_{1-6}$alkyl; wherein said $C_{1-6}$alkyl, $C_{6-12}$aryl$C_{1-6}$alkyl, can be unsubstituted or substituted with one, two or three $Z^6$.

28. The compound according to any one of the previous statements, wherein each $R^8$ is independently selected from the group consisting of $C_{1-6}$alkyl, hydrogen, $C_{6-12}$aryl, and $C_{3-10}$cycloalkyl.

29. The compound according to any one of the previous statements, wherein each $R^9$ is independently selected from the group consisting of $C_{1-6}$alkyl, hydrogen, $C_{6-12}$aryl, and $C_{3-10}$cycloalkyl.

30. The compound according to any one of the previous statements, wherein each $R^{10}$ is independently selected from the group consisting of $C_{1-6}$alkyl, hydrogen, $C_{6-12}$aryl, and $C_{3-10}$cycloalkyl.

31. The compound according to any one of the previous statements, wherein each $Z^B$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, $-S(O)_2H$, $C_{1-4}$akylsulfonyl, $-SO_2NH_2$, mono-$C_{1-4}$akylaminosulfonyl, di-$C_{1-4}$akylaminosulfonyl nitro.

32. The compound according to any one of the previous statements, wherein each $Z^B$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl, nitro.

33. The compound according to any one of the previous statements, wherein each $Z^B$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl.

34. The compound according to any one of the previous statements, wherein each $Z^B$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

35. The compound according to any one of the previous statements, wherein each $Z^B$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbo-

nyl, mono-Ca-$_{10}$cycloakylaminocarbonyl, $C_{1-4}$akylcarbonyl.

36. The compound according to any one of the previous statements, wherein each $Z^B$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

37. The compound according to any one of the previous statements, wherein each $Z^B$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

38. The compound according to any one of the previous statements, wherein each $Z^C$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

39. The compound according to any one of the previous statements, wherein each $Z^C$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl, nitro.

40. The compound according to any one of the previous statements, wherein each $Z^C$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl.

41. The compound according to any one of the previous statements, wherein each $Z^C$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

42. The compound according to any one of the previous statements, wherein each $Z^C$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

43. The compound according to any one of the previous statements, wherein each $Z^C$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

44. The compound according to any one of the previous statements, wherein each $Z^C$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

45. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

46. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akyl-

sulfinyl, $-S(O)_2H$, $C_{1-4}$akylsulfonyl, $-SO_2NH_2$, mono-$C_{1-4}$akylaminosulfonyl, nitro.

47. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl.

48. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

49. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-Ca-$_{10}$cycloakylaminocarbonyl, $C_{1-4}$akylcarbonyl.

50. The compound according to any one of the previous statements, wherein each $Z^1$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

51. The compound according to any one of the previous statements, wherein each $Z^1$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

52. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylam inocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, $-S(O)_2H$, $C_{1-4}$akylsulfonyl, $-SO_2NH_2$, mono-$C_{1-4}$akylaminosulfonyl, di-$C_{1-4}$akylaminosulfonyl nitro.

53. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl, nitro.

54. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl.

55. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

56. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-Ca-$_{10}$cycloakylaminocarbonyl, $C_{1-4}$akylcarbonyl.

57. The compound according to any one of the previous statements, wherein each $Z^2$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

58. The compound according to any one of the previous statements, wherein each $Z^2$ is independently selected from

the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

29. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylam inocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

60. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl, nitro.

61. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl.

62. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

63. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

64. The compound according to any one of the previous statements, wherein each $Z^3$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

65. The compound according to any one of the previous statements, wherein each $Z^3$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

66. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylam inocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

67. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl, nitro.

68. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxy-

carbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl.

69. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

70. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

71. The compound according to any one of the previous statements, wherein each $Z^4$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

72. The compound according to any one of the previous statements, wherein each $Z^4$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

73. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylam inocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -$S(O)_2H$, $C_{1-4}$akylsulfonyl, -$SO_2NH_2$, mono-$C_{1-4}$akylaminosulfonyl, di-$C_{1-4}$akylaminosulfonyl nitro.

74. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl, nitro.

75. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl.

76. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

77. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-Ca-$_{10}$cycloakylaminocarbonyl, $C_{1-4}$akylcarbonyl.

78. The compound according to any one of the previous statements, wherein each $Z^5$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

79. The compound according to any one of the previous statements, wherein each $Z^5$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

80. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-

$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylam inocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S$(O)_2$H, $C_{1-4}$akylsulfonyl, -$SO_2NH_2$, mono-$C_{1-4}$akylaminosulfonyl, di-$C_{1-4}$akylaminosulfonyl nitro.

81. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -S$(O)_2$H, $C_{1-4}$akylsulfonyl, -$SO_2NH_2$, mono-$C_{1-4}$akylaminosulfonyl, nitro.

82. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -S$(O)_2$H, $C_{1-4}$akylsulfonyl, -$SO_2NH_2$, mono-$C_{1-4}$akylaminosulfonyl.

83. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

84. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

85. The compound according to any one of the previous statements, wherein each $Z^6$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

86. The compound according to any one of the previous statements, wherein each $Z^6$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

87. The compound according to any one of the previous statements, wherein

cycle B is selected the group consisting of $C_{6-12}$aryl, $C_{3-10}$cycloalkyl, heterocyclyl, and heteroaryl, wherein said $C_{6-12}$aryl, $C_{3-10}$cycloalkyl, heterocyclyl or heteroaryl can be unsubstituted or substituted with one or more $Z^B$;
cycle C is selected the group consisting of $C_{6-12}$aryl, $C_{3-10}$cycloalkyl, heterocyclyl, and heteroaryl, wherein said $C_{6-12}$aryl, $C_{3-10}$cycloalkyl, heterocyclyl or heteroaryl can be unsubstituted or substituted with one or more $Z^C$;
R' is selected from the group consisting of heterocyclyl, -$NR^8R^9$, $C_{3-10}$cycloalkyl, $C_{6-12}$aryl, $C_{6-12}$aryl$C_{1-6}$alkyl, heteroaryl, cyano, -$NR^8R^9S(O)_2R^{10}$, -$NR^8R^9C(O)_2R^{10}$, wherein said heterocyclyl $C_{3-10}$cycloalkyl, $C_{6-12}$aryl, $C_{6-12}$aryl$C_{1-6}$alkyl, or heteroaryl can be unsubstituted or substituted with one or more $Z^1$;
$R^2$ is selected from the group consisting of $C_{3-10}$cycloalkyl, hydrogen, $C_{6-12}$aryl$C_{1-6}$alkyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;
$R^3$ is selected from the group consisting of hydrogen, halogen, hydroxyl, amino, $C_{1-6}$alkyl; wherein said $C_{1-6}$alkyl can be unsubstituted or substituted with one or more $Z^3$;
$R^4$ is selected from the group consisting of hydrogen, halogen, hydroxyl, amino, $C_{1-6}$alkyl; wherein said $C_{1-6}$alkyl can be unsubstituted or substituted with one or more $Z^4$;
$R^5$ is selected from the group consisting of hydrogen, halogen, hydroxyl, amino, $C_{1-6}$alkyl; wherein said $C_{1-6}$alkyl can be unsubstituted or substituted with one or more $Z^5$;
$R^6$ is selected from the group consisting of hydrogen, halogen, hydroxyl, amino, $C_{1-6}$alkyl; wherein said $C_{1-6}$alkyl can be unsubstituted or substituted with one or more $Z^6$.

88. A pharmaceutical composition comprising a compound of formula (I) according to any one of the previous statements and a pharmaceutically acceptable carrier.

89. A compound of formula (I) according to any one of statements 1 to 87, or a pharmaceutical composition according

to statement 88, for use as a medicament.

90. A compound of formula (I) according to any one of statement 1 to 87, or a pharmaceutical composition according to statement 88, for use in the prevention or treatment of a disease associated with ferroptosis and/or oxytosis.

91. A compound, or its stereoisomer, or tautomer, or solvate, hydrate, pharmaceutically acceptable salt, or prodrug thereof, wherein said compound is

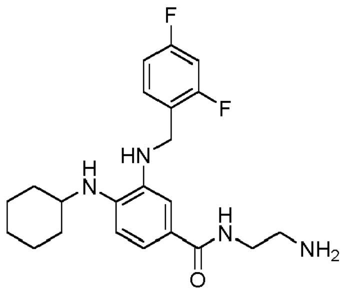

,

for use as a medicament for oral or parenteral, preferably parenteral administration.

92. A compound, or its stereoisomer, or tautomer, or solvate, hydrate, pharmaceutically acceptable salt, or prodrug thereof, wherein said compound is

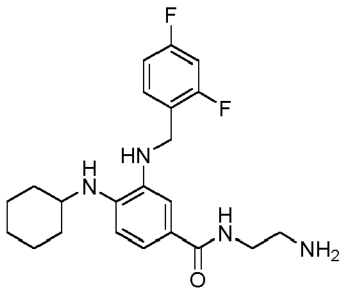

,

for use as a medicament for parenteral administration in the prevention or treatment of a disease associated with ferroptosis and/or oxytosis.

93. The compound for use according to statements 90 or 92, wherein the disease associated with ferroptosis and/or oxytosis is selected from the group consisting of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.

94. The compound for use according to statement 93, wherein the liver disease is selected from hemochromatosis, primary biliary cholangitis, non-alcoholic steatohepatitis or liver fibrosis.

95. The compound for use according to statement 93, wherein the neurological disease is selected from Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

96. The compound for use according to statement 93, wherein the ischemia-reperfusion injury is selected from myocardial ischemia-reperfusion injury, liver ischemia-reperfusion injury, renal ischemia-reperfusion injury, intestinal ischemia-reperfusion injury, cerebral ischemia-reperfusion injury, lung ischemia- reperfusion injury, or any surgical ischemia-reperfusion injury.

97. The compound for use according to statement 93, wherein the iron metabolism-related disease is selected from atherosclerosis or diabetes.

98. The compound for use according to statement 93, wherein the lung disease is selected from acute respiratory distress syndrome (ARDS), lung diseases caused by infections such as COVID-19 pulmonary disease, mucoviscidosis, or asthma.

99. The compound for use according to statements 90 or 92 or 93, wherein the disease is attributable to a genetic disorder of GPX4, preferably the disease is Sedaghatian-type spondylometaphyseal dysplasia.

100. A method of prevention and/or of treatment of a disease associated with ferroptosis and/or oxytosis, said method comprising administering to a subject in need thereof an effective amount of a compound according to any one of statements 1 to 87, or a pharmaceutical composition according to statement 88.

101. A method of prevention and/or of treatment of a disease associated with ferroptosis and/or oxytosis, said method comprising parenterally administering to a subject in need thereof an effective amount of a compound or its stereoisomer, or tautomer, or solvate, hydrate, pharmaceutically acceptable salt, or prodrug thereof, wherein said compound is

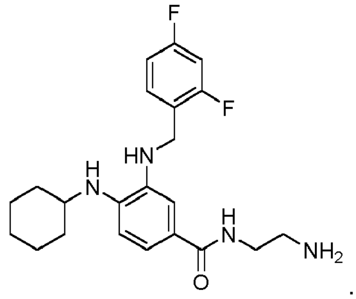

102. The method according to statements 100 or 101, wherein the disease associated with ferroptosis and/or oxytosis is selected from the group consisting of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.
103. The method according to statement 102, wherein the liver disease is selected from hemochromatosis, primary biliary cholangitis, non-alcoholic steatohepatitis or liver fibrosis.
104. The method according to statement 102, wherein neurological disease is selected from Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.
105. The method according to statement 102, wherein the ischemia-reperfusion injury is selected from myocardial ischemia-reperfusion injury, liver ischemia-reperfusion injury, renal ischemia-reperfusion injury, intestinal ischemia-reperfusion injury, cerebral ischemia-reperfusion injury, lung ischemia- reperfusion injury, or any surgical ischemia-reperfusion injury.
106. The method according to statement 102, wherein the iron metabolism-related disease is selected from atherosclerosis or diabetes.
107. The method according to statement 102, wherein the lung disease is selected from acute respiratory distress syndrome (ARDS), lung diseases caused by infections such as COVID-19 pulmonary disease, mucoviscidosis, or asthma.
108. The method according to statement 102, wherein the disease is attributable to a genetic disorder of GPX4, preferably the disease is Sedaghatian-type spondylometaphyseal dysplasia.

The present invention provides a compound of formula (I) or a stereoisomer, or tautomer thereof,

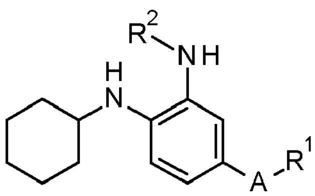

(I)

wherein,

A is selected from

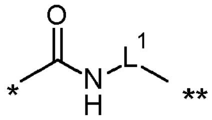

or

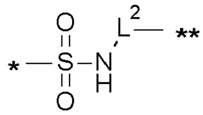

;

wherein * represents where moiety A is bound to the rest of the molecule; and ** represents where moiety A is bound to $R^1$;

$L^1$ is selected from $-(CR^3R^4)_n-$ or

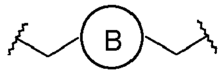

;

$L^2$ is selected from $-(CR^5R^6)_m-$ or

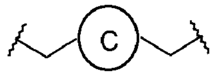

;

n is an integer selected from 1, 2, 3, 4, 5, 6, 7, 8, 9 or 10; preferably n is 2, 3, 4, 5, 6, 7, 8 or 9;

m is an integer selected from 1, 2, 3, 4, 5, 6, 7, 8, 9 or 10; preferably m is 2, 3, 4, 5, 6, 7, 8 or 9;

cycle B is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl; preferably cycle B is selected the group consisting of $C_{6-12}$aryl and $C_{3-9}$cycloalkyl;

wherein said groups can be unsubstituted or substituted with one or more $Z^B$; preferably said groups can be unsubstituted or substituted with one, two or three $Z^B$;

cycle C is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl; preferably cycle C is selected the group consisting of $C_{6-12}$aryl and $C_{3-9}$cycloalkyl;

wherein said groups can be unsubstituted or substituted with one or more $Z^C$; preferably said groups can be unsubstituted or substituted with one, two or three $Z^C$;

R' is selected from the group consisting of heterocyclyl, $-NR^8R^9$, halogen, halo$C_{1-6}$alkyl, haloC1-6alkyloxy, $C_{3-10}$cycloalkyl, C6-12aryl, $C_{6-12}$aryl$C_{1-6}$alkyl, heteroaryl, cyano, - $NR^8R^9S(O)_2R^{10}$, $-NR^8R^9C(O)_2R^{10}$, $-C(O)R^{10}$, $-C(O)_2R^{10}$, $-S(O)_2R^{10}$; preferably $R^1$ is selected from the group consisting of heterocyclyl, $-NR^8R^9$, halogen, halo$C_{1-6}$alkyl, $C_{3-10}$cycloalkyl, $C_{6-12}$aryl, heteroaryl, cyano, $-NR^8R^9S(O)_2R^{10}$, $-N_R{}^8{}_R{}^9C(O)_2R^{10}$; preferably $R^1$ is selected from the group consisting of heterocyclyl, $-NR^8R^9$, halogen, $C_{3-8}$cycloalkyl, $C_{6-12}$aryl, heteroaryl, cyano, $-NR^8R^9C(O)_2R^{10}$;

wherein said groups can be unsubstituted or substituted with one or more $Z^1$; preferably said groups can be unsubstituted or substituted with one, two or three $Z^1$;

$R^2$ is selected from the group consisting of $C_{3-10}$cycloalkyl, hydrogen, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl; preferably $R^2$ is selected from the group consisting of hydrogen, $C_{3-10}$cycloalkyl, $C_{6-12}$aryl$C_{1-6}$alkyl, $C_{3-10}$cycloalkenyl, $C_{6-12}$aryl, heterocyclyl, heterocyclyl$C_{1-6}$alkyl, and heteroaryl$C_{1-6}$alkyl; preferably $R^2$ is selected from the group consisting of hydrogen, $C_{3-s}$cycloalkyl, $C_{6-12}$aryl$C_{1-6}$alkyl, $C_{3-8}$cycloalkenyl, heterocyclyl, heterocyclyl$C_{1-6}$alkyl, and heteroaryl-$C_{1-6}$alkyl; preferably $R^2$ is selected from the group consisting of hydrogen, $C_{3-s}$cycloalkyl, $C_{6-12}$aryl$C_{1-6}$alkyl, heterocyclyl, heterocyclyl$C_{1-6}$alkyl, and heteroaryl$C_{1-6}$alkyl; wherein said groups can be unsubstituted or substituted with one or more $Z^2$; preferably said groups can be unsubstituted or substituted with one, two or three $Z^2$;

$R^3$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; preferably $R^3$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; preferably $R^3$ is selected from the group consisting of hydrogen, halogen, $C_{1-6}$alkyl, $C_{6-12}$aryl$C_{1-6}$alkyl;

wherein said groups can be unsubstituted or substituted with one or more $Z^3$; preferably said groups can be unsubstituted or substituted with one, two or three $Z^3$;

$R^4$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; preferably $R^4$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; preferably $R^4$ is selected from the group consisting of hydrogen, halogen, $C_{1-6}$alkyl, $C_{6-12}$aryl$C_{1-6}$alkyl;

wherein said groups can be unsubstituted or substituted with one or more $Z^4$; preferably said groups can be

unsubstituted or substituted with one, two or three $Z^4$;

$R^5$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; preferably $R^5$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; preferably $R^5$ is selected from the group consisting of hydrogen, halogen, $C_{1-6}$alkyl, $C_{6-12}$aryl$C_{1-6}$alkyl;

wherein said groups can be unsubstituted or substituted with one or more $Z^5$; preferably said groups can be unsubstituted or substituted with one, two or three $Z^5$;

$R^6$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; preferably $R^6$ is selected from the group consisting of hydrogen, halogen, hydroxyl, amino, $C_{1-6}$alkyl, $C_{6-12}$aryl$C_{1-6}$alkyl, heterocyclyl$C_{1-6}$alkyl, and heteroaryl$C_{1-6}$alkyl; preferably $R^6$ is selected from the group consisting of hydrogen, halogen, $C_{1-6}$alkyl, $C_{6-12}$aryl$C_{1-6}$alkyl;

wherein said groups can be unsubstituted or substituted with one or $Z^6$; preferably said groups can be unsubstituted or substituted with one, two or three $Z^6$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro; preferably each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, $C_{3-8}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-S(O)R^{10}$, $-S(O)_2R^{10}$, $-S(O)_2NR^8R^9$, nitro; preferably each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, hydroxyl, $-OR^{10}$, cyano, amino, $-NR^8R^9$, $-C(O)_2R^{10}$, $-C(O)NR^8R^9$, $-S(O)_2R^{10}$, $-S(O)_2NR^8R^9$;

or a solvate, hydrate, pharmaceutically acceptable salt, or prodrug thereof;

wherein when moiety A is $-S(O)_2-(CH_2)_2-$ then $R^2$ is not hydrogen, benzyl or pyridyl;

wherein when moiety A is $-C(O)-(CH_2)_2-$ then $R^2$ is not hydrogen or benzyl;

with the proviso that said compound is not

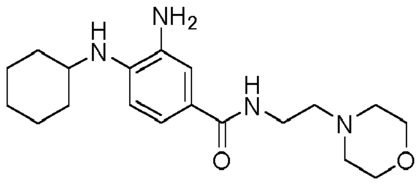

.

The present invention also provides a compound or its stereoisomer, or tautomer, or solvate, hydrate, pharmaceutically acceptable salt, or prodrug thereof, wherein said compound is

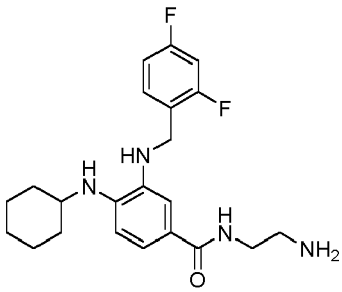

,

for use as a medicament for parenteral administration.

The present invention also provides a compound or its stereoisomer, or tautomer, or solvate, hydrate, pharmaceutically acceptable salt, or prodrug thereof, wherein said compound is

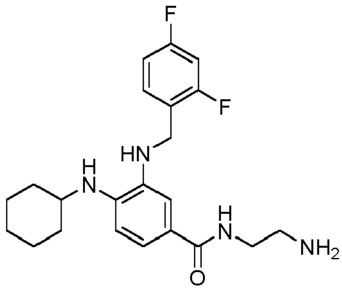

,

for use as a medicament for parenteral administration in the prevention or treatment of a disease associated with ferroptosis and/or oxytosis. Particularly preferred compounds of the invention are those compounds listed in Table 1.

Table 1.

| Compound code | Structure |
|---|---|
| CPD-001 | 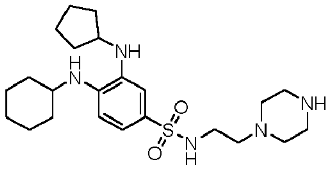 |
| CPD-002 | 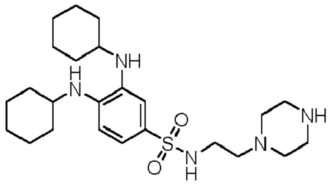 |
| CPD-003 | 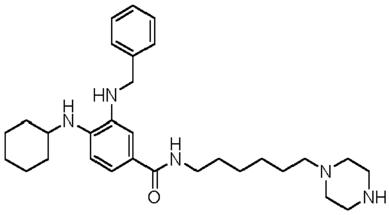 |
| CPD-004 | 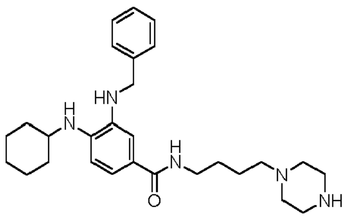 |
| CPD-005 | 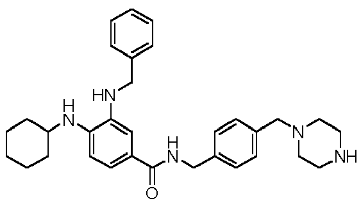 |

(continued)

| Compound code | Structure |
|---|---|
| CPD-006 | 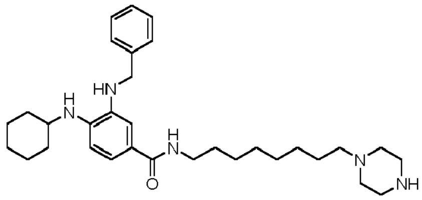 |
| CPD-007 | 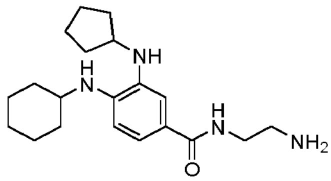 |
| CPD-008 | 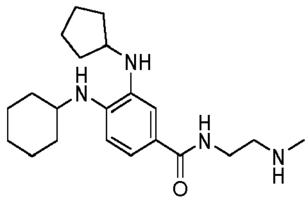 |
| CPD-009 | 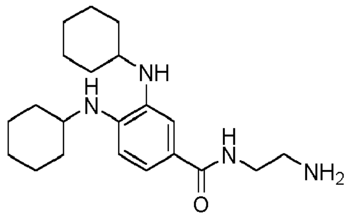 |
| CPD-010 | 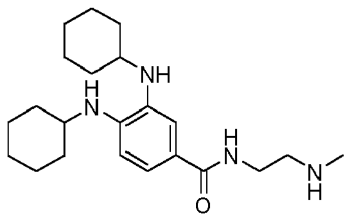 |
| CPD-011 | 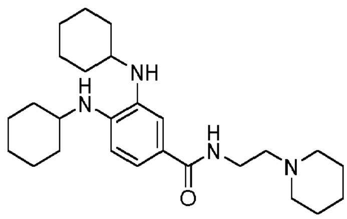 |
| CPD-012 | 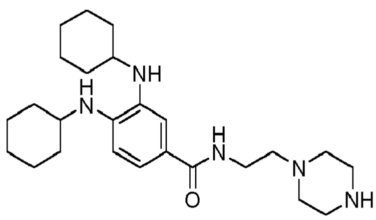 |

(continued)

| Compound code | Structure |
|---|---|
| CPD-013 | 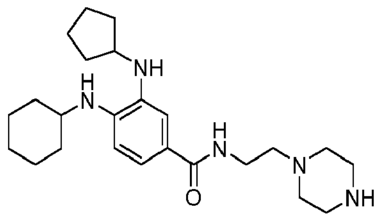 |
| CPD-014 | 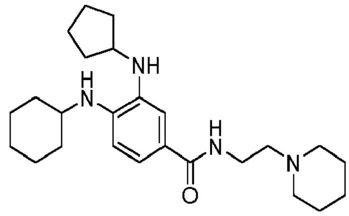 |
| CPD-015 | 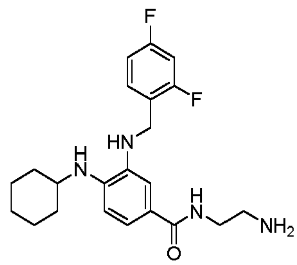 |

The compounds of the present invention have been found to be potent inhibitors of ferroptosis and/or oxytosis. Accordingly, the invention provides for the compounds of the invention for use in therapy. Their use is particularly advantageous in view of their properties making them particularly suited for use *in vivo,* i.e. demonstrating an improved effect *in vivo.*

More particularly, the compounds have been found to have moderate to high solubility .

A number of diseases are characterized by a dysregulation of ferroptosis and/or oxytosis, such as, but not limited to an excess in ferroptosis and/or oxytosis, causing cell-death. Accordingly, the present invention provides compounds of formula (I), and any subgroup thereof such as (I), (II), (II), (IV), (IA), (IB), (IC), (ID), (IVA), (IVB), (IVC), (IVD) for use in the prevention or treatment of a disease associated with ferroptosis and/or oxytosis, more particularly an excess of ferroptosis and/or oxytosis leading to cell-death.

In a particular embodiment the invention provides the compounds of the invention or a pharmaceutical composition comprising one or more compounds of the invention for use in the treatment of a mammal suffering from excessive ferroptosis in one or more organs. The compounds of the invention are of use in a method of treatment or prevention of a disease characterized by a dysregulation of ferroptosis and/or oxytosis, such as, but not limited to an excess in ferroptosis and/or oxytosis, causing cell-death, which comprises administering one or more of the compounds of the invention to a patient suffering from said disease.

In some embodiments the disease associated with ferroptosis and/or oxytosis is selected from the group consisting of liver disease (NASH, NAFLD), lung disease, chronic kidney disease, ocular surface diseases, wound healing, multiple organ dysfunction syndrome, neurological disease, acute renal failure, ischemia-reperfusion injury, sepsis, iron toxicity or iron poisoning, prevention of transplant rejection, and iron metabolism-related disease.

Non-limiting examples of disorders according to the present disclosure include epilepsy, kidney disease, stroke, myocardial infarction, type I diabetes, traumatic brain injury (TBI), periventricular leukomalacia (PVL), and neurological disease. Non-limiting examples of neurological diseases according to the present disclosure include Alzheimer's, Parkinson's, Amyotrophic lateral sclerosis, Friedreich's ataxia, Multiple sclerosis, Huntington's Disease, Transmissible spongiform encephalopathy, Charcot-Marie-Tooth disease, Dementia with Lewy bodies, Corticobasal degeneration, Progressive supranuclear palsy, Chronic Traumatic Encephalopathy (CTE), and Hereditary spastic paraparesis.

Non-limiting examples of liver disease include hemochromatosis, primary biliary cholangitis, non-alcoholic steatohepatitis or liver fibrosis.

Non-limiting examples of neurological disease include Alzheimer's Disease, Parkinson's Disease, Amyotrophic lateral sclerosis, Multiple Sclerosis, Huntington's Disease, Dementia with Lewy bodies, Friedreich's ataxia, multiple

sclerosis, stroke, periventricular leukomalacia, intracerebral haemorrhage, frontotemporal dementia, neurodegeneration with brain iron accumulation, or traumatic brain injury.

Non-limiting examples of ischemia-reperfusion injury include myocardial ischemia-reperfusion injury, liver ischemia-reperfusion injury, lung ischemia-reperfusion injury, intestinal ischemia-reperfusion injury, renal ischemia-reperfusion injury, cerebral ischemia-reperfusion injury or any surgical ischemia-reperfusion injury.

Non-limiting examples of iron metabolism-related disease include atherosclerosis or diabetes.

Non-limiting examples of lung disease include acute respiratory distress syndrome (ARDS), lung diseases caused by infections such as COVID-19 pulmonary disease, mucoviscidosis, or asthma.

Non-limiting examples of genetic disorder of GPX4, for instance a mutation-related disease of GPX4, such as Sedaghatian-type spondylometaphyseal dysplasia. In a particular embodiment, the compounds of the invention are envisaged for use as a medicament for parenteral administration in the treatment or prevention of a disease or disorder such as those described above, wherein the disease or disorder is attributable to a genetic disorder of GPX4, for instance a mutation-related disease of GPX4, such as Sedaghatian-type spondylometaphyseal dysplasia. In particular embodiments, the parenteral administration is intravenous, intraperitoneal, intramuscular, intradermal or subcutaneous. In particular embodiments the administration is intravenous.

In a particular embodiment the invention provides the compounds of the invention or a pharmaceutical composition comprising one or more compounds of the invention for the treatment of a mammal suffering from excessive ferroptosis and/or oxytosis in one or more organs.

In a particular embodiment the invention provides the compounds of the invention or a pharmaceutical composition comprising one or more compounds of the invention for the treatment of diseases caused by excitatory amino acids. A well-known example of an excitatory amino acid is glutamate and the condition is designated as glutamine excitotoxicity.

In a particular embodiment the invention provides the compounds of the invention or a pharmaceutical composition comprising one or more compounds of the invention for the treatment of diseases caused by increased levels of phospholipid peroxides.

In a particular embodiment the invention provides the compounds of the invention or a pharmaceutical composition comprising one or more compounds of the invention for the treatment of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, and iron metabolism-related disease.

In the present invention the potency of a compound inhibiting (or reducing) ferroptosis and/or oxytosis are determined in (bio)chemical antioxidant activity assays, *in vitro* assays. Typically FENIX assay is used to quantify radical trapping antioxidant activity in phospholipid bilayers to predict anti-ferroptotic potency of lipophilic antioxidants in cells. Typically, *in vitro* assays are used to measure the potency of a candidate compound. Examples of suitable *in vitro* assays are cellular assays. One non-limiting example of an assay involves the use of the IMR-32 neuroblastoma cell line. The latter is stimulated to enter ferroptosis upon stimulation with 10$\mu$M erastin, a documented ferroptosis inducer (see for example Dixon et al (2012) Cell 149, 1060-1072 and ferroptosis inhibitors are evaluated for the prevention of erastin induced ferroptosis. Another assay involves the use of ML162-induced ferroptosis in HT1080 human fibrosarcoma cells. Yet another assay is based on the glutamate-induced cell death in the hippocampal cell line HT22 and ferroptosis/oxytosis inhibitors are evaluated for the prevention of cell death (see Henke N. et al (2013) Cell Death and Disease 4, e470). Still another assay is based on the sorafenib induced cell death (described to be iron dependent cell death) in hepatocellular carcinoma cells and ferroptosis inhibitors are evaluated for the prevention of cell death (see Louandre C. et al (2013) Int. J. Cancer 133, 1732). The calculated potency of a compound inhibiting ferroptosis and/or oxytosis is typically depicted as an IC50 value. Examples of suitable *in vivo* assays are typically pre-clinical disease models of for example mice for the diseases benefiting the application of ferroptosis and/or oxytosis inhibitors, as described herein.

One non-limiting example of an *in vivo* assay is based on inducing organ injury by iron overload or using liver, kidney, lung, brain or intestine-specific Gpx4-deficient mouse lines and ferroptosis inhibitors are evaluated based on the level of reduction in plasma injury biomarkers LDH, CK, AST and ALT, and body temperature (see Van Coillie et al. (2022) Nat Comm 13: 1046).

The compounds of this invention can be administered as the sole pharmaceutical agent or in combination with one or more other pharmaceutical agents where the combination causes no unacceptable adverse effects. The present invention relates also to such combinations. For example, the compounds of this invention can be combined with known therapeutic agents for the treatment of diseases mentioned herein, as well as with admixtures and combinations thereof. Particularly preferred combinations are necroptosis inhibitors (e.g. necrostatin-1) and ferroptosis inhibitors. Examples of these combinations are described in Linkermann *et al* 2014.

The compounds of the invention may be in the form of salts, preferably pharmaceutically acceptable salts, as generally described below. Some preferred, but non-limiting examples of suitable pharmaceutically acceptable organic and/or inorganic acids are as hydrochloric acid, trifluoroacetic acid (or triflate), hydrobromic acid, sulfuric acid, nitric acid,

acetic acid and citric acid, as well as other pharmaceutically acceptable acids known per se (for which reference is made to the prior art referred to below).

When the compounds of the invention contain an acidic group as well as a basic group the compounds of the invention may also form internal salts, and such compounds are within the scope of the invention. When the compounds of the invention contain a hydrogen-donating heteroatom (e.g. NH), the invention also covers salts and/or isomers formed by transfer of said hydrogen atom to a basic group or atom within the molecule.

Pharmaceutically acceptable salts of the compounds of formula (I) and any subgroup thereof include the acid addition and base salts thereof. Suitable acid addition salts are formed from acids which form non-toxic salts. Examples include the acetate, adipate, aspartate, benzoate, besylate, bicarbonate/carbonate, bisulfate/sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate (or triflate) and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002), incorporated herein by reference.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically, such materials do not give distinctive X-ray diffraction patterns and, while exhibiting the properties of a solid, are more formally described as a liquid. Upon heating, a change from solid to liquid properties occurs which is characterized by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterized by a phase change, typically first order ('melting point').

Pharmaceutically acceptable salts of compounds of formula (I) may be prepared by one or more of these methods:

(i) by reacting the compound of formula (I) with the desired acid;

(ii) by reacting the compound of formula (I) with the desired base;

(iii) by removing an acid- or base-labile protecting group from a suitable precursor of the compound of formula (I) or by ring-opening a suitable cyclic precursor, for example, a lactone or lactam, using the desired acid; or

(iv) by converting one salt of the compound of formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Britain, Marcel Dekker, 1995), incorporated herein by reference. Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

Also included within the scope of the invention are multi-component complexes (other than salts and solvates)

wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallization, by recrystallization from solvents, or by physically grinding the components together - see Chem Commun, 17, 1889-1896, by O. Almarsson and M. J. Zaworotko (2004), incorporated herein by reference. For a general review of multi-component complexes, see J Pharm Sci, 64 (8), 1269-1288, by Haleblian (August 1975), incorporated herein by reference.

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as - $COO^-Na^+$, $-COO^-K^+$, or $-SO_3^-Na^+$) or non-ionic (such as $-N^-N^+(CH_3)_3$) polar head group. For more information, see Crystals and the Polarizing Microscope by N. H. Hartshorne and A. Stuart, 4th Edition (Edward Arnold, 1970), incorporated herein by reference.

All references to compounds of formula (I) or any subgroups thereof include references to salts, solvates, multi-component complexes and liquid crystals thereof and to solvates, multi-component complexes and liquid crystals of salts thereof.

The compounds of the invention include compounds of formula (I) or any subgroups thereof as hereinbefore defined, including all polymorphs and crystal habits thereof, prodrugs and isomers thereof (including optical, geometric and tautomeric isomers) as hereinafter defined and isotopically-labelled compounds of formula (I).

In addition, although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention.

A further related aspect of the present invention provides a pharmaceutical composition comprising a compound of formula (I) or a stereoisomer, or tautomer thereof, and a pharmaceutically acceptable carrier.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic compositions may be contemplated.

Illustrative, non-limiting carriers for use in formulating the pharmaceutical compositions include, for example, oil-in-water or water-in-oil emulsions, aqueous compositions with or without inclusion of organic co-solvents suitable for intravenous (IV) use, liposomes or surfactant-containing vesicles, microspheres, microbeads and microsomes, powders, tablets, capsules, suppositories, aqueous suspensions, aerosols, and other carriers apparent to one of ordinary skill in the art.

Pharmaceutical compositions as intended herein may be formulated for essentially any route of administration, such as without limitation, oral administration (such as, e.g., oral ingestion or inhalation), intranasal administration (such as, e.g., intranasal inhalation or intranasal mucosal application), parenteral administration (such as, e.g., subcutaneous, intravenous (I.V.), intramuscular, intraperitoneal or intrasternal injection or infusion), transdermal or transmucosal (such as, e.g., oral, sublingual, intranasal) administration, topical administration, rectal, vaginal or intra-tracheal instillation, and the like. In this way, the therapeutic effects attainable by the methods and compositions can be, for example, systemic, local, tissue-specific, etc., depending of the specific needs of a given application.

In preferred embodiments, the compound or the pharmaceutical composition as taught herein is administered parenterally. More preferably, the compound or the pharmaceutical composition as taught herein is administered intravenously, for example by infusion.

The dosage or amount of the agent as taught herein, optionally in combination with one or more other active compounds to be administered, depends on the individual case and is, as is customary, to be adapted to the individual circumstances to achieve an optimum effect. Thus, the unit dose and regimen depend on the nature and the severity of the disorder to be treated, and also on factors such as the species of the subject, the sex, age, body weight, general health, diet, mode and time of administration, immune status, and individual responsiveness of the human or animal to be treated, efficacy, metabolic stability and duration of action of the compounds used, on whether the therapy is acute or chronic or

prophylactic, or on whether other active compounds are administered in addition to the agent of the invention. In order to optimize therapeutic efficacy, the compound or the pharmaceutical composition as taught herein can be first administered at different dosing regimes. Typically, levels of the agent in a tissue can be monitored using appropriate screening assays as part of a clinical testing procedure, e.g., to determine the efficacy of a given treatment regimen. The frequency of dosing is within the skills and clinical judgement of medical practitioners (e.g., doctors, veterinarians or nurses). Typically, the administration regime is established by clinical trials which may establish optimal administration parameters. However, the practitioner may vary such administration regimes according to the one or more of the aforementioned factors, e.g., subject's age, health, weight, sex and medical status. The frequency of dosing can be varied depending on whether the treatment is prophylactic or therapeutic.

Toxicity and therapeutic efficacy of the agent as described herein or pharmaceutical compositions comprising the same can be determined by known pharmaceutical procedures in, for example, cell cultures or experimental animals. These procedures can be used, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Pharmaceutical compositions that exhibit high therapeutic indices are preferred. While pharmaceutical compositions that exhibit toxic side effects can be used, care should be taken to design a delivery system that targets such compounds to the site of affected tissue in order to minimize potential damage to normal cells (e.g., non-target cells) and, thereby, reduce side effects.

The data obtained from the cell culture assays and animal studies can be used in formulating a range of dosage for use in appropriate subjects. The dosage of such pharmaceutical compositions lies generally within a range of circulating concentrations that include the ED50 with little or no toxicity. The dosage may vary within this range depending upon the dosage form employed and the route of administration utilised. For a pharmaceutical composition used as described herein, the therapeutically effective dose can be estimated initially from cell culture assays. A dose can be formulated in animal models to achieve a circulating plasma concentration range that includes the IC50 (i.e., the concentration of the pharmaceutical composition which achieves a half-maximal inhibition of symptoms) as determined in cell culture. Such information can be used to more accurately determine useful doses in humans. Levels in plasma can be measured, for example, by high performance liquid chromatography.

In particular embodiment, the compound as taught herein is the main or only active ingredient of the pharmaceutical composition.

## Examples

The following examples are provided for the purpose of illustrating the present invention and by no means should be interpreted to limit the scope of the present invention.

### 1. Chemical synthesis of representative compounds of the invention

Unless otherwise stated, laboratory reagent grade solvents were used. Reagents were obtained from various commercial sources and were used without any prior purification.

Characterisation of all compounds was done with $^{1}H$ and $^{13}C$ NMR and mass spectrometry. NMR spectra were recorded with a 400 MHz Bruker Avance III Nanobay spectrometer with Ultrashield. All obtained spectra were analysed using MestReNova analytical chemistry software. Chemical shifts are displayed in ppm and coupling constants are shown in hertz (Hz). ES mass spectra were obtained from an Esquire 3000plus Ion Trap Mass Spectrometer from Bruker Daltonics.

The UPLC (ultra performance liquid chromatography), used to quantify the purity of the products, was an ACQUITY UPLC H-Class system with a TUV detector Waters coupled to an MS detector Waters Qda. Waters Acquity UPLC BEH C18 1.7 $\mu$m, 2.1 mm $\times$ 50 mm column was used. The eluent was composed of two different solvents. Solvent C consisted of water with 0.1% formic acid, solvent D was acetonitrile. For most of the experiments, unless stated otherwise, the general method was used. The column was first equilibrated for 0.15 min with a mixture of 95% solvent C and 5% solvent D. After that, solvent D was increased linearly to 100% over 1.75 min before being held constant for 0.25 min, followed by a mixture of 95% solvent C and 5% solvent D for 0.75 min (flow rate 0.7 ml/min). All mass spectra were recorded over a *m*/*z* range of 100-1000. The wavelength for UV detection was 254 nm. Method II starts with equilibration of column for 0.15 min with a mixture of 95% solvent C and 5% solvent D. After that, solvent D was increased linearly to 100% over 2.50 min before being held constant for 0.75 min, followed by a mixture of 95% solvent C and 5% solvent D for 0.75 min (flow rate 0.7 ml/min).

Selected compounds of the invention were analyzed by high resolution mass spectrometry: 10$\mu$L of each sample (concentration = $10^{-5}$ M) was injected using the CapLC system (Waters, Manchester, UK) and electrosprayed using a standard electrospray source. Samples were injected with an interval of 5 min. Positive ion mode accurate mass spectra were acquired usinga Q-TOF II instrument (Waters, Man-chester, UK). The MS was calibrated prior to use with a 0.2%

$H_3PO_4$ solution. The spectra were lock mass corrected using the known mass of the nearest $H_3PO_4$ cluster.

During the chemical synthesis, flash purification was performed when necessary, on a Biotage ISOLERA One flash system equipped with an internal variable dual wavelength diode array detector (200-400 nm). Reverse phase purifications were done using Buchi EcoFlex C18 cartridges, dry sample loading was done by self-packing sample cartridges using Celite® 545. Gradients used varied for each purification. Several synthesis procedures that were used in the preparation of intermediates and final products are summarized here as "General Procedures".

The final products have a purity above 95% unless otherwise stated.

*Scheme 1.*

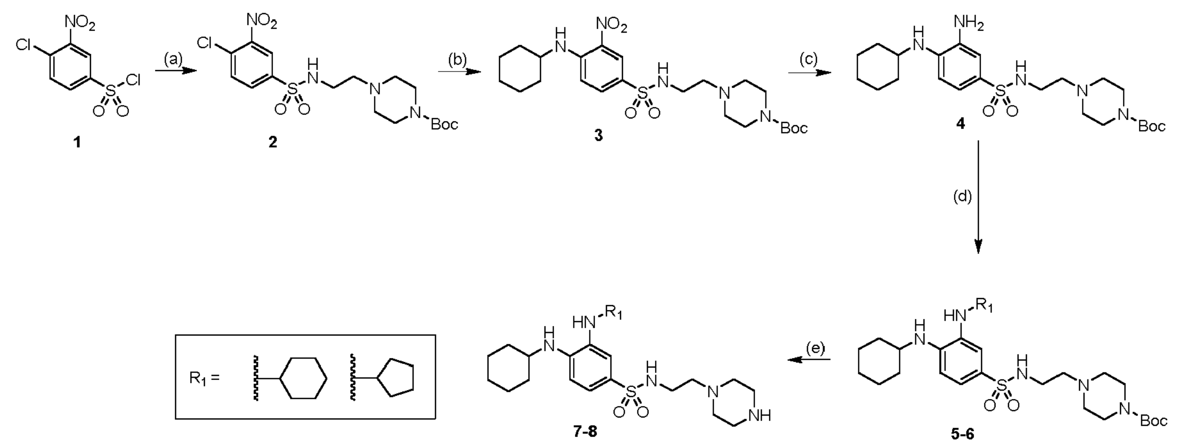


*Scheme 2.*

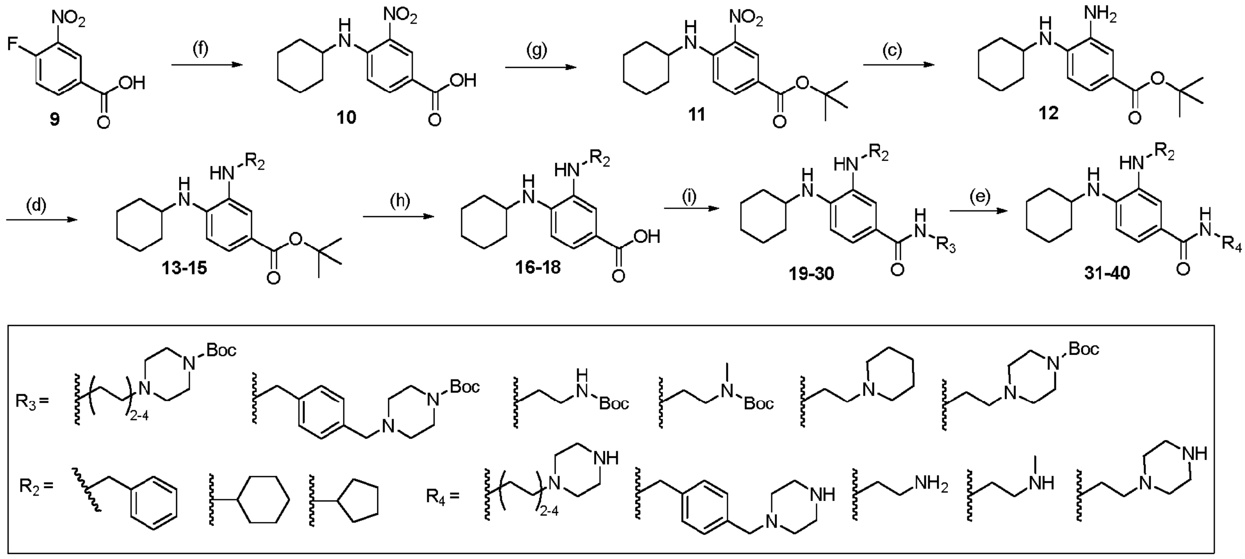


<u>General procedure A: (sulfone)amide coupling (Scheme 1, step (a))</u>

In a round-bottom flask 3-chloro-4-nitrobenzenesulfonyl chloride **1** (1.0 eq) was dissolved in THF. After cooling down to -40°C a solution containing the appropriate aliphatic amine substituent (1.0 eq) and triethylamine (2.0 eq) in THF was added dropwise. After the addition was complete, the resulting mixture was allowed to warm up to room temperature over the course of 1 hour. Subsequently, the reaction mixture was diluted with EtOAc (20-50 ml) and washed with water (3 × 10-50 mL). The organic layer was dried using anhydrous sodium sulfate before being concentrated *in vacuo*. The crude reaction products **2** was used without any further purification for the next step.

General procedure B: nucleophilic aromatic substitution (Scheme 1, step (b))

The intermediate **2** was dissolved in ACN (20-75 mL), and *N,N*-diisopropylethylamine (3.0 eq) and cyclohexylamine (3.0 eq) were added to the reaction mixture. It was then heated to 90°C and stirred overnight. After the consumption of the starting material, the mixture was cooled to room temperature, and the solvent was concentrated under vacuum. It was further diluted with water and EtOAc. The aqueous layer was extracted with EtOAc (3 × 10-50 mL). The collected organic layers were washed with brine (20 mL), then dried using anhydrous sodium sulfate, and concentrated under vacuum. The residue was either directly used for the subsequent step without purification or purified by reversed-phase flash column chromatography (water/MeOH) to obtain the intermediate **3.**

General procedure C: catalytic hydrogenation (Schemes 1 or 2 step (c))

Intermediate **3** and **11** (1.0 eq) were dissolved in MeOH and the solution was purged with argon. Palladium hydroxide on carbon (20% $Pd(OH)_2/C$, 50% of water, 0.1 eq) was added under inert atmosphere while continuously stirring. Next, the reaction mixture was put under hydrogen atmosphere and stirred for 12-72 hours at room temperature. After completion of the reaction, the solution was filtered through a patch of Celite® and the filtrate was concentrated under reduced pressure. The crude compound was purified by reversed-phase flash chromatography (water/MeOH) to yield products **4** and **12.**

General procedure D: reductive amination (Schemes 1 or 2 step (d))

Compounds **4** or **12** (1.0 eq.) were dissolved in THF, followed by the addition of acetic acid (23.0 eq.) and the corresponding aldehyde or ketone (1.5 eq.). After 15 minutes, sodium cyanoborohydride (2.5 eq.) was added and the reaction mixture was stirred at room temperature for 12-16 hours. After completion, the reaction was quenched with water and the product was extracted twice into DCM. The combined organic layers were dried over sodium sulphate, filtered and loaded on Celite®. The crude compounds were purified by reversed-phase flash chromatography (water/MeOH) to yield products **5-6** or **13-15.**

General procedure E: Boc deprotection (Schemes 1 and 2 step (e))

Compounds **5-6, 19-26 or 28-29** (1.0 eq.) were dissolved in dichloromethane followed by the addition of a 4 M solution of hydrochloric acid in dioxane (15 eq.) The reaction mixture was stirred at room temperature for 2 hours. After reaction, diethyl ether was added to the reaction mixture to precipitate compounds **7-8** or **31-40.** The obtained HCl salts were subsequently washed with a minimal amount of diethyl ether.

General procedure F: nucleophilic aromatic substitution (Scheme 2 step (f))

To a stirred solution of compounds 4-fluoro-3-nitrobenzoic acid **9** (1.0 eq) in ACN, DIPEA (3.0 eq) was added, followed by the addition of cyclohexylamine (3.0 eq). The reaction mixture was stirred for 72-96 hours at room temperature. After completion of the reaction, the yellow precipitate was filtered, washed with water and dried under vacuum, providing compounds 4-(cyclohexylamino)-3-nitrobenzoic acid **10** which was used for the next step without further purification.

General procedure G: esterification (Scheme 2 step (g))

To a stirred solution of 4-(cyclohexylamino)-3-nitrobenzoic acid **10** (1.0 eq) in THF, DIPEA (3.0 eq) and DMAP (1.05 eq) were added followed by the addition of di-*t*-butyldicarbonate (2.5 eq). The reaction mixture was refluxed for 72-96 hours. Then, the reaction mixture was directly loaded on Celite® and the crude compound was purified by flash chromatography (Hep/EtOAc) to yield *tert*-butyl 4-(cyclohexylamino)-3-nitrobenzoate **11.**

General procedure H: t-Bu ester hydrolysis (Scheme 2 step (h))

To a stirred solution of compounds **13-15** (1.0 eq.) in DCM, trifluoroacetic acid (10 eq.) was added and the reaction mixture was stirred 16 hours at room temperature. After completion, acetonitrile was added to the reaction mixture to precipitate compounds **16-18** obtained as trifluoroacetate salts.

General procedure I: amide coupling (Scheme 2 step (i))

A stirred solution of compound **16-18** (1.0 eq) in THF was cooled to 0°C in an ice bath, followed by the addition of

HATU (1.4 eq), DIPEA (3.0 eq) and the corresponding amine (1.5 eq). The reaction mixture was stirred for 16 hours at room temperature. After completion, the reaction mixture was directly loaded on Celite® without any additional work-up. The crude compound was purified by reverse-phase flash chromatography (water/MeOH) to yield products **19-30.**

*tert*-butyl 4-(2-((4-chloro-3-nitrophenyl)sulfonamido)ethyl)piperazine-1-carboxylate (**2**)

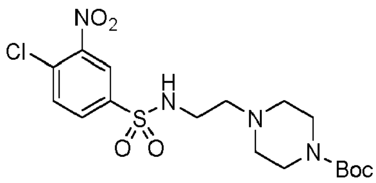

General procedure A was followed using compound **1** (2.000 g, 7.810 mmol) to obtain compound **2** (3.249 g, 7.240 mmol, 93 %).

**$^1$H NMR** (400 MHz, DMSO-$d_6$) δ 1.98 (s, 9H), 2.21 - 2.23 (m, 4H), 2.32 - 2.34 (m, 2H), 2.97 (m, 2H), 3.18 - 3.21 (m, 4H), 8.02 (s, 1H), 8.03 (d, 1H, *J* = 8.5 Hz), 8.08 (dd, 1H, *J* = 8.5 Hz, *J* = 2.1 Hz), 8.45 (d, 1H, *J* = 2.1 Hz).

**MS (ESI)** *m*/*z* = 449.2 $[M+H]^+$.

*tert*-butyl 4-(2-((4-(cyclohexylamino)-3-nitrophenyl)sulfonamido)ethyl)piperazine-1-carboxylate (**3**)

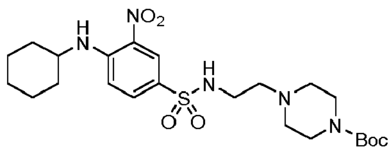

General procedure B was followed using compound **2** (3.249 g, 7.240 mmol) to obtain compound **3** (3.700 g, 7.240 mmol, 100 %).

**$^1$H NMR** (400 MHz, DMSO-$d_6$) δ 1.38 (s, 9H), 1.40 - 1.47 (m, 5H), 1.56 - 1.65 (m, 1H), 1.68 - 1.77 (m, 2H), 1.92 - 1.99 (m, 2H), 2.22 (t, 4H, *J* = 5.0 Hz), 3.73 (d, 1H, *J* = 9.3 Hz), 7.22 (s, 1H), 7.33 (d, 1H, *J* = 9.3 Hz), 7.81 (ddd, 1H, *J* = 9.3, 2.3, 0.7 Hz), 8.29 (d, 1H, *J* = 7.8 Hz), 8.44 (d, 1H, *J* = 2.2 Hz).

**MS (ESI)** *m*/*z* = 512.3 $[M+H]^+$.

*tert*-butyl 4-(2-((3-amino-4-(cyclohexylamino)phenyl)sulfonamido)ethyl)piperazine-1-carboxylate (**4**)

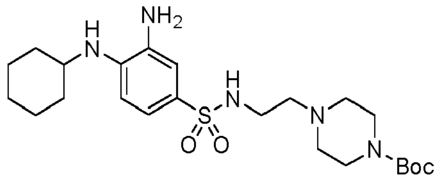

General procedure C was followed using compound **3** (3.906 g, 7.630 mmol) to obtain compound **4** (3.097 g, 6.430 mmol, 84 %).

**$^1$H NMR** (400 MHz, DMSO-$d_6$) δ 1.12 - 1.27 (m, 3H), 1.29 - 1.36 (m, 2H), 1.39 (s, 9H), 1.58-1.68 (m, 1H), 1.68 - 1.78 (m, 2H), 1.90 - 2.01 (m, 2H), 2.21 (t, *J* = 5.3 Hz, 4H), 2.30 (dd, *J* = 7.7, 6.2 Hz, 2H), 2.77 (dt, *J* = 7.7, 6.0 Hz, 2H), 3.24 (t, *J* = 5.1 Hz, 4H), 3.27 - 3.32 (m, 1H), 4.85 (d, *J* = 7.5 Hz, 1H), 4.95 (s, 2H), 6.50 (d, *J* = 9.0 Hz, 1H), 6.81 (t, *J* = 5.8 Hz, 1H), 6.93 (dq, *J* = 4.3, 2.2 Hz, 2H).

**MS (ESI)** *m*/*z* = 482.4 $[M+H]^+$.

*tert*-butyl 4-(2-((4-(cyclohexylamino)-3-(cyclopentylamino)phenyl)sulfonamido)ethyl)piperazine-1-carboxylate (**5**)

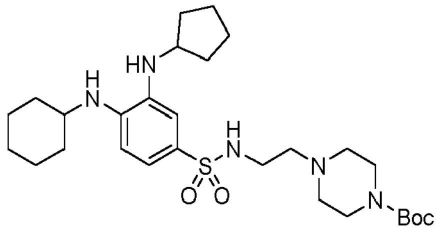

General procedure D was followed using compound **4** (0.200 g, 0.415 mmol) to afford compound **5** (0.025 g, 0.045 mmol, 11 % yield).

**$^1$H NMR** (400 MHz, DMSO-$d_6$) δ 1.16 - 1.25 (m, 3H), 1.31 - 1.35 (m, 1H), 1.38 (s, 9H), 1.41 (s, 1H), 1.45 - 1.76 (m, 10H), 1.96 - 2.03 (m, 4H), 2.17 - 2.20 (m, 4H), 2.28 (t, *J* = 6.3 Hz, 2H), 2.75 (q, *J* = 6.0 Hz, 2H), 3.22 (m, 4H), 3.65 - 3.73 (m, 1H), 4.86 (d, , *J* = 5.6 Hz, 1H), 5.07 (d, *J* = 7.3 Hz, 1H), 6.49 (d, *J* = 8.5 Hz, 1H), 6.77 (d, *J* = 2.1 Hz, 1H), 6.91 - 6.97 (m, 2H).

**$^{13}$C NMR** (101 MHz, DMSO-$d_6$) δ 24.39, 25.28, 26.08, 28.50, 32.89, 33.00, 40.61, 51.40, 52.78, 54.58, 56.99, 79.16, 107.63, 108.11, 117.48, 126.19, 135.07, 138.84, 154.23.

**MS (ESI)** *m/z* =550.4 $[M+H]^+$.

*tert*-butyl 4-(2-((3,4-bis(cyclohexylamino)phenyl)sulfonamido)ethyl)piperazine-1-carboxylate (**6**)

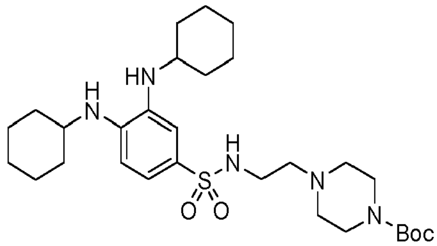

General procedure D was followed using compound **4** (0.200 g, 0.415 mmol) to afford compound **6** (0.039 g, 0.069 mmol, 17 % yield).

**$^1$H NMR** (400 MHz, DMSO-$d_6$) δ 1.13 - 1.25 (m, 7H), 1.31 - 1.38 (m, 3H), 1.39 (s, 9H), 1.41 (s, 1H), 1.62 - 1.65 (m, 2H), 1.74 - 1.75 (m, 4H), 1.98 (m, 4H), 2.19 - 2.29 (m, 5H), 2.76 (s, 2H), 3.19 - 3.20 (m, 4H), 3.27 - 3.29 (m, 2H), 4.73 (s, 1H), 5.09 (s, 1H), 6.50 (d, 1H, *J* = 8.5 Hz), 6.76 (d, 1H; *J* = 2.2 Hz), 6.94 (dd, 1H, *J* = 8.5 Hz, *J* = 2.0 Hz).

**$^{13}$C NMR** (101 MHz, DMSO-$d_6$) δ 25.26, 25.38, 26.07, 26.16, 28.47, 32.98, 33.04, 51.41, 51.68, 107.97, 109.77, 117.39, 122.82, 134.37, 138.92, 153.63.

**MS (ESI)** *m/z* =564.4 $[M+H]^+$.

4-(cyclohexylamino)-3-(cyclopentylamino)-N-(2-(piperazin-1-yl)ethyl)benzenesulfonamide tetrahydrochloride (**7, CPD-001**)

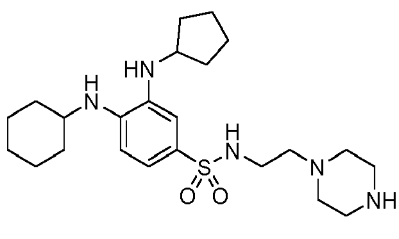

General procedure E was followed using compound **5** (0.250 g, 0.045 mmol) to afford compound **7** (0.015 g, 0.033 mmol, 73 % yield). The reported NMR spectrum refers to tetrahydrochoride salt.

**$^1$H NMR** (400 MHz, DMSO-$d_6$) δ 1.18 - 1.22 (m, 1H), 1.27 - 1.38 (m, 4H), 1.53 - 1.62 (m, 4H), 1.71 - 1.75 (m, 6H), 1.87 - 1.97 (m, 4H), 3.10 - 3.14 (m, 3H), 3.23 - 3.27 (m, 3H), 3.37 - 3.46 (m, 5H), 3.82 - 3.87 (m, 2H), 6.90 - 6.93 (m, 1H), 7.36 - 7.42 (m, 2H), 7.86 - 7.89 (m, 1H), 9.79 (s, 2H).

**MS (ESI)** *m/z* =450.4 $[M+H]^+$.

3,4-bis(cyclohexylamino)-N-(2-(piperazin-1-yl)ethyl)benzenesulfonamide tetrahydrochloride (**8, CPD-002**)

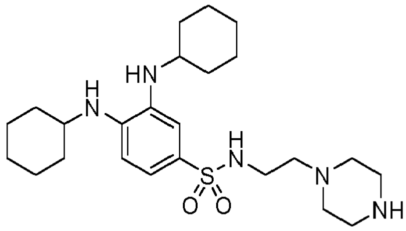

General procedure E was followed using compound **6** (0.160 g, 0.028 mmol) to afford compound **8** (0.013 g,0.028 mmol, 99 %). The reported NMR spectrum refers to tetrahydrochoride salt.

**$^1$H NMR** (400 MHz, DMSO-$d_6$) δ 1.15 - 1.22 (m, 1H), 1.27 - 1.38 (m, 4H), 1.54 - 1.62 (m, 4H), 1.67 - 1.77 (m, 6H), 1.87 - 1.97 (m, 4H), 3.10 - 3.14 (m, 2H), 3.23 - 3.27 (m, 2H), 3.37 - 3.47 (m, 5H), 3.82 - 3.87 (m, 2H), 6.91 - 6.93 (m, 1H), 7.36 - 7.38 (m, 2H), 7.86 - 7.89 (m, 1H), 9.80 (s, 2H).

**MS (ESI)** *m/z* =464.5 $[M+H]^+$.

4-(cyclohexylamino)-3-nitrobenzoic acid (**10**)

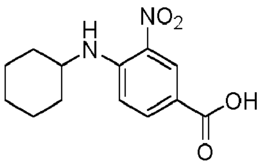

General procedure F was followed using compound **9** (2.250 g, 12.15 mmol) to provide crude compound **10** (3.210 g, 12.15 mmol, 100 % yield). The compound was used for the next step without further purification.

**$^1$H NMR (400 MHz, $CDCl_3$)** δ 1.18 - 1.60 (m, 5H), 1.61 - 1.78 (m, 1H), 1.78 - 1.96 (m, 2H), 2.14 (d, *J* = 22.7 Hz, 2H), 3.54 - 3.67 (m, 1H), 6.93 (d, *J* = 9.2 Hz, 1H), 8.07 (dd, *J* = 2.1, 9.1 Hz, 1H), 8.17 - 8.47 (m, 1H), 8.51 (d, *J* = 7.5 Hz, 1H), 8.98 (d, *J* = 2.1 Hz, 1H).

**$^{13}$C NMR (101 MHz, DMSO-$d_6$)** δ 15.13, 24.47, 25.42, 32.23, 37.96, 51.16, 116.50, 126.53, 130.02, 133.79, 146.32.

**MS (ESI)** m/z = 265.2 $[M+H]^+$.

*tert*-butyl 4-(cyclohexylamino)-3-nitrobenzoate (**11**)

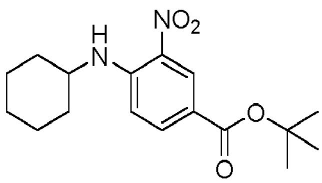

General procedure G was followed using compound **10** (3.210 g, 12.15 mmol) to provide compound **11** (3.750 g, 11.70 mmol, 96 % yield). **$^1$H NMR (400 MHz, MeOD)** δ1.09 - 1.59 (m, 12H), 1.62 - 1.92 (m, 5H), 2.08 (dt, *J* = 3.4, 10.6 Hz, 2H), 3.70 (tdt, *J* = 3.8, 7.4, 10.8 Hz, 1H), 7.08 (d, *J* = 9.1 Hz, 1H), 7.86 - 8.06 (m, 1H), 8.36 (d, *J* = 7.6 Hz, 1H), 8.71 (d, *J* = 2.1

Hz, 1H).

**MS (ESI)** m/z = 265.2 $[M+H]^+$.

*tert*-butyl 3-amino-4-(cyclohexylamino)benzoate (**12**)

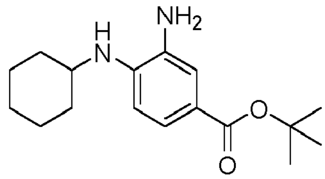

General procedure C was followed using compound **11** (3.750 g, 11.70 mmol) to provide compound **12** (1.230 g, 4.240 mmol, 36 % yield).

**$^1$H NMR (400 MHz, MeOD)** δ 1.19 - 1.35 (m, 3H), 1.37- 1.51 (m, 2H), 1.55 (s, 9H), 1.65 - 1.74 (m, 1H), 1.81 (dt, *J* = 3.7, 13.1 Hz, 2H), 2.03 - 2.13 (m, 2H), 3.32 - 3.39 (m, 1H, ), 6.55 (dd, *J* = 0.6, 8.4 Hz, 1H), 7.30 (d, *J* = 2.0 Hz, 1H), 7.35 (dd, *J* = 2.1, 8.4 Hz, 1H).

**$^{13}$C NMR (101 MHz, MeOD)** δ 26.23, 27.07, 28.61, 34.21, 52.63, 80.89, 110.07, 118.29, 120.19, 124.16, 133.71, 142.39, 168.80.

**MS (ESI)** m/z = 291.2 $[M+H]^+$.

*tert*-butyl 4-(cyclohexylamino)-3-(cyclopentylamino)benzoate (**13**)

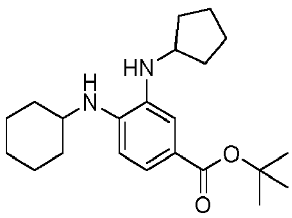

General procedure D was followed using compound **12** (1.000 g, 3.440 mmol) to provide compound **13** (1.150 g, 3.210 mmol, 91% yield).

**$^1$H NMR (400 MHz, MeOD)** δ 1.18 - 1.36 (m, 4H), 1.43 (dtt, *J* = 3.3, 10.4, 12.8 Hz, 2H), 1.50 - 1.55 (m, 1H), 1.56 (s, 9H), 1.60 - 1.73 (m, 3H), 1.72 - 1.86 (m, 4H), 1.96 - 2.12 (m, 4H), 3.32 - 3.37 (m, 1H), 3.78 (ddd, *J* = 5.4, 6.9, 12.4 Hz, 1H), 6.54 (d, *J* = 8.4 Hz, 1H), 7.23 (d, *J* = 2.0 Hz, 1H), 7.35 (dd, *J* = 2.0, 8.4 Hz, 1H).

**$^{13}$C NMR (101 MHz, MeOD)** δ 25.35, 26.31, 27.09, 28.63, 34.14, 34.17, 52.76, 56.37, 80.85, 109.62, 115.10, 120.12, 123.28, 135.64, 142.75, 169.13. **MS (ESI)** m/z =359.0 $[M+H]^+$.

*tert*-butyl 3,4-bis(cyclohexylamino)benzoate (**14**)

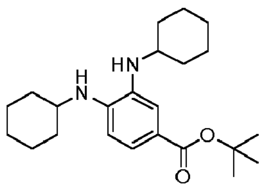

General procedure D was followed using compound **12** (4.740 g, 16.32 mmol) to provide compound **14** (5.520 g, 14.82 mmol, 91% yield).

**$^1$H NMR (400 MHz, MeOD)** δ 1.18 - 1.50 (m, 10H), 1.56 (s, 9H), 1.69 (dt, *J* = 3.5, 12.3 Hz, 2H), 1.75 -1.85 (m, 4H), 2.05 (dt, *J* = 3.9, 12.2 Hz, 4H), 3.15 (tt, *J* = 3.7, 10.5 Hz, 1H), 3.32 - 3.37 (m, 1H), 6.55 (d, *J* = 8.5 Hz, 1H), 7.24 (d, *J* = 2.0 Hz,

1H), 7.35 (dd, *J* = 2.0, 8.4 Hz, 1H).

**$^{13}$C NMR (101 MHz, MeOD)** δ 26.24, 26.44, 27.08, 27.24, 28.61, 34.18, 34.40, 52.70, 54.14, 80.90, 110.04, 116.26, 120.11, 123.68, 134.65, 143.33, 169.05.

**MS (ESI)** m/z =373.0 $[M+H]^+$.

*tert*-butyl 3-(benzylamino)-4-(cyclohexylamino)benzoate (**15**)

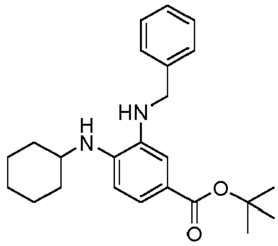

General procedure D was followed using compound **12** (2.000 g, 6.890 mmol) to provide compound **15** (0.780 g, 2.050 mmol, 30% yield).

**$^1$H NMR (400 MHz, $CDCl_3$)** δ 1.28 - 1.47 (m, 12H), 1.67 (dd, *J* = 5.0, 8.8 Hz, 2H), 1.77 (dt, *J* = 3.7, 13.2 Hz, 2H), 1.92 (dd, *J* = 3.9, 12.9 Hz, 1H), 2.06 (dt, *J* = 3.7, 12.1 Hz, 2H), 3.31 (td, *J* = 4.8, 9.5 Hz, 1H), 4.29 (s, 2H), 6.61 (d, *J* = 8.4 Hz, 1H), 7.30 - 7.34 (m, 1H), 7.34 - 7.45 (m, 5H), 7.54 (dd, *J* = 1.9, 8.3 Hz, 1H).

**$^{13}$C NMR (101 MHz, MeOD)** δ 25.83, 26.66, 28.17, 33.78, 49.08, 52.31, 80.38, 109.28, 113.80, 119.82, 122.78, 127.50, 128.24, 129.01, 135.38, 140.80, 141.87, 168.56.

**MS (ESI)** m/z = 381.4 $[M+H]^+$.

4-(cyclohexylamino)-3-(cyclopentylamino)benzoic acid (**16**)

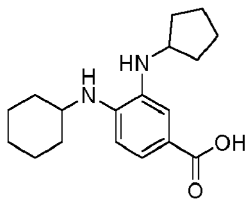

General procedure H was followed using compound **13** (1,348 g, 3.760 mmol) to provide compound**16** (1.093 g, 3.610 mmol, 96 % yield).

**$^1$H NMR (400 MHz, DMSO-*$d_6$*)** δ 1.11 - 1.28 (m, 4H), 1.35 (qt, *J* = 3.2, 12.5 Hz, 2H), 1.47 - 1.78 (m, 8H), 1.85 - 2.01 (m, 4H), 3.31 (ddt, *J* = 3.8, 7.4, 10.6 Hz, 1H), 3.73 (p, *J* = 6.3 Hz, 1H), 6.55 (d, *J* = 8.5 Hz, 1H), 7.18 (s, 1H), 7.32 (d, *J* = 8.4 Hz, 1H). **$^{13}$C NMR (101 MHz, DMSO-*$d_6$*)** δ 23.91, 24.74, 25.57, 31.89, 32.46, 51.03, 55.55, 108.78, 117.50, 122.88, 131.14, 140.26, 167.85.

**MS (ESI)** m/z = 303.2 $[M+H]^+$.

3,4-bis(cyclohexylamino)benzoic acid (**17**)

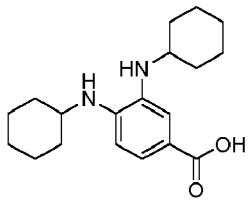

General procedure H was followed using compound **13** (2.940 g, 7.890 mmol) to provide compound **17** (2.219 g, 7.01 mmol, 89 % yield).

**$^1$H NMR (400 MHz, MeOD)** δ 1.14- 1.55 (m, 10H), 1.72 (dt, *J* = 3.6, 13.2 Hz, 2H), 1.78-1.91 (m, 4H), 1.97 - 2.13 (m, 4H), 3.37 - 3.54 (m, 2H), 6.93 (d, *J* = 8.7 Hz, 1H), 7.76 (s, 1H), 7.86 (d, *J* = 8.7 Hz, 1H).

**$^{13}$C NMR (101 MHz, MeOD)** δ 25.68, 26.00, 26.14, 26.77, 31.03, 33.55, 53.23, 60.62, 113.86, 116.53, 119.43, 126.67, 132.03, 145.07, 169.08.

**MS (ESI)** m/z = 317.3 [M+H]$^+$.

3-(benzylamino)-4-(cyclohexylamino)benzoic acid (**18**)

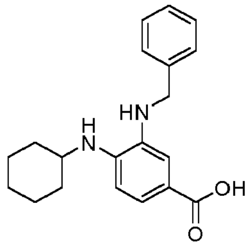

General procedure H was followed using compound **15** (0.724 g, 1.903 mmol) to providecompound **18** (0.617 g, 1.903 mmol, 100 % yield).

**$^1$H NMR (400 MHz, DMSO-*d$_6$*)** δ 1.24 (d, *J*= 4.6 Hz, 2H), 1.36 (dddd, *J* = 3.0, 7.6, 12.3, 15.5 Hz, 2H), 1.56 - 1.65 (m, 1H), 1.72 (dt, *J* = 3.4, 7.6 Hz, 2H), 1.86 (dd, *J* = 4.3, 9.6 Hz, 1H), 1.98 (dd, *J* = 3.6, 12.5 Hz, 2H), 2.90 - 3.04 (m, 1H), 3.33 (td, *J* = 3.3, 6.8 Hz, 1H), 4.31 (s, 2H), 6.53 (d, *J* = 8.4 Hz, 1H), 6.97 (d, *J* = 2.0 Hz, 1H), 7.21 - 7.27 (m, 2H), 7.34 (td, *J* = 6.1, 8.3 Hz, 4H), 7.72 (s, 1H).

**$^{13}$C NMR (101 MHz, DMSO-*d$_6$*)** δ 24.68, 25.57, 32.46, 40.15, 47.27, 51.05, 111.40, 114.43, 117.34, 121.26, 126.83, 127.40, 128.32, 133.69, 136.54, 139.50, 167.91.

**MS (ESI)** m/z = 325.0 [M+H]$^+$.

*tert*-butyl 4-(6-(3-(benzylamino)-4-(cyclohexylamino)benzamido)hexyl)piperazine-1-carboxylate (**19**)

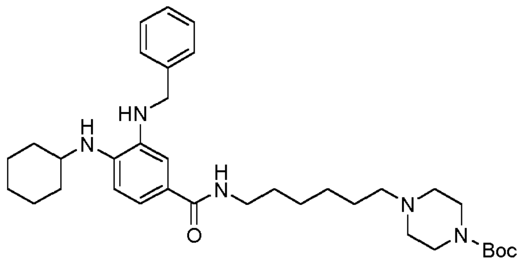

General procedure I was followed using compound **18** (0.200 g, 0.616 mmol) to provide compound **19** (0.074 g, 0.125 mmol, 20 % yield).

**$^1$H NMR(400 MHz, MeOD)** δ 1.19 -1.73 (m, 26H), 1.81 (d, *J* = 13.2 Hz, 2H), 2.08 (d, *J* = 12.2 Hz, 2H), 2.79 - 3.11 (m, 10H), 4.37 (s, 2H), 6.60 (d, *J* = 8.7 Hz, 1H), 7.08 (d, *J* = 2.2 Hz, 1H), 7.17 - 7.25 (m, 2H), 7.28 - 7.35 (m, 2H), 7.37 - 7.42 (m, 2H).

**MS (ESI)** m/z = 592.2 [M+H]$^+$.

*tert*-butyl 4-(4-(3-(benzylamino)-4-(cyclohexylamino)benzamido)butyl)piperazine-1-carboxylate (**20**)

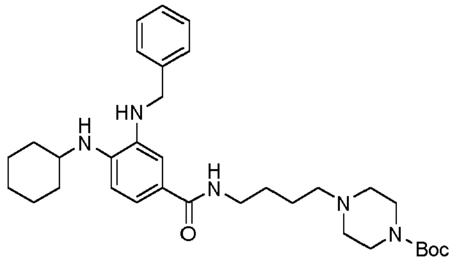

General procedure I was followed using compound **18** (0.200 g, 0.616 mmol) to provide compound **20** (0.074 g, 0.131 mmol, 21 % yield).

**$^1$H NMR(400 MHz, MeOD)** δ 1.20 - 1.73 (m, 21H), 1.76 - 1.84 (m, 2H), 2.08 (d, *J* = 10.1 Hz, 2H), 2.40 (q, *J* = 5.1 Hz, 4H), 3.33 - 3.36 (m, 2H), 3.39 - 3.44 (m, 5H), 4.37 (s, 2H), 6.60 (d, *J* = 8.6 Hz, 1H), 7.09 (d, *J* = 2.1 Hz, 1H), 7.17 - 7.25 (m, 2H), 7.31 (td, *J* = 1.7, 6.6 Hz, 2H), 7.36 - 7.43 (m, 2H).

**$^{13}$C NMR (101 MHz, MeOD)** δ 24.85, 26.27, 27.11, 28.57, 28.64, 34.23, 40.52, 52.88, 53.93, 59.23, 81.28, 110.47, 112.19, 119.98, 123.40, 127.98, 128.76, 129.44, 136.62, 141.03, 141.18, 156.37, 171.21.

**MS (ESI)** m/z = 564.5 [M+H]$^+$.

<u>*tert*-butyl 4-(4-((3-(benzylamino)-4-(cyclohexylamino)benzamido)methyl)benzyl)piperazine-1-carboxylate (**21**)</u>

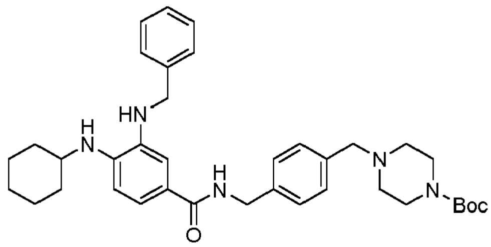

General procedure I was followed using compound **18** (0.199 g, 0.613 mmol) to provide compound **21** (0.021 g, 0.131 mmol, 6 % yield).

**$^1$H NMR(400 MHz, MeOD)** δ 1.13- 1.48 (m, 15H), 1.63 (d, *J* = 12.0 Hz, 1H), 1.74 (dt, *J* = 4.0, 13.2 Hz, 2H), 1.98 - 2.07 (m, 2H), 2.32 (t, *J* = 5.2 Hz, 4H), 3.35 (t, *J* = 5.1 Hz, 4H), 3.44 (d, *J* = 1.3 Hz, 2H), 4.28 (s, 2H), 4.45 (s, 2H), 6.55 (dd, *J* = 1.4, 8.6 Hz, 1H), 7.11 (t, *J* = 1.9 Hz, 1H), 7.14 - 7.28 (m, 8H), 7.32 (d, *J* = 7.8 Hz, 2H).

**$^{13}$C NMR (101 MHz, MeOD)** δ 26.24, 27.08, 28.66, 34.20, 44.06, 52.83, 53.73, 63.53, 81.20, 110.44, 112.36, 120.27, 123.06, 127.94, 128.40, 128.80, 129.41, 130.68, 136.56, 137.02, 139.97, 141.11, 141.20, 156.38, 170.95.

**MS (ESI)** m/z = 612.5 [M+H]$^+$.

<u>*tert*-butyl 4-(8-(3-(benzylamino)-4-(cyclohexylamino)benzamido)octyl)piperazine-1-carboxylate (**22**)</u>

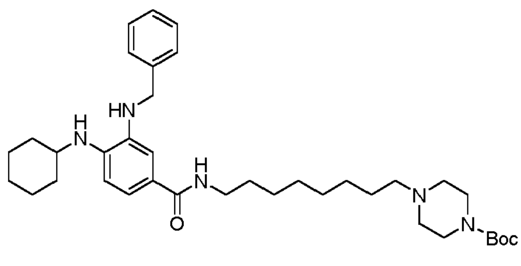

General procedure I was followed using compound **18** (0.280 g, 0.863 mmol) to provide compound **22** (0.018 g, 0.030 mmol, 3 % yield).

**$^1$H NMR(400 MHz, MeOD)** δ 1.19- 1.61 (m, 27H), 1.68 (dt, *J* = 3.5, 12.7 Hz, 1H), 1.79 (dp, *J* = 4.0, 11.2 Hz, 2H), 2.03 - 2.11 (m, 2H), 2.31 - 2.37 (m, 2H), 2.41 (t, *J* = 5.3 Hz, 4H), 3.25 - 3.34 (m, 2H), 3.42 (t, *J* = 5.3 Hz, 4H), 4.35 (s, 2H), 6.59 (d, *J* = 8.6 Hz, 1H), 7.10 (d, *J* = 2.1 Hz, 1H), 7.17 - 7.25 (m, 2H), 7.27 - 7.33 (m, 2H), 7.39 (dd, *J* = 1.3, 8.3 Hz, 2H).

**$^{13}$C NMR (101 MHz, MeOD)** δ 26.27, 27.10, 27.35, 27.95, 28.47, 28.64, 30.31, 30.45, 30.58, 34.23, 40.82, 52.86, 53.94, 59.69, 81.30, 110.45, 112.20, 120.01, 123.43, 127.97, 128.77, 128.79, 129.39, 129.43, 136.58, 140.98, 141.16, 156.33, 171.10.

**MS (ESI)** m/z = 620.5 [M+H]$^+$.

*tert*-butyl (2-(4-(cyclohexylamino)-3-(cyclopentylamino)benzamido)ethyl)carbamate (**23**)

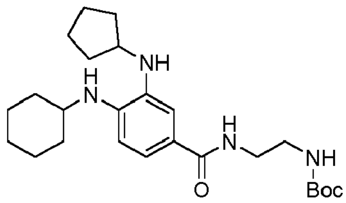

General procedure I was followed using compound **16** (0.200 g, 0.531 mmol) to provide compound **23** (0.093 g, 0.209 mmol, 39 % yield).

**$^1$H NMR (400 MHz, CDCl$_3$)** δ 1.16 - 1.39 (m, 5H), 1.41 (s, 9H), 1.51 - 1.70 (m, 5H), 1.76 (s, 4H), 2.04 (dd, *J* = 6.4, 12.4 Hz, 4H), 3.27 (s, 1H), 3.37 (t, *J* = 5.9 Hz, 2H), 3.53 (d, *J* = 6.8 Hz, 2H), 3.83 (s, 1H), 5.19 (d, *J* = 6.2 Hz, 1H), 6.53 - 6.73 (m, 1H), 7.00 (s, 1H), 7.30 (d, *J* = 29.1 Hz, 2H).

**$^{13}$C NMR (101 MHz, CDCl$_3$)** δ 24.20, 24.97, 25.82, 28.38, 32.98, 40.40, 41.53, 52.17, 79.63, 105.44, 111.01, 114.84, 120.55, 139.72, 157.23, 168.25.

**MS (ESI)** m/z = 455.4 [M+H]$^+$.

*tert*-butyl (2-(4-(cyclohexylamino)-3-(cyclopentylamino)benzamido)ethyl)(methyl)carbamate (**24**)

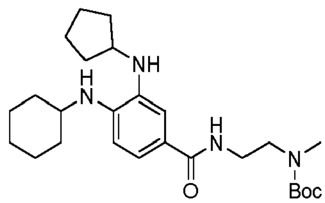

General procedure I was followed using compound **16** (0.135 g, 0.446 mmol) to provide compound **24** (0.015 g, 0.033 mmol, 7 %).

**$^1$H NMR (400 MHz, MeOD)** δ 1.20 - 1.83 (m, 23H), 2.06 (dd, *J* = 3.8, 12.8 Hz, 4H), 2.91 (d, *J* = 6.4 Hz, 3H), 3.26 - 3.35 (m, 1H), 3.43 - 3.52 (m, 4H), 3.79 - 3.90 (m, 1H), 6.56 (d, *J* = 8.3 Hz, 1H), 7.19 (td, *J* = 5.3, 13.7 Hz, 2H).

**$^{13}$C NMR (101 MHz, MeOD)** δ 25.26, 26.32, 27.12, 28.64, 34.14, 34.20, 34.73, 35.44, 38.97, 52.90, 56.23, 81.03, 110.33, 113.31, 119.97, 122.95, 136.38, 141.47, 157.92, 171.12.

**MS (ESI)** m/z = 459.1 [M+H]$^+$.

*tert*-butyl (2-(3,4-bis(cyclohexylamino)benzamido)ethyl)carbamate (**25**)

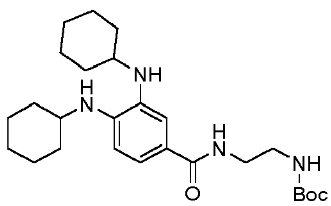

General procedure I was followed using compound **17** (0.200 g, 0.531 mmol) to provide compound **25** (0.162 g,

0.353 mmol, 66 % yield).

**$^1$H NMR (400 MHz, $CDCl_3$)** δ 1.24 (ddt, *J* = 5.0, 9.4, 17.3 Hz, 6H), 1.38 (dt, *J* = 3.2, 12.2 Hz, 3H), 1.44 (s, 9H), 1.67 (dq, *J* = 3.6, 7.4 Hz, 2H), 1.72 - 1.89 (m, 4H), 1.97 - 2.13 (m, 4H), 3.26 (d, *J* = 30.3 Hz, 2H), 3.38 (q, *J* = 5.8 Hz, 2H), 3.54 (t, *J* = 5.6 Hz, 2H), 5.09 (s, 1H), 6.60 (d, *J* = 8.2 Hz, 1H), 6.84 (s, 1H), 7.17 - 7.28 (m, 1H), 7.30 (s, 1H).

**$^{13}$C NMR (101 MHz, $CDCl_3$)** δ 24.93, 24.99, 25.93, 25.98, 28.39, 33.26, 33.51, 40.42, 41.39, 51.54, 53.01, 79.62, 110.26, 115.23, 119.96, 122.61, 141.49, 157.15, 168.39.

**MS (ESI)** m/z = 459.4 [M+H]$^+$.

*tert*-butyl (2-(3,4-bis(cyclohexylamino)benzamido)ethyl)(methyl)carbamate (**26**)

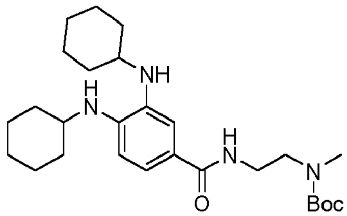

General procedure I was followed using compound **17** (0.150 g, 0.317 mmol) to provide compound **26** (0.150 g, 0.317 mmol, 67 % yield).

**$^1$H NMR (400 MHz, MeOD)** δ 1.17 - 1.30 (m, 6H), 1.33 - 1.47 (m, 13H), 1.67 (ddt, *J* = 4.8, 9.5, 13.3 Hz, 2H), 1.79 (dt, *J* = 4.2, 13.0 Hz, 4H), 2.05 (dd, *J* = 3.7, 12.9 Hz, 4H), 2.91 (d, *J* = 5.7 Hz, 3H), 3.20 - 3.29 (m, 2H), 3.40 - 3.53 (m, 4H), 6.58 (d, *J* = 8.3 Hz, 1H), 7.21 (s, 2H).

**$^{13}$C NMR (101 MHz, MeOD)** δ 26.25, 26.42, 27.11, 27.21, 28.64, 34.20, 34.47, 34.70, 35.43, 39.09, 52.86, 53.77, 81.02, 110.82, 114.17, 120.30, 123.01, 135.47, 141.93, 157.88, 171.02. **MS (ESI)** m/z = 473.3 [M+H]$^+$.

3,4-bis(cyclohexylamino)-N-(2-(piperidin-1-yl)ethyl)benzamide (**27, CPD-011**)

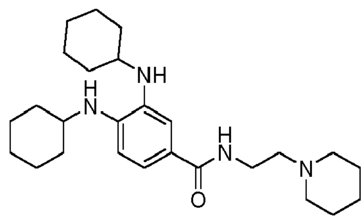

General procedure I was followed using compound **17** (0.150 g, 0.474 mmol) to provide compound **27** (0.097 g, 0.227 mmol, 48 % yield).

**$^1$H NMR(400 MHz, MeOD)** δ 1.25 (tdd, *J* = 3.8, 9.8, 13.4 Hz, 7H), 1.34 - 1.53 (m, 6H), 1.58-1.72 (m, 6H), 1.79 (dt, *J* = 3.8, 13.1 Hz, 5H), 2.06 (dd, *J* = 3.9, 12.8 Hz, 4H), 2.45 - 2.60 (m, 6H), 3.19 - 3.29 (m, 2H), 3.51 (t, *J* = 7.0 Hz, 2H), 6.58 (d, *J* = 8.4 Hz, 1H), 7.17 (d, *J* = 2.1 Hz, 1H), 7.21 (dd, *J* = 2.1, 8.3 Hz, 1H).

**$^{13}$C NMR (101 MHz, MeOD)** δ 25.36, 26.48, 26.66, 26.79, 27.33, 27.44, 34.44, 34.68, 37.94, 53.08, 53.96, 55.77, 59.38, 111.01, 114.15, 120.37, 123.25, 135.67, 142.09, 171.24.

**HRMS** calcd 427.3431; found 427.3441 [M+H]$^+$.

*tert*-butyl 4-(2-(3,4-bis(cyclohexylamino)benzamido)ethyl)piperazine-1-carboxylate (**28**)

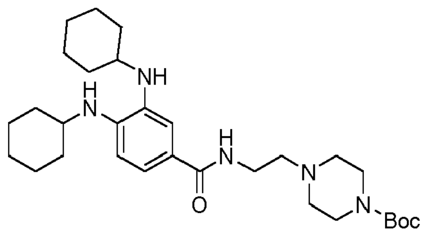

General procedure I was followed using compound **17** (0.150 g, 0.337 mmol) to provide compound **28** (0.025 g, 47.4 mmol, 14 % yield).

**$^1$H NMR (400 MHz, MeOD)** δ 1.16 - 1.34 (m, 8H), 1.34 - 1.43 (s, 4H), 1.43 - 1.49 (s, 9H), 1.64 - 1.73 (m, 2H), 1.74 - 1.85 (dt, *J* = 3.7, 13.3 Hz, 4H), 2.00 - 2.11 (m, 4H), 2.47 - 2.54 (t, *J* = 5.1 Hz, 4H), 2.58 - 2.64 (t, *J* = 6.7 Hz, 2H), 3.19 - 3.27 (m, 2H), 3.41 - 3.47 (m, 4H), 3.48 - 3.54 (t, *J* = 6.7 Hz, 2H), 6.56 - 6.61 (d, *J* = 8.5 Hz, 1H), 7.13 - 7.16 (d, *J* = 2.1 Hz, 1H), 7.16 - 7.21 (dd, *J* = 2.1, 8.3 Hz, 1H).

**$^{13}$C NMR (101 MHz, MeOD)** δ 26.29, 26.48, 27.12, 27.22, 28.63, 34.22, 34.45, 37.80, 52.88, 53.75, 53.97, 58.34, 81.29, 110.79, 113.81, 120.12, 123.03, 135.45, 143.63, 156.40.

**MS (ESI)** m/z = 528.5 [M+H]$^+$.

<u>*tert*-butyl 4-(2-(4-(cyclohexylamino)-3-(cyclopentylamino)benzamido)ethyl)piperazine-1-carboxylate (**29**)</u>

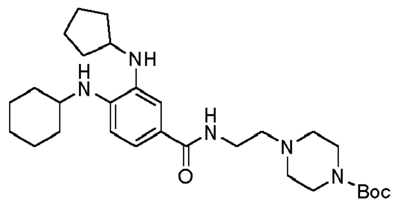

General procedure I was followed using compound **16** (0.150 g, 0.337 mmol) to provide compound **29** (0.053 g, 0.103 mmol, 31 % yield).

**$^1$H NMR (400 MHz, MeOD)** δ 1.21 (qd, *J* = 3.3, 12.6 Hz, 3H), 1.33 - 1.41 (m, 1H), 1.43 (s, 9H), 1.47 - 1.69 (m, 2H), 1.74 (ddt, *J* = 5.0, 9.9, 13.0 Hz, 4H), 2.00 - 2.08 (m, 4H), 2.43 (t, *J* = 5.0 Hz, 4H), 2.54 (t, *J* = 6.7 Hz, 2H), 3.22 - 3.31 (m, 1H), 3.40 (t, *J* = 5.1 Hz, 4H), 3.47 (t, *J* = 6.7 Hz, 2H), 3.79 (t, *J* = 6.3 Hz, 1H), 6.53 (d, *J* = 8.3 Hz, 1H), 7.14 (d, *J* = 2.0 Hz, 1H), 7.19 (dd, *J* = 2.0, 8.3 Hz, 1H).

**$^{13}$C NMR (101 MHz, MeOD)** δ 25.25, 26.31, 27.09, 28.67, 34.11, 34.16, 37.78, 52.81, 53.91, 56.10, 58.30, 81.15, 110.15, 112.78, 119.70, 122.89, 136.30, 141.24, 156.26, 170.93.

**MS (ESI)** m/z = 514.15 [M+H]$^+$.

<u>4-(cyclohexylamino)-3-(cyclopentylamino)-*N*-(2-(piperidin-1-yl)ethyl)benzamide (**30**, **CPD-014**)</u>

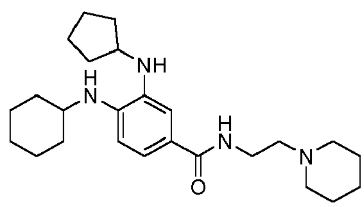

General procedure I was followed using compound **16** (0.200 g, 0.661 mmol) to provide compound **30** (0.034 g, 0.082 mmol, 12 % yield).

**$^1$H NMR (400 MHz, MeOD)** δ 1.19 - 1.33 (m, 3H), 1.36 - 1.59 (m, 6H), 1.59 - 1.85 (m, 11H), 2.00 - 2.13 (m, 4H), 2.48 - 2.55 (m, 4H), 2.57 (t, *J* = 6.9 Hz, 2H), 3.51 (t, *J* = 6.9 Hz, 2H), 3.84 (p, *J* = 6.3 Hz, 1H), 6.57 (d, *J* = 8.7 Hz, 1H), 7.16 (d, *J* = 2.1 Hz, 1H), 7.20 (dd, *J* = 2.1, 8.3 Hz, 1H).

**$^{13}$C NMR (101 MHz, MeOD)** δ 24.93, 25.04, 26.10, 26.38, 26.91, 33.94, 34.02, 37.52, 52.71, 55.35, 56.07, 58.95,

110.21, 112.97, 119.70, 122.86, 136.21, 141.32, 170.92.

**HRMS** calcd 413.3275; found 413.3289 [M+H]$^+$.

3-(benzylamino)-4-(cyclohexylamino)-*N*-(6-(piperazin-1-yl)hexyl)benzamide tetrahydrochloride (**31, CPD-003**)

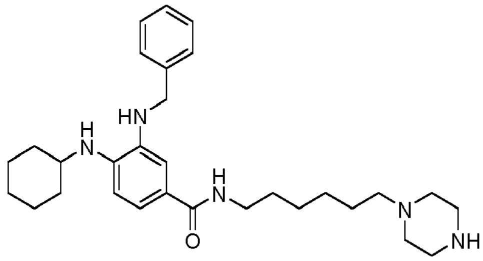

General procedure E was followed using compound **19** (0.074 g, 0.125 mmol) to provide compound **31** (0.048 g, 0.075 mmol, 60 % yield). The reported NMR spectrum refers to tetrahydrochoride salt.

**$^1$H NMR (400 MHz, MeOD)** δ 1.16 - 1.54 (m, 11H), 1.57 - 1.72 (m, 4H), 1.83 (dq, *J* = 3.1, 11.0 Hz, 4H), 1.94 - 2.04 (m, 2H), 3.50 (tq, *J* = 3.3, 10.5 Hz, 2H), 3.57 - 3.79 (m, 8H), 4.52 (s, 2H), 7.18 (d, *J* = 8.9 Hz, 1H), 7.28 - 7.44 (m, 7H).

**$^{13}$C NMR (101 MHz, MeOD)** δ 24.56, 25.62, 26.12, 26.85, 27.13, 29.90, 31.46, 40.67, 41.88, 49.48, 50.29, 58.12, 58.33, 118.15, 121.57, 122.06, 129.26, 129.63, 129.81, 130.46, 130.89, 131.01, 136.86, 169.09.

**HRMS** calcd 492.3697; found 492.3702 [M+H]$^+$.

3-(benzylamino)-4-(cyclohexylamino)-*N*-(4-(piperazin-1-yl)butyl)benzamide tetrahydrochloride (**32, CPD-004**)

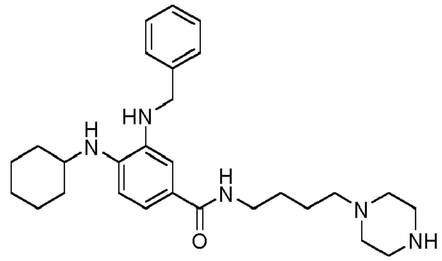

General procedure E was followed using compound **20** (0.074 g, 0.131 mmol) to provide compound **32** (0.048 g, 0.079 mmol, 60 % yield). The reported NMR spectrum refers to tetrahydrochoride salt.

**$^1$H NMR (400 MHz, MeOD)** δ 1.20 - 1.45 (m, 6H), 1.70 (t, *J* = 7.3 Hz, 3H), 1.80 - 1.93 (m, 4H), 2.00 (d, *J* = 9.8 Hz, 2H), 3.32 - 3.37 (m, 2H) 3.41 (t, *J* = 6.8 Hz, 2H), 3.45 - 3.54 (m, 2H), 3.57 - 3.72 (m, 6H), 4.52 (s, 2H), 7.11 - 7.18 (m, 1H), 7.28 - 7.46 (m,7H).

**HRMS** calcd 464.3384; found 464.3391 [M+H]$^+$.

3-(benzylamino)-4-(cyclohexylamino)-*N*-(4-(piperazin-1-ylmethyl)benzyl)benzamide tetrahydrochloride (**33, CPD-005**)

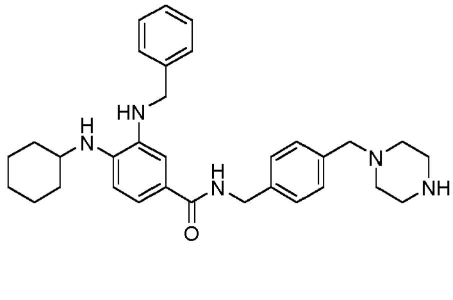

General procedure E was followed using compound **21** (0.021g, 0.131 mmol) to provide compound **33** (0.013 g, 0.020 mmol, 57 % yield). The reported NMR spectrum refers to tetrahydrochoride salt.

**$^1$H NMR (400 MHz, MeOD)** δ 1.20 - 1.47 (m, 6H), 1.68 (d, *J* = 12.3 Hz, 1H), 1.84 (d, *J* = 12.7 Hz, 2H), 1.99 (d, *J* = 12.7 Hz, 2H), 3.49 (td, *J* = 4.2, 9.5 Hz, 1H), 3.60 (s, 8H), 4.51 (d, *J* = 12.1 Hz, 4H), 4.56 (s, 2H), 7.18 (d, *J* = 8.4 Hz, 1H), 7.35 (dh, *J* = 2.7, 5.3 Hz, 5H), 7.40 - 7.47 (m, 4H), 7.54 - 7.60 (m, 2H).

**$^{13}$C NMR (101 MHz, MeOD)** δ 19.45, 25.59, 26.09, 31.52, 41.82, 44.04, 49.84, 50.48, 57.96, 61.45, 118.63, 121.85, 122.06, 127.79, 129.34, 129.40, 129.44, 129.71, 129.86, 130.49, 132.74, 136.47, 142.68, 142.88, 169.12.

**HRMS** calcd 512.3384; found 512.3393 $[M+H]^+$.

3-(benzylamino)-4-(cyclohexylamino)-*N*-(8-(piperazin-1-yl)octyl)benzamide tetrahydrochloride (**34**, **CPD-006)**

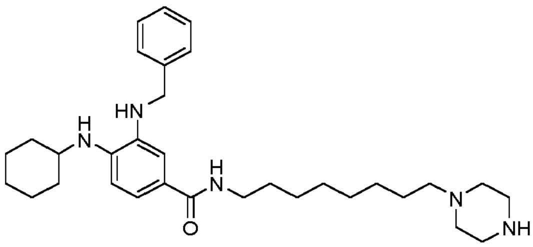

General procedure E was followed using compound **22** (0.018 g, 0.030 mmol) to provide compound **34** (0.008 g, 0.012 mmol, 42 % yield). The reported NMR spectrum refers to tetrahydrochoride salt.

**$^1$H NMR (400 MHz, MeOD)** δ 1.18 - 1.49 (m, 13H), 1.53 - 1.64 (m, 2H), 1.68 (d, *J* = 11.9 Hz, 1H), 1.81 (tt, *J* = 5.2, 11.6 Hz, 4H), 1.95 - 2.03 (m, 2H), 3.22 - 3.37 (m, 3H), 3.51 (ddt, *J* = 3.5, 7.5, 10.6 Hz, 2H), 3.64 (s, 8H), 4.52 (s, 2H), 7.19 (d, *J* = 8.9 Hz, 1H), 7.27 - 7.42 (m, 7H). **$^{13}$C NMR (101 MHz, MeOD)** δ 24.63, 25.59, 26.08, 27.19, 27.63, 29.78, 29.83, 30.13, 31.39, 40.90, 41.88, 49.47, 50.27, 58.31, 58.45, 118.15, 121.50, 122.38, 129.14, 129.27, 129.61, 129.82, 130.47, 132.84, 136.82, 169.11.

**HRMS** calcd 520.4010; found 520.4023 $[M+H]^+$.

*N*-(2-aminoethyl)-4-(cyclohexylamino)-3-(cyclopentylamino)benzamide trihydrochloride (**35**, **CPD-007**)

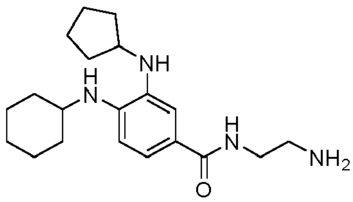

General procedure E was followed using compound **23** (0.093 g, 0.209 mmol) to provide compound **35** (0.077 g, 0.157 mmol, 75 % yield). The reported NMR spectrum refers to trihydrochoride salt.

**$^1$H NMR (400 MHz, $D_2O$)** δ 1.03- 1.33 (m, 5H), 1.46 - 1.75 (m, 9H), 1.81 - 1.96 (m, 4H), 3.13 (t, *J* = 5.9 Hz, 2H), 3.37 (s, 1H), 3.57 (t, *J* = 6.0 Hz, 2H), 3.93 (s, 1H), 6.96 (d, *J* = 8.7 Hz, 1H), 7.40 - 7.66 (m, 2H).

**$^{13}$C NMR (101 MHz, $D_2O$)** δ 23.48, 24.34, 25.07, 29.83, 31.49, 37.31, 39.38, 53.18, 60.39, 115.37, 122.12, 122.82, 124.24, 126.61, 139.90, 169.91.

**HRMS** calcd 345.2649; found 345.2650 $[M+H]^+$.

4-(cyclohexylamino)-3-(cyclopentylamino)-*N*-(2-(methylamino)ethyl)benzamide trihydrochloride (**36, CPD-008**)

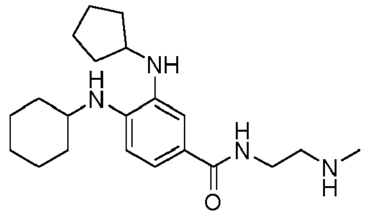

General procedure E was followed using compound **24** (0.015 g, 0.033 mmol) to provide compound **36** (0.009 g, 0.020 mmol, 61 % yield). The reported NMR spectrum refers to trihydrochoride salt.

**$^1$H NMR (400 MHz, MeOD)** δ 1.21 - 1.50 (m, 5H), 1.69 (dt, *J* = 3.4, 7.8 Hz, 3H), 1.76 - 1.94 (m, 6H), 1.96 - 2.10 (m, 4H), 2.75 (s, 3H), 3.25 (t, *J* = 5.7 Hz, 2H), 3.46 (d, *J* = 6.2 Hz, 1H), 3.70 (h, *J* = 5.4 Hz, 2H), 4.01 - 4.11 (m, 1H), 7.02 (d, *J* = 9.4 Hz, 1H), 7.74 - 7.80 (m, 2H). **$^{13}$C NMR (101 MHz, MeOD)** δ 24.85, 25.87, 26.59, 31.10, 33.02, 33.91, 37.50, 50.54, 54.45, 62.03, 116.12, 124.00, 124.62, 128.47, 169.67.

**HRMS** calcd 359.2805; found 359.2811 $[M+H]^+$.

*N*-(2-aminoethyl)-3,4-bis(cyclohexylamino)benzamide trihydrochloride (**37**, **CPD-009)**

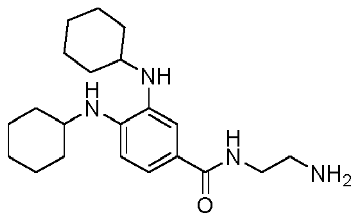

General procedure E was followed using compound **25** (0.155 g, 0.338 mmol) to obtain compound **37** (0.106 g, 0.210 mmol, 62 % yield). The reported NMR spectrum refers to trihydrochoride salt.

**$^1$H NMR (400 MHz, MeOD)** δ 1.43 (ddt, *J* = 12.7, 20.4, 44.2 Hz, 8H), 1.63 (q, *J* = 13.2 Hz, 2H), 1.78 (d, *J* = 11.9 Hz, 2H), 1.93 (dd, *J* = 11.9, 24.5 Hz, 4H), 2.13 (t, *J* = 9.9 Hz, 4H), 3.41 (s, 1H), 3.57 (d, *J* = 11.6 Hz, 2H), 3.78 (d, *J* = 6.2 Hz, 2H), 7.09 (d, *J* = 8.8 Hz, 1H), 7.79 - 7.99 (m, 2H).

**$^{13}$C NMR (101 MHz, MeOD)** δ 24.30, 24.48, 24.63, 25.30, 29.33, 31.93, 37.34, 39.52, 52.22, 60.26, 113.72, 120.27, 121.80, 124.00, 128.34, 142.31, 168.60.

**HRMS** calcd 359.2805; found 359.2811 $[M+H]^+$.

3,4-bis(cyclohexylamino)-*N*-(2-(methylamino)ethyl)benzamide trihydrochloride (**38, CPD-010**)

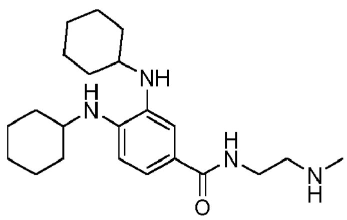

General procedure E was followed using compound **26** (0.150 g, 0.317 mmol) to obtain compound **38** (0.123 g, 0.255 mmol, 80 % yield). The reported NMR spectrum refers to trihydrochoride salt.

**$^1$H NMR (400 MHz, MeOD)** δ 1.20 - 1.49 (m, 8H), 1.55 (qd, *J* = 9.6, 12.7 Hz, 2H), 1.65 - 1.74 (m, 2H), 1.79 - 1.92 (m, 4H), 2.02 - 2.10 (m, 4H), 2.75 (s, 3H), 3.25 (t, *J* = 5.6 Hz, 2H), 3.42 - 3.60 (m, 2H), 3.70 (t, *J* = 5.6 Hz, 2H), 6.99 (d, *J* = 8.6 Hz, 1H), 7.78 - 7.86 (m, 2H).

**$^{13}$C NMR (101 MHz, MeOD)** δ 25.65, 25.95, 26.05, 26.71, 30.71, 33.33, 33.90, 37.55, 50.76, 53.79, 61.55, 115.15, 123.52, 125.60, 129.78, 169.49.

**HRMS** calcd 373.3962; found 373.2964 $[M+H]^+$.

3,4-bis(cyclohexylamino)-N-(2-(piperazin-1-yl)ethyl)benzamide tetrahydrochloride (**39**, **CPD-012**)

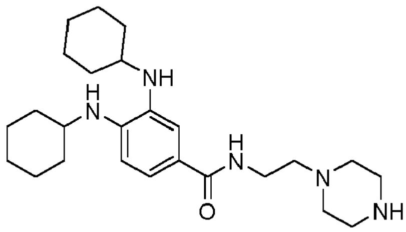

General procedure E was followed using compound **28** (0.025 g, 0.047 mmol) to obtain compound **39** (0.011 g, 0.026 mmol, 54 % yield). The reported NMR spectrum refers to tetrahydrochoride salt.

**$^1$H NMR (400 MHz, MeOD)** δ 1.21 - 1.64 (m, 10H), 1.73 (d, *J* = 12.5 Hz, 2H), 1.86 (d, *J* = 16.1 Hz, 4H), 1.96 - 2.15 (m, 4H), 3.51 (dt, *J* = 7.3, 15.4 Hz, 4H), 3.59 (dt, *J* = 3.6, 7.4 Hz, 2H), 3.70 (s, 4H), 3.84 (t, *J* = 5.5 Hz, 4H), 7.02 (d, *J* = 8.7 Hz, 1H), 7.81 - 7.94 (m, 2H).

**HRMS** calcd 428.3384; found 428.3387 $[M+H]^+$.

4-(cyclohexylamino)-3-(cyclopentylamino)-N-(2-(piperazin-1-yl)ethyl)benzamide tetrahydrochloride (**40, CPD-013**)

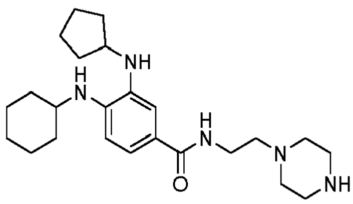

General procedure E was followed using compound **29** (0.044 g, 0.086 mmol) to obtain compound **40** (0.033 g, 0.800 mmol, 93 % yield). The reported NMR spectrum refers to tetrahydrochoride salt.

**$^1$H NMR (400 MHz, DMSO-$d_6$+$D_2O$)** δ 1.09 - 1.33 (m, 6H), 1.49 - 1.61 (m, 5H), 1.67 (d, *J* = 12.4 Hz, 4H), 1.88 (d, *J* = 12.4 Hz, 4H), 3.24 - 3.30 (m, 2H), 3.36 - 3.42 (m, 4H), 3.44 - 3.51 (m, 4H), 3.56 - 3.63 (m, 2H), 3.83 (s, 1H), 6.89 (d, *J* = 8.7 Hz, 1H), 7.52 (s, 1H), 7.55 (d, *J* = 9.1 Hz, 1H).

**HRMS** calcd 414.3227; found 414.3233 $[M+H]^+$.

## 2. Biological evaluation of the compounds of the invention

Inhibition of ML162-Induced Ferroptosis in HT1080 human fibrosarcoma cells

Human fibrosarcoma cells HT1080 cells were obtained from American Type Culture Collection (ATCC). HT1080 cells were cultured in EMEM medium supplemented with 10% FCS and L-glutamine (1 mM), sodium pyruvate and nonessential amino acids. Cell death was measured using Envision multimode plate reader (PE). In order to determine $IC_{50}$ values, HT1080 were seeded in a 384-well plate at a density of 3500 cells/well in 40μl in an incubator overnight at 37 °C with 5% CO2. The next day, the cells were pretreated for 1h (in triplicates) with a 1/3 dilution series of ferrostatin-1 analogues ranging from 1 μM to 0.5 nM and Sytox Green (1.6 μM). All the compound's stock solutions were prepared in DMSO at 100 mM concentration. After stimulating the cells with ML162 1 μM the plate was transferred to the incubator. SytoxGreen intensity was measured after 8, 12, 16 and 24 h using an excitation filter of 485 nm and an emission filter of 535 nm. Dose-response curves were made as % cell death inhibition, taking ML162 treated samples as 0 % inhibition and Fer-1 (1 μM) + ML-162 samples as the 100 % inhibition. Cell death percentage was calculates as 100 - (( x - 100%inh) / ( 0%inh - 100%inh))*100). Curves were plotted in Spotfire software, and $IC_{50}$ values were calculated using logistic regression curves. Data is presented in Table 2.

Fluorescence-Enabled Inhibited Autoxidation (FENIX)

Ref. Shah *et al.* (2019) Cell Chem. Biol. 26, 1594-1607

Unless otherwise stated, laboratory reagent grade solvents were used. Reagents were obtained from various commercial sources and were used without any prior purification. L-α-phosphatidylcholine (Egg, Chicken), powder was purchased from Merck Life Science B.V.. DTUN and STY-BODIPY were synthesized according to methods described in Shah et al. (2019) Cell Chem. Biol. 26, 1594-1607. Fluorescence was measured using the UV/vis spectrophotometer Synergy MX, Biotek with Gen5.

To quantify radical trapping antioxidant activity in phospholipid bilayers to a clear bottom black-walled 96-well plate for fluorescence-base assays (Invitrogen by Thermo Fisher Scientific) was added 250 μL of a solution containing liposomes (1 mM), styrene-conjugated BODIPY (STY-BODIPY) (1 μM), and the respective radical trapping antioxidant (RTA) (4 μM). The solutions were made in larger volumes in Eppendorfs, pre-mixed and 250 μL aliquot was transferred to each well. The plate was incubated for 10 minutes at 37°C in the BioTek SynergyMx plate reader, followed by a fast-mixing protocol for 5 minutes. The plate was ejected from the plate reader and autoxidation was initiated by the addition of a 50 μL aliquot of (E)-1,2-bis((2-methyldecan-2-yl)oxy)diazene (DTUN) (0.2 mM in EtOH/PBS (3/47, v/v), followed by another mixing protocol for 5 minutes. Data were acquired by excitation probes at 488 nm and emission was measured at 518 nm (read intervals 1.0 min). Kinetic read parameters: (i) optic position: bottom, (ii) gain: 80, (iii) bandwidth: 9.0. The results of the FENIX assay are presented in Table 2 as inhibition rate constants ($k_{inh}$), $\log k_{inh}$ and stoichiometries (n) of tested compounds.

<u>Kinetic solubility</u>

A turbidimetric method was used. First, a series of DMSO compound stock solutions were prepared (0.15-5 mM) from the stock solution of the compound in DMSO (10 mM). An aliquot of 4μL stock solution was added to 196μL PBS buffer (pH 7.4). A series of concentrations were prepared (3.13-200 μM), including a blank on a microtiter plate. The microtiter plate was shaken for 10 seconds and incubated for 2 hours at 37°C. Turbidity was measured using the UV/vis spectrophotometer Synergy MX, Biotek with Gen5. When there was no turbidity measured at a given concentration the sample was assumed to be dissolved. Data is presented in Table 2.

Table 2.

| Compound Code | FENIX assay | | | Kin. solubility (μM) | In vitro |
|---|---|---|---|---|---|
| | $k_{inh}$ ($10^4$ $M^{-1}s^{-1}$) ± SD | $\log k_{inh}$ | Stoichiometry | | $IC_{50}$ (nM) |
| CE 1 | 0.10 | 3.00 | 0 | >200 | 139 ± 125 (n=2) |
| CPD-001 | 1.5 | 4.18 | 3.0 | 25-50 | 6.58 |
| CPD-002 | 1.5 | 4.18 | 2.8 | 25-50 | 5.97 |
| CPD-003 | n.d. | n.d. | n.d. | >200 | 23.0 ± 13.2 (n=2) |
| CPD-004 | n.d. | n.d. | n.d. | >200 | 17.0 ± 3.73 (n=2) |
| CPD-005 | n.d. | n.d. | n.d. | n.d. | 17.9 ± 4.85 (n=2) |
| CPD-006 | n.d. | n.d. | n.d. | n.d. | 16.4 ± 4.35 (n=2) |
| CPD-007 | 1.5 | 4.17 | 4.4 | >200 | 4.52 ± 3.29 (n=2) |
| CPD-008 | 1.7 | 4.23 | 3.6 | >200 | 3.77 ± 2.07 (n=2) |
| CPD-009 | 1.4 | 4.14 | 4.0 | >200 | 3.74 ± 2.03 (n=2) |
| CPD-010 | 1.6 | 4.19 | 3.8 | >200 | 8.10 ± 7.95 (n=2) |
| CPD-011 | 3.0 | 4.48 | 3.2 | >200 | 2.92 ± 0.731 (n=2) |
| CPD-012 | 1.6 | 4.21 | 3.2 | >200 | 2.60 |
| CPD-013 | 1.5 | 4.18 | 3.9 | >200 | 4.43 |
| CPD-014 | 3.3 | 4.52 | 3.2 | 100-200 | 2.26 |
| CPD-015 | 1.8 | 4.24 | 2.7 | >200 | 6.91 ± 4.99 (n=3) |

CE corresponds to compound

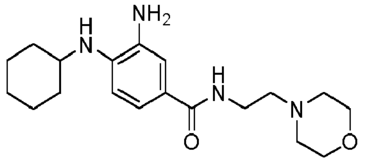

disclosed in WO2020113028A1 as compound 3.

Human and mouse microsomal stability

**Experimental procedure**

Pooled liver microsomes (Ultrapool Human Liver microsomes or Mouse CD-1 male Liver microsomes), with a protein concentration of 20 mg/ml, were purchased from Corning Gentest™ (now Discovery Life Science - Gentest) and stored at -80°C until use. The microsomes (final protein concentration 0.5 mg/ml), 0.1 M phosphate buffer pH 7.4 and test compound (final substance concentration 1 μM), were preincubated at 37°C for 10 min before the addition of NADPH Regenerating System (solution A and B from Corning Gentest™, final concentration 1 mM) to initiate the reaction. For each compound tested, a control without the cofactor was included. In this case, 0.1 M phosphate buffer at pH 7.4 was added instead of the NADPH regenerating system. In addition, with each new batch of microsomes, two positive control compounds were incorporated. These controls represented substances with high (verapamil, with human $Cl_{int}$ = 178.9 μL/min*mg; diazepam, with mouse $Cl_{int}$ = 549 μL/min*mg) and low clearance (dextromethorphan, with human $Cl_{int}$ = 34.6 μL/min*mg; diphenhydramine, with mouse Clint = 64.0 μL/min*mg).

Each compound was incubated for 45 minutes at 37°C and shaken at 500 rpm. The reactions were stopped by transferring the incubate into acetonitrile, containing an internal standard, in Eppendorfs at the appropriate time points in a 1:3 ratio (0, 5, 10, 15, 30, and 45 minutes for compounds, and 0, 15, and 45 minutes for the negative control of those compounds). Subsequently, the Eppendorfs were centrifuged at 3000 rpm for 20 minutes at 4°C to precipitate the protein.

**Quantitative Analysis**

After protein precipitation, the sample supernatant from each time point was analysed using UPLC-MS/MS. The analysis followed the general UPLC method for microsomal stability described below. The UPLC (ultra-performance liquid chromatography), used to quantify the microsomal stability of the products, was an ACQUITY UPLC H-Class system with a TUV detector Waters (not used in this assay) coupled to an MS/MS detector Xevo Waters TQD. Waters Acquity UPLC BEH C18 1.7 μm, 2.1 mm × 50 mm column was used. The eluent was composed of two different solvents. Solvent A consisted of water with 0.1% formic acid, solvent B was acetonitrile with 0.1% formic acid. The column was first equilibrated for with a mixture of 95% solvent A and 5% solvent B until the delta of psi decrease below 40 psi. The method began with a short equilibration to reach 50% of solvent A and B in 0.15 min. Following this, solvent B was increased linearly to 95% over 2.75 min before being held constant for 0.70 min (flow rate 0.7 ml/min). The specific masses of the compounds were tracked using tuning files generated via Intellistart®, and quantification was aided by an internal standard.

**Data processing**

All obtained readouts were processed using Microsoft Excel. From a plot of ln peak area ratio (compound peak area/ internal standard peak area) against time, the gradient of a line is determined by linear regression. Subsequently several parameters were calculated using the equations below:

- $$\text{Elimination rate constant } (k) = (-\text{ gradient})$$
- $$\text{Half Time } (t_{1/2}) = \ln(2)/k$$
- 

$$Cl_{int}\ (\mu L/min/mg\ of\ protein) = V \times \ln(2) \times t_{1/2}$$

- Where V = (Incubation volume µL)/(Microsomal protein mg/g liver)

Data is presented in Table 3.

Table 3.

| Compound code | Microsomal stability in mouse | | Microsomal stability in human | |
|---|---|---|---|---|
| | $t_{1/2}$ (min)[a] | $Cl_{int}$ in liver microsomes (µL/min*mg)[b] | $t_{1/2}$ (min)[a] | $Cl_{int}$ in liver microsomes (wL/min*mg)[b] |
| Diazepam | 2.0 | 707.0 | n.d. | n.d. |
| Diphenhydramine | 30.7 | 45.1 | n.d. | n.d. |
| Verapamil | n.d. | n.d. | 7.7 | 178.9 |
| Dextromethorphan | n.d. | n.d. | 40.0 | 34.6 |
| CE | n.d. | n.d. | n.d. | n.d. |
| CPD-001 | n.d. | n.d. | 110 | 12.6 |
| CPD-002 | n.d. | n.d. | 34.7 | 39.9 |
| CPD-003 | n.d. | n.d. | n.d. | n.d. |
| CPD-004 | n.d. | n.d. | n.d. | n.d. |
| CPD-005 | n.d. | n.d. | n.d. | n.d. |
| CPD-006 | n.d. | n.d. | n.d. | n.d. |
| CPD-007 | 139.1 | 10.0 | >250 | <5.55 |
| CPD-008 | 96.3 | 14.4 | >250 | <5.55 |
| CPD-009 | 213.0 | 6.5 | >250 | <5.55 |
| CPD-010 | 85.0 | 16.3 | >250 | <5.55 |
| CPD-011 | n.d. | n.d. | 25.2 | 55.1 |
| CPD-012 | n.d. | n.d. | 70.8 | 19.6 |
| CPD-013 | n.d. | n.d. | 99.5 | 13.9 |
| CPD-014 | n.d. | n.d. | 27.8 | 49.9 |
| CPD-015 | 188 | 7.36 | >250 | <5.55 |

[a] Metabolism determined in Corning ultra pool human liver microsome (HLM) or Mouse CD-1 liver microsome (MLM) activated with NAPDH and expressed as half-life ($t_{1/2}$ in min); [b]The $Cl_{int}$ (liver microsomes) was calculated using the measured microsomal $t_{1/2}$ and considers several experimental variables such as the protein concentration and the volume of incubation. Diazepam, diphenhydramine, verapamil and dextromethorphan were used as controls.

**Other experiments related to CPD-015.**

High Throughput Aqueous Solubility - MS

A 100 µM solution of CPD-015 is made by diluting 5 µL of the 10 mM solution in 495 µL of DMSO. A 5 point standard curve of CPD-015 is made in 75:25 DMSO: water to give a range of concentrations; 5 µM, 500 nM, 50 nM, 5 nM and 0.5 nM. 1 mL of aqueous buffer (selected from list above, other buffers are available on request) is added to solid CPD-015 in the vial supplied and placed on a rotary incubator table at room temperature for 24 hr. Following incubation, 200 µL of each of the incubated solutions is removed and filtered using a 96-well 0.4 µM MultiScreen HTS PCF Polycarbonate centrifugal filter plate. The filtrate is serially diluted 1 in 4, 1 in 40 and 1 in 400 fold into 75:25 DMSO:water. The standards and samples are quantified using a Waters Acquity UPLC-MS/MS system. The aqueous solubility of

CPD-015 is quantified from a linear fit of the standard curve. Data is presented in Table 4.

LogD Shake Flask

CPD-015 (0.5 mg) is dissolved in octanol pre-saturated with 0.1 M phosphate buffer (pH 7.4) and sonicated for 30 min. 0.1 M phosphate buffer (pH 7.4) pre-saturated with octanol is added to the octanol solution and shaken at 1000 rpm for 30 min at 22 °C. The mixed samples are then centrifuged at 1000 rpm for 5 min at 22 °C. An aliquot of the octanol fraction is removed from each mixture and diluted into DMSO. The remainder of the octanol fraction is removed and an aliquot of the aqueous fraction is transferred into a fresh tube. The aqueous aliquot is centrifuged again at 1000 rpm for 5 min at 22 °C and an aliquot of the resulting aqueous fraction is diluted into DMSO. The resulting octanol and aqueous fraction solutions are analysed by LC-MS/MS using Cyprotex generic conditions. The relative amount of CPD-015 in the aqueous fraction solution is quantified against a 5 point standard curve, which is produced by serially diluting the octanol fraction solution. LogD is calculated using the following equation:

$$\mathrm{LogD} = \mathrm{Log}\left(\frac{C_{oct}}{C_{aq}}\right)$$

Where: $C_{oct}$ = Concentration in octanol standard (corrected for dilution); $C_{aq}$ = Concentration in aqueous sample (corrected for dilution). Data is presented in Table 4.

Biological stability in plasma

Species-specific plasma is adjusted to pH 7.4 at 37 °C and CPD-015 (final substrate concentration 1 μM; final DMSO concentration 0.25 %) is added to initiate the reaction. The final incubation volume is 500 μL. A solvent control incubation is included, along with a positive control known to be metabolised specifically by each species. The solvent control incubation is incubated for 120 min only. CPD-015 is incubated for 0, 5, 15, 30, 60 and 120 min at 37 °C.

The reactions are stopped by transferring 50 μL of incubate to 150 μL acetonitrile containing internal standard at the appropriate time points. The termination plates are centrifuged at 3,000 rpm for 30 min at 4 °C to precipitate the protein. This experimental procedure can also be applied to other biological matrices, such as blood, homogenate, simulated biological fluids and buffers.

Quantitative Analysis

Following protein precipitation, the sample supernatants are combined in cassettes of up to 4 test articles, diluted in a 1:6 ratio of supernatant:water and analysed using Cyprotex generic LC-MS/MS conditions. If the metabolite profiling option is chosen prior to the commencement of the study, samples are set aside for profiling. The level of metabolite profiling chosen depends on amount of knowledge and support required by the client and can include rapid high-resolution analysis to identify whether metabolites are formed, or more in-depth analysis and/or detailed fragmentation analysis to understand metabolite derivation.

From a plot of In peak area ratio (peak area/internal standard peak area) against time, the gradient of the line is determined. Subsequently, half-life is calculated using the equations below:

$$\text{Elimination rate constant (k)} = (\text{- gradient})$$

$$\text{Half-life } (t_{1/2})(\text{min}) = \frac{0.693}{k}$$

Data is presented in Table 4.

Liver Microsomal Stability (by Contract Research Organization)

Pooled liver microsomes are purchased from a reputable commercial supplier. A range of species and strains are available upon request. Microsomes are stored at 80 °C prior to use. Microsomes (final protein concentration 0.5 mg/mL), 0.1 M phosphate buffer pH 7.4 and CPD-015 (final substrate concentration 1 μM; final DMSO concentration 0.25 %) are preincubated at 37 °C prior to the addition of NADPH (final concentration 1 mM) to initiate the reaction. A minus cofactor control incubation is included for each CPD-015 where 0.1 M phosphate buffer pH 7.4 is added instead of NADPH (minus

NADPH). Two species-specific positive controls are also incubated alongside CPD-015 for each species. All incubations are performed singularly for CPD-015. CPD-015is incubated for 0, 5, 15, 30 and 45 min. The control (minus NADPH) is incubated for 45 min only. The reactions are stopped by transferring incubate into acetonitrile at the appropriate time points, in a 1:3 ratio. The termination plates are centrifuged at 3,000 rpm for 10 min at 4 °C to precipitate the protein.

Quantitative Analysis

Following protein precipitation, the sample supernatants are combined in cassettes of up to 4 test articles, internal standard is added and samples analysed using Cyprotex generic LCMS/MS conditions

Data analysis

From a plot of In peak area ratio (peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half-life } (t_{½})(\text{min}) = \frac{0.693}{k}$$

$$\text{Intrinsic clearance } (CL_{int})(\mu L/\text{min/mg protein}) = \frac{V \times 0.693}{t_{½}}$$

where V = Incubation volume (μL)/Microsomal protein (mg). Data is presented in Table 4.

Hepatocyte Stability

Cryopreserved pooled hepatocytes are purchased from a reputable commercial supplier. A range of species and strains are available upon request. Cryopreserved hepatocytes are stored in liquid nitrogen prior to use. A suspension of cryopreserved hepatocytes (final cell density 0.5 × 106 viable cells/mL in Williams E media supplemented with 2 mM L glutamine and 25 mM HEPES) is pre-incubated at 37 °C prior to the addition of CPD-015 (final substrate concentration 1 μM; final DMSO concentration 0.25%) to initiate the reaction. The final incubation volume is 500 μL. Two positive controls are included with each species. Each CPD-015 is incubated for 0, 5, 10, 20, 40 and 60 min at 37 °C. The reactions are stopped by transferring incubate into acetonitrile at the appropriate time points, in a 1:3 ratio. The termination plates are centrifuged at 3,000 rpm for 30 min at 4 °C to precipitate the protein. Following protein precipitation, the sample supernatants are combined in cassettes of up to 4 test articles, internal standard is added and samples analysed using Cyprotex generic LC-MS/MS conditions. If the metabolite profiling option is chosen prior to the commencement of the study, samples are set aside for profiling. The time points are changed to exclude the 5 min time point and to include a 120 min time point. The time point at which 30-70% of parent has degraded and the 120 min time point can then be investigated at 3 different levels of metabolite profiling and/or identification. The level of metabolite profiling chosen depends on amount of knowledge and support required by the client and can include rapid, low-resolution analysis to identify whether metabolites are formed, cross species comparisons with high resolution analysis or detailed fragmentation analysis to understand metabolite derivation.

From a plot of In peak area ratio (compound peak area/internal standard peak area) against time, the gradient of the line is determined. Subsequently, half-life (t½) and intrinsic clearance (CLint) are calculated using the equations below:

$$\text{Elimination rate constant (k)} = (\text{- gradient})$$

$$\text{Half-life } (t_{½})(\text{min}) = \frac{0.693}{k}$$

$$\text{Intrinsic clearance } (CL_{int})\ (\mu L/\text{min/million cells}) = \frac{V \times 0.693}{t_{½}}$$

where V = Incubation volume (μL)/Number of cells CLint values falling below the lower limit of assay sensitivity (calculated

based on t½>3 × incubation time) are categorised as below the lower limit of quantification (<LOQ). Data is presented in Table 4.

Reactive Metabolite Formation (Stable Labelled Glutathione Trapping,)

Objective: To assess the potential of a glutathione reactive metabolite formation in human liver microsomes.

Procedure

Pooled human liver microsomes (final protein concentration 1 mg/mL), 0.1 M phosphate buffer pH 7.4 with MgCl2 (final concentration 3 mM) and CPD-015 (final substrate concentration 50 μM) is incubated at 37°C prior to the addition of NADPH (final concentration 2 mM) and GSH (final concentration 1 mM, 1:1 unlabelled GSH to 13C2, 15NGSH). A control incubation is included for each CPD-015 assessed where 0.1 M phosphate buffer pH 7.4 with MgCl2 (final concentration 3 mM) is added instead of NADPH (minus NADPH). CPD-015 is incubated for 60 min. The reactions are stopped by the addition of acetonitrile. The incubation plates are centrifuged, and the supernatants are diluted 1:2 with water and then analysed by HR-LCMS. Ticlopidine is screened alongside the CPD-015 as a positive control.

Data Analysis

Following the assay, samples are analysed by MSe over a range of m/z 50 to 1200, with MSe collision energy ramp of 10-40 V in the high energy function. Potential reactive metabolites are identified by processing samples through UNIFI. Metabolites are identified by searching against a list of expected biotransformations with the addition of GSH or an addition of CysGly (when pyroglutamic acid is lost from glutathione prior to conjugation). Reactive metabolites are reported where an isotopic doublet of +3 amu is observed. The reactive metabolite must have a ratio of at least three to one when compared to a corresponding peak in the control sample (unless proposed biotransformation indicates a non-enzymatic i.e. chemical reaction). A ppm error of less than 5 is desirable; when the response for a metabolite observed is low the ppm error may be greater than 5. Additionally, the following diagnostic losses are reported where observed:

- Loss of glycine, $C_2H_5NO_2$, mass of 75.0320
- Loss of pyroglutamic acid, $C_5H_7O_3N$, mass of 129.0426
- Loss of reduced CysGly, $C_5H_8N_2O_3S$, mass of 176.0256
- Loss of CysGly, $C_5H_{10}N_2O_3S$, mass of 178.0412
- Loss of reduced glutathione, $C_{10}H_{15}N_3O_6S$, mass of 305.0682
- Loss of glutathione, $C_{10}H_{17}N_3O_6S$, mass of 307.0838 All potential reactive metabolites are confirmed by expert review prior to release of data.

Data is summarized in Table 4.

Phospholipidosis and Steatosis Assay

Objective: To assess the potential for a compound to exhibit cytotoxic effects on a panel of cell health parameters (cell count, nuclear size, DNA structure, phospholipidosis and steatosis).

Experimental Procedure

HepG2 human hepatocellular carcinoma cells (other cell lines are available upon request) are plated on an appropriately sized multi-well plate in suitable media at 37 °C in 5 % CO2 for 24 hr prior to dosing of the cells. Test compound is diluted in DMSO and serial dilutions are made in 0.5 % DMSO in growth media. Test compound at 8 concentrations in triplicate is then incubated for 72 hr (dependent upon customer requirements). Appropriate controls are run alongside the assay. At the end of the incubation period, the cells are loaded with the relevant dyes for the appropriate time for each cell health marker. The plate is then scanned using an automated fluorescent cellular imager, ArrayScan® VTI or XTI (Thermo Scientific Cellomics). The assay provides simultaneous measurement of multiple cell health parameters including cell count, nuclear size, DNA structure, phospholipidosis and steatosis.

Data Analysis

For readouts other than cell viability, a statistical approach to data analysis is used. The solvent controls are used to determine the definitions of "normal" for each parameter, then the software calculates the percentage of cells that are low or high responders (depending on the biological significance of a particular readout). The solvent control wells are then used to determine significance limits for wells that have a greater than expected fraction of low or high responders. The minimum effective concentration is determined from the lowest concentration whose mean value exceeds the significance level, provided either a clear dose-response relationship is observed, or at least two consecutive concentration points are above the significance level. AC50 values are also determined provided a clear dose-response relationship is observed. Data is summarized in Table 4.

Cytotoxicity Screening Panel (MMP, MP, Cyto C)

Objective: To assess the potential of CPD-015 to exhibit toxic effects on a panel of cell health parameters (cell loss, nuclear morphology, cell membrane permeability, mitochondrial membrane potential changes, mitochondrial mass, DNA fragmentation and cytochrome C localisation). These targets have been shown to be predictive for compounds causing various forms of toxicity.

Procedure

HepG2 human hepatocellular carcinoma cells (other cell lines are available upon request) are plated on appropriately sized multi-well plates in suitable media at 37°C in 5 % CO2 for 24 hr prior to dosing of the cells. CPD-015 is diluted in solvent, and serial dilutions are made in 0.5 % solvent, in growth media. CPD-015 at 8 concentrations (0.04, 0.1, 0.4, 1, 4, 10, 40, 100 μM) in triplicate is then incubated for 72 hr. Appropriate controls are run alongside the assay. At the end of the incubation period, the cells are loaded with the relevant dye/antibody for the appropriate time for each cell health marker. The plate is then scanned using an automated fluorescent cellular imager. The assay provides simultaneous measurement of multiple cell health parameters:

- Cell count: A decreasing number of cells per well indicates toxicity due to necrosis, apoptosis or a reduction in cellular proliferation.
- Nuclear size: An increase in nuclear area can indicate necrosis or G2 cell cycle arrest and a decrease can indicate apoptosis.
- DNA structure: An increase in DNA structure can indicate chromosomal instability and DNA fragmentation.
- Cell membrane permeability: An increase in cell membrane permeability is a general indicator of cell death.
- Mitochondrial mass: A decrease in mitochondrial mass indicates loss of total mitochondria and an increase implies mitochondrial swelling or an adaptive response to cellular energy demands.
- Mitochondrial membrane potential ($\Delta\psi$m): A decrease indicates a loss of mitochondrial membrane potential and mitochondrial toxicity, as well as a potential role in apoptosis signalling, an increase in mitochondrial membrane potential indicates an adaptive response to cellular energy demands.
- Cytochrome c: An increase in cytochrome c release is one of the hallmarks of the apoptosis cascade.

Data analysis

For readouts other than cell count, a statistical approach to data analysis is used. The solvent controls are used to determine the definitions of "normal" for each parameter, then the software calculates the percentage of cells that are low or high responders (depending on the biological significance of a particular readout). The solvent control wells are then used to determine significance limits for wells that have a greater than expected fraction of low or high responders. The minimum effective concentration is determined from the lowest concentration whose mean value exceeds the significance level, provided either a clear dose-response relationship is observed, or at least two consecutive concentration points are above the significance level. $AC_{50}$ values are also determined provided a clear dose-response relationship is observed.

Table 4.

| Properties/ assay | Explanation/additi onal information | CPD-015 |
|---|---|---|
| High Throughput Aqueous Solubility | analysis via UPLC-MS/MS, @pH 2 and 7.4 | 1265 μM [pH 2]<br>439 μM [pH 7] |

(continued)

| Properties/ assay | Explanation/additi onal information | CPD-015 | |
|---|---|---|---|
| LogD Shake Flask Assay | pH 7.4 | 2.12 | |
| Plasma Stability | Human | Highly stable (no degradation over 2h) | |
| | Mouse | Highly stable (no degradation over 2h) | |
| Microsomal stability | Mouse $CL_{int}$ Low < 13.1; high > 71.1 | **Low** $CL_{int}$ = 9.70 μL/min/mg protein<br>$t_{1/2}$ = 143 min | |
| | Human $CL_{int}$ Low < 8.6; high > 47.0 | **Low** $CL_{int}$ = 2.90 μL/min/mg protein<br>$t_{1/2}$ = 478 min | |
| Hepatocyte stability | Mouse $CL_{int}$ Low < 3.3; high > 17.8 | **High** $CL_{int}$ = 31.2 μL/min/$10^6$ cells<br>$t_{1/2}$ = 44.5 min | |
| | Human $CL_{int}$ Low < 3.5; high > 19.0 | **High** $CL_{int}$ = 24.0 μL/min/$10^6$ cells<br>$t_{1/2}$ = 57.7 min | |
| Reactive metabolite formation - GSH trapping assay | | Parent compound - **99.97** % of response reported<br>Negligible metabolites formation | |
| Phospholipidosis and Steatosis (HepG2) | | No response observed for phospholipidosis PLD potential: NO<br>No response observed for steatosis | |
| Cytotoxicity screening panel | Cell health parameter | MEC (μM) | AC50 (μM) |
| | Cell count | 4.84 | 5.87 |
| | DNA structure | 3.15 | >4 |
| | Nuclear size; Cell membrane permeability; Mitochondrial mass; Mitochondrial membrane potential; Cytochrome c | NR | NR |
| MEC - minimum effective concentration that significantly crosses vehicle control threshold; $AC_{50}$ - the concentration at which 50% maximum effect is observed for each cell health parameter; NR - no response observed. | | | |

Pharmacokinetic profiling of CPD-015 in the CD-1 male mouse following IV and PO dosing Formulation Preparation

Formulations were prepared fresh on the day of the *in vivo* experiment.

Pre-weighed CPD-015 was dissolved in DMSO/Cremophor/PBS (10/10/80) to reach final concentration of 2 mg/mL for an intravenous dose of 10 mg/kg (IV) and 1 mg/mL for an oral dose of 10 mg/kg (PO).

Animals

This study was performed in in male CD-1 mice 7 weeks old at the time of administration. Mice were kept in solid bottom cages (TECNIPLAST S.p A., Italy, Type III polysulphone cage; 425 mm × 266 mm × 180 mm). The animals were kept on 4 cm thick layer of corn cob grit, dust-free bedding (Scobis Due - Mucedola, Italy), with a provision of one paper shelters (Lillico Biotechnology, UK). Animals were kept in the holding room with following environmental conditions:

temperature 22°C ± 2, relative humidity 55% ± 10, 15 - 20 air changes per hour, artificial light cycle of 12 hours light (7:00 to 19:00).

Animals had free access to potable water and food (SDS VRF 1 (P), UK). All animals were managed similarly and with due regard for their well-being according to prevailing practices and the experimental plan approved by CARE Zagreb (Committee for Animal Research Ethics).

Treatment/Experimental Design

Animals were assigned to the groups as in the table below:

| Route | Total No. mice | Feeding status | Dose (mg/kg) | Dose volume (mL/kg) | Samples |
|---|---|---|---|---|---|
| IV | 9 | fed | 10 | 5 | Plasma, at all t.p. Tissue sampling at 3 timepoints |
| PO | 3 | fed | 10 | 10 | Plasma |

Compound administration

Intravenous administration was performed into the lateral tail vein with a volume of administration of 5 mL/kg.

Oral administration was performed by oral gavage using a syringe and a blunt metal probe, with a volume of administration of 10 mL/kg.

Sampling

Following IV dosing, plasma samples and selected tissues - brain, lung, heart, kidney, liver - were collected as follows:

| Animal ID | 0.05 h | 025 h | 0.5 h | 1 h | 2 h | 4 h | 8 h | 24h |
|---|---|---|---|---|---|---|---|---|
| 1-3 | P | | P, T | | | | | |
| 4-6 | | P | | | P | P, T | | |
| 7-9 | P | | | P | | | P | P, T |
| P=blood sampling for plasma; T=terminal sampling for tissues | | | | | | | | |

Following PO dosing, plasma samples were collected by serial sampling from 3 animals as follows:

| Animal ID | 0.05 h | 025 h | 0.5 h | 1 h | 2 h | 4 h | 8 h | 24h |
|---|---|---|---|---|---|---|---|---|
| 1-3 | P | P | P | P | P | P | P | P |

Blood and plasma collection

Prior to blood sampling from the tail vein, mice were placed in a warming cabinet (up to 10 minutes, maximum at 38°C). Serial blood samples (ca. 100uL) were collected following successful puncture of the lateral tail vein using a needle, into K2EDTA-coated tubes.

For terminal sampling, before sacrifice, the mice were anesthetized with a cocktail of ketamine and xylazine administered ip. For each terminal time point, blood samples were collected via jugular vein bleeds; ca 0.4 mL of blood were collected and transferred into K2EDTA-coated tubes.

Within 30 minutes after blood collection, samples were centrifuged at 1560 g for 10 min at 4°C and the resulting plasma samples were aliquoted into polypropylene tubes (two aliquots of 20 μL) and stored at -20°C until analysis.

Tissue sampling

Following terminal blood sampling at 0.5, 4 and 24h, animals were euthanized by exsanguination, Tissues were

collected into Precellys® tubes, weighed individually, frozen and stored at -70° until analysis.

Bioanalysis

Analytical procedure

Plasma and tissue concentrations were determined by research qualified LC-MS/MS methods.

Samples were analyzed on a SCIEX triple quadrupole mass spectrometer operating in TurbolonSpray mode. Blank mouse plasma was used for preparation of blank samples, calibrators and quality control samples (QC samples). Tissue samples were homogenized by the addition of water. An aliquot of homogenate was diluted with blank plasma and quantified against plasma calibration curve.

The study samples were assayed by batch with a calibration curve (six calibration standard levels at least) including one level corresponding to the target LLOQ and the target ULOQ. A generic internal standard will be used.

Each batch included QC samples in duplicate at three concentration levels (one near the limit of quantification, one in the mid-range and one near the high end of the range).

Washing solvent and double blank samples was run to avoid any contamination of a sample by its preceding one.

Back-calculated concentration of calibration and QC samples provided the basis of accepting or rejecting the batch.

The calibration equation was computed by least-squares regression using the selected model, and the concentration of each calibration sample was calculated. If the back-calculated concentration of a calibration sample does not fall within $\pm$15% of the nominal concentration ($\pm$20% at the LLOQ), that sample was discarded, and the equation was recalculated. For the calibration and the batch to be valid, the coefficient of determination ($r^2$) must be higher than 0.98 and at least 75% of calibration samples have to remain included. Moreover, for the lowest and the highest levels, only one replicate out of two may be rejected.

To qualify the batch, at least 67% of QCs must be within $\pm$15% of their respective nominal, and $\geq$ 50% of QCs per level should be $\pm$15% of their nominal value.

Data analysis

Plasma and tissue concentrations

Plasma concentrations were expressed in ng/mL and tissue concentrations in ng/g. Individual and mean concentrations (n=3 individual values) with standard deviation (SD) and coefficients of variation (CV%, not shown).

Pharmacokinetic parameters

Pharmacokinetic analysis was performed using WinNonlin Phoenix® software (Certara, version 8.3) from animal plasma concentrations, non-compartmental analysis, and the target dose.

The following PK parameters were calculated:

- $C_o$ (ng/mL): Initial concentration
- $C_{max}$ (ng/mL): maximum observed concentration, occurring at $T_{max}$
- $C_{max}$ /Dose (ng/mL): dose normalised $C_{max}$
- $AUC_{0\text{-last}}$ (ng*h/mL): area under the plasma concentration versus time curve up to last quantifiable concentration, and/or up to infinity $AUC_{0\text{-inf}}$ (ng*h/mL) will be calculated according to the linear up log down method.
- t½ (h): Apparent terminal elimination half-life only reported if three or more time points in the elimination phase - excluding the $T_{max}$ - were used for linear regression, and if $r_{adj}^2$>0.9, and if % of AUC extrapolation to infinity is lower than 20%,
- CL (mL/min/kg): total clearance determined after intravenous administration
- $V_{ss}$ (L/kg): Apparent volume of distribution at equilibrium determined after intravenous administration ($V_{ss}$/F after oral dosing)
- F (%): Bioavailability

The table below shows the mean plasma concentrations of CPD-015 after intravenous (IV) administration.

| Time (h) | Mean concentration (ng/ mL) ± SD | N |
|---|---|---|
| 0.05 | 4862 ± 1011 | 6 |

(continued)

| Time (h) | Mean concentration (ng/ mL) ± SD | N |
|---|---|---|
| 0.25 | 2640 ± 668 | 3 |
| 0.5 | 1493 ± 176 | 3 |
| 1 | 1130 ± 43.6 | 3 |
| 2 | 1303 ± 124 | 3 |
| 4 | 470 ± 49.7 | 3 |
| 8 | 209 ± 34.1 | 3 |
| 24 | BLQ | 0 |
| SD = standard deviation of N; BLQ - blank, below the detection limits. | | |

The table below shows the tissue distribution of CPD-015 after intravenous (IV) administration. Data are expressed as the mean value of n=3 unless stated otherwise.

| **Time** | **Liver** (ng/g) | **Lung** (ng/g) | **Heart** (ng/g) | **Kidney** (ng/g) | **Brain** (ng/g) |
|---|---|---|---|---|---|
| 0.5 h | 171[a] | 248[b] | BLQ | 209 | BLQ |
| 4h | 6043 | 16500 | 3757 | 32300 | BLQ |
| 24h | 35767 | 795[b] | 874 | 78867 | 116[a] |
| BLQ - blank, below the detection limits. [a]n=1; [b]n=2 | | | | | |

The table below shows the mean plasma concentrations of CPD-015 after oral (PO) administration.

| Time (h) | Mean concentration (ng/ mL) ± SD | N |
|---|---|---|
| 0.25 | 38.7 ± 14.5 | 3 |
| 0.5 | 62.9 ± 32.1 | 3 |
| 1 | 128 ± 69.0 | 3 |
| 2 | 135 ± 42.1 | 3 |
| 4 | 97.1 ± 33.3 | 3 |
| 8 | 32.1 ± 9.31 | 3 |
| 24 | 9.86 | 2 |

The table below shows the PK parameters of CPD-015 after intravenous (IV) and oral (PO) administration.

| PK parameter | PO (10 mg/kg) | IV (10 mg/kg) |
|---|---|---|
| $t_{½}$ (h) | 5.7 | 0.27[#] 2.59* (predominant) |
| $T_{max}$ (h) | 2.0 | |
| $C_{max}$ (ng/mL) | 141 | |
| $C_0$ (ng/mL) | | 5663 |
| $AUC_{0\text{-}last}$ (hr*ng/mL)/(mg/kg) | 880 | 6284 |
| $AUC_{0\text{-}inf}$ (h r*ng/m L) | 1054 | 7064 |
| F (%) | 14 | |
| CL (mL/min/kg) | | 23.6 |

(continued)

| PK parameter | PO (10 mg/kg) | IV (10 mg/kg) |
|---|---|---|
| $V_{ss}$ (L/kg) | | 4.77 |

Data were expressed as the mean value (n=1-3); #αt1/2 (initial phase); *βt1/2 (terminal phase).

Summary

Following IV dosing at 10 mg/kg, CPD-015 is quantifiable in plasma up to 8h and in tissue samples up to 24h. CPD-015 is characterized by a low CL in plasma (26% LBF; assuming mouse LBF of 90 mL/min/kg) and a moderate volume of distribution (4.77 L/kg). βt1/2 (terminal phase) of 2.59 h was the predominant half-life with Rsq >0.93. αt1/2 (initial phase) was 0.27 h.

The highest concentration is measured in the kidney at 24 h (ca. 2-fold higher than liver and 100-fold than lung and heart tissue). Maximum lung concentration was measured at 4h. Following PO dosing at 10 mg/kg, maximum plasma levels of CPD-015 were reached between 1 and 2 hr post dose, with a half-life of 5.7 h (n=1) and mean oral bioavailability of 14%.

## Claims

**1.** A compound of formula (I) or a stereoisomer, or tautomer thereof,

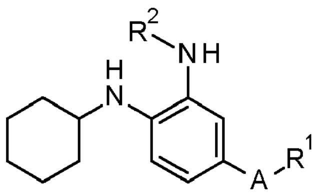

(I)

wherein,

A is selected from

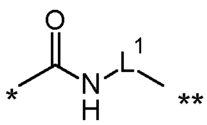

or

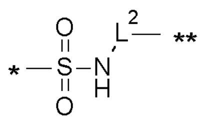

;

wherein * represents where moiety A is bound to the rest of the molecule; and ** represents where moiety A is bound to $R^1$;

$L^1$ is selected from $-(CR^3R^4)_{n}-$ or

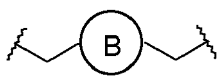

;

$L^2$ is selected from $-(CR^5R^6)_{m}-$ or

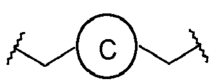

;

n is an integer selected from 1, 2, 3, 4, 5, 6, 7, 8, 9 or 10;

m is an integer selected from 1, 2, 3, 4, 5, 6, 7, 8, 9 or 10;

cycle B is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^B$;

cycle C is selected the group consisting of $C_{6-12}$aryl, $C_{3-10}$cycloalkyl, $C_{3-10}$cycloalkenyl, $C_{3-10}$cycloalkynyl, heterocyclyl, and heteroaryl, wherein said $C_{6-12}$aryl, $C_{3-10}$cycloalkyl, $C_{3-10}$cycloalkenyl, $C_{3-10}$cycloalkynyl, heterocyclyl or heteroaryl can be unsubstituted or substituted with one or more $Z^c$;

$R^1$ is selected from the group consisting of heterocyclyl, -$NR^8R^9$, halogen, halo$C_{1-6}$alkyl, halo$C_{1-6}$alkyloxy, $C_{3-10}$cycloalkyl, $C_{6-12}$aryl, $C_{6-12}$aryl$C_{1-6}$alkyl, heteroaryl, cyano, - $NR^8R^9S(O)_2R^{10}$, -$NR^8R^9C(O)_2R^{10}$, -$C(O)R^{10}$, -$C(O)_2R^{10}$, -$S(O)_2R^{10}$, wherein said heterocyclyl $C_{3-10}$cycloalkyl, $C_{6-12}$aryl, $C_{6-12}$aryl$C_{1-6}$alkyl, or heteroaryl can be unsubstituted or substituted with one or more $Z^1$;

$R^2$ is selected from the group consisting of $C_{3-10}$cycloalkyl, hydrogen, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;

$R^3$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^3$;

$R^4$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^4$;

$R^5$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or more $Z^5$;

$R^6$ is selected from the group consisting of hydrogen, halogen, hydroxyl, amino, $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl; wherein said $C_{1-6}$alkyl, $C_{3-10}$cycloalkyl, $C_{6-12}$aryl, $C_{6-12}$aryl$C_{1-6}$alkyl, heterocyclyl, heterocyclyl$C_{1-6}$alkyl, heteroaryl, and heteroaryl$C_{1-6}$alkyl can be unsubstituted or substituted with one or $Z^6$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^B$, $Z^C$, $Z^1$, $Z^2$, $Z^3$, $Z^5$, and $Z^6$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, -$OR^{10}$, cyano, amino, -$NR^8R^9$, -$C(O)_2R^{10}$, -$C(O)NR^8R^9$, -$C(O)R^{10}$, -$S(O)R^{10}$, -$S(O)_2R^{10}$, -$S(O)_2NR^8R^9$, nitro;

or a solvate, hydrate, pharmaceutically acceptable salt, or prodrug thereof;

wherein when moiety A is -$S(O)_2$-$(CH_2)_2$- then $R^2$ is not hydrogen, benzyl or pyridyl; wherein when moiety A is -$C(O)$-$(CH_2)_2$- then $R^2$ is not hydrogen or benzyl; with the proviso that said compound is not or

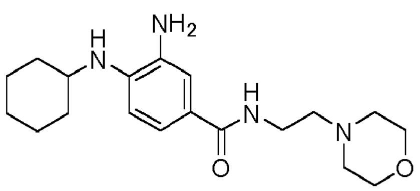

.

**2.** The compound according to claim 1, having structural formula (II), (II) or (IV),

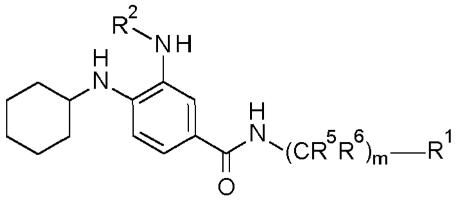

(II)

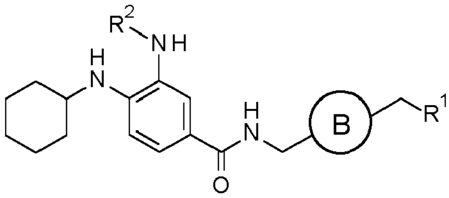

(III)

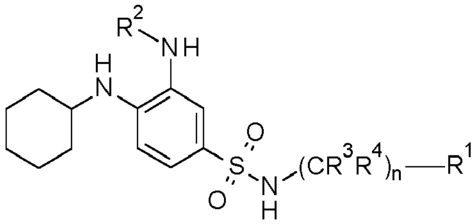

(IV)

wherein n, m, cycle B, $R^1$, $R^2$, $R^3$, $R^4$, $R^5$, $R^6$ have the same meaning as that defined in claim 1.

**3.** The compound according to claim 1 or 2, having structural formula (IA), (IB), (IC), or (ID)

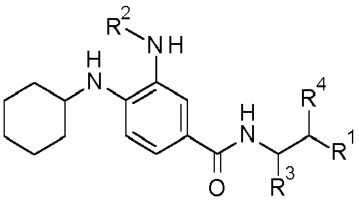

(IA)

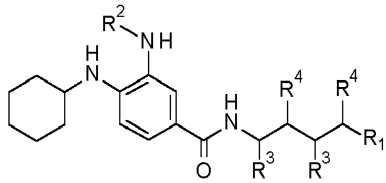

(IB)

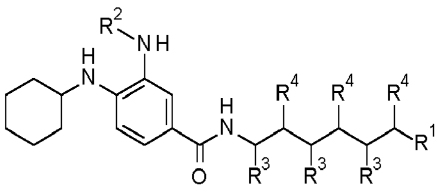

(IC)

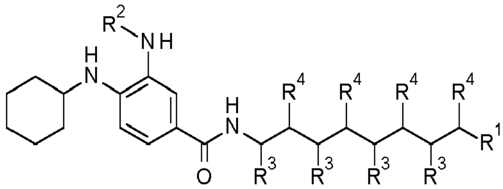

(ID),

wherein $R^1$, $R^2$, $R^3$ and $R^4$ have the same meaning as that defined in claim 1.

**4.** The compound according to any one of claims 1 to 3, wherein

cycle B is selected the group consisting of $C_{6-12}$aryl, $C_{3-10}$cycloalkyl, heterocyclyl, and heteroaryl, wherein said $C_{6-12}$aryl, $C_{3-10}$cycloalkyl, heterocyclyl or heteroaryl can be unsubstituted or substituted with one or more $Z^B$;
cycle C is selected the group consisting of $C_{6-12}$aryl, $C_{3-10}$cycloalkyl, heterocyclyl, and heteroaryl, wherein said $C_{6-12}$aryl, $C_{3-10}$cycloalkyl, heterocyclyl or heteroaryl can be unsubstituted or substituted with one or more $Z^C$;
$R^1$ is selected from the group consisting of heterocyclyl, $-NR^8R^9$, $C_{3-10}$cycloalkyl, $C_{6-12}$aryl, $C_{6-12}$aryl$C_{1-6}$alkyl, heteroaryl, cyano, $-NR^8R^9S(O)_2R^{10}$, $-NR^8R^9C(O)_2R^{10}$, wherein said heterocyclyl $C_{3-10}$cycloalkyl, $C_{6-12}$aryl,

$C_{6-12}$aryl$C_{1-6}$alkyl, or heteroaryl can be unsubstituted or substituted with one or more $Z^1$;
$R^2$ is selected from the group consisting of $C_{3-10}$cycloalkyl, hydrogen, $C_{6-12}$aryl$C_{1-6}$alkyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;
$R^3$ is selected from the group consisting of hydrogen, halogen, hydroxyl, amino, $C_{1-6}$alkyl; wherein said $C_{1-6}$alkyl can be unsubstituted or substituted with one or more $Z^3$;
$R^4$ is selected from the group consisting of hydrogen, halogen, hydroxyl, amino, $C_{1-6}$alkyl; wherein said $C_{1-6}$alkyl can be unsubstituted or substituted with one or more $Z^4$;
$R^5$ is selected from the group consisting of hydrogen, halogen, hydroxyl, amino, $C_{1-6}$alkyl; wherein said $C_{1-6}$alkyl can be unsubstituted or substituted with one or more $Z^5$;
$R^6$ is selected from the group consisting of hydrogen, halogen, hydroxyl, amino, $C_{1-6}$alkyl; wherein said $C_{1-6}$alkyl can be unsubstituted or substituted with one or more $Z^6$.

**5.** A pharmaceutical composition comprising a compound of formula (I) according to any one of claims 1 to 4 and a pharmaceutically acceptable carrier.

**6.** A compound of formula (I) according to any one of claims 1 to 4, or a pharmaceutical composition according to claim 5, for use as a medicament.

**7.** A compound, or its stereoisomer, or tautomer, or solvate, hydrate, pharmaceutically acceptable salt, or prodrug thereof, wherein said compound is

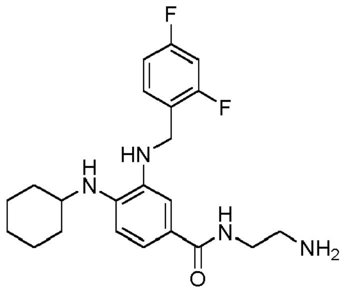

,

for use as a medicament for parenteral or oral, preferably parenteral administration.

**8.** A compound, or its stereoisomer, or tautomer, or solvate, hydrate, pharmaceutically acceptable salt, or prodrug thereof, wherein said compound is

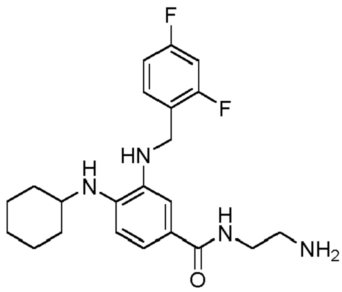

,

for use as a medicament for parenteral administration in the prevention or treatment of a disease associated with ferroptosis and/or oxytosis.

**9.** A compound of formula (I) according to any one of claims 1 to 4, or a pharmaceutical composition according to claim 5, for use in the prevention or treatment of a disease associated with ferroptosis and/or oxytosis.

**10.** The compound for use according to claim 8 or 9, wherein the disease associated with ferroptosis and/or oxytosis is selected from the group consisting of liver disease, chronic kidney disease, lung diseases, ocular surface diseases,

wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4, preferably a genetic disorder which is a Sedaghatian-type spondylometaphyseal dysplasia.

**11.** The compound for use according to claim 10, wherein the liver disease is selected from hemochromatosis, primary biliary cholangitis, non-alcoholic steatohepatitis or liver fibrosis.

**12.** The compound for use according to claim 10, wherein the neurological disease is selected from Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

**13.** The compound for use according to claim 10, wherein the ischemia-reperfusion injury is selected from myocardial ischemia-reperfusion injury, liver ischemia-reperfusion injury, or renal ischemia-reperfusion injury, intestinal ischemia-reperfusion injury, cerebral ischemia-reperfusion injury, or lung ischemia-reperfusion injury.

**14.** The compound for use according to claim 10, wherein the iron metabolism-related disease is selected from atherosclerosis or diabetes.

**15.** The compound for use according to claim 10, wherein the lung disease is selected from acute respiratory distress syndrome (ARDS), lung diseases caused by infections such as COVID-19 pulmonary disease, mucoviscidosis, or asthma.

Europäisches Patentamt
European Patent Office
Office européen des brevets

# EUROPEAN SEARCH REPORT

Application Number
EP 24 18 7620

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DEVISSCHER ET AL: "Discovery of Novel, Drug-Like Ferroptosis Inhibitors with in Vivo Efficacy", JOURNAL OF MEDICINAL CHEMISTRY, AMERICAN CHEMICAL SOCIETY, US, vol. 61, no. 22, 25 October 2018 (2018-10-25), pages 10126-10140, XP002790074, ISSN: 0022-2623, DOI: 10.1021/ACS.JMEDCHEM.8B01299 * examples * ----- | 1-15 | INV. C07C211/02 C07C211/03 C07D295/125 C07D295/135 A61P9/10 A61P11/00 A61K31/495 |
| X | GASCHLER MICHAEL M. ET AL: "Determination of the Subcellular Localization and Mechanism of Action of Ferrostatins in Suppressing Ferroptosis", ACS CHEMICAL BIOLOGY, vol. 13, no. 4, 7 March 2018 (2018-03-07), pages 1013-1020, XP055929023, ISSN: 1554-8929, DOI: 10.1021/acschembio.8b00199 * example 3 * ----- | 1-15 | TECHNICAL FIELDS SEARCHED (IPC) |
| X | WO 2019/154795 A1 (VIB VZW [BE]; UNIV ANTWERPEN [BE]; UNIV GENT [BE]) 15 August 2019 (2019-08-15) * examples 27-29 * ----- | 1-15 | C07C C07D A61P A61K |
| X | WO 2016/075330 A1 (VIB VZW [BE]; UNIV GENT [BE]; UNIV ANTWERPEN [BE]) 19 May 2016 (2016-05-19) * examples * ----- -/-- | 1-15 | |

The present search report has been drawn up for all claims

2

| Place of search | Date of completion of the search | Examiner |
|---|---|---|
| Munich | 16 January 2025 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 18 7620

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAM HOFMANS ET AL: "Novel Ferroptosis Inhibitors with Improved Potency and ADME Properties", JOURNAL OF MEDICINAL CHEMISTRY, vol. 59, no. 5, 8 February 2016 (2016-02-08), pages 2041-2053, XP055485611, US ISSN: 0022-2623, DOI: 10.1021/acs.jmedchem.5b01641 * figure 3 * ----- | 1-15 | |
| X | WO 2013/152039 A1 (UNIV COLUMBIA [US]; STOCKWELL BRENT R [US] ET AL.) 10 October 2013 (2013-10-10) * page 27 * ----- | 1 | |

TECHNICAL FIELDS SEARCHED (IPC)

The present search report has been drawn up for all claims

2

| Place of search | Date of completion of the search | Examiner |
|---|---|---|
| Munich | 16 January 2025 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 24 18 7620

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | Publication date | Patent family member(s) | Publication date |
|---|---|---|---|
| WO 2019154795 A1 | 15-08-2019 | CN 112105601 A | 18-12-2020 |
| | | EP 3749645 A1 | 16-12-2020 |
| | | US 2021094909 A1 | 01-04-2021 |
| | | WO 2019154795 A1 | 15-08-2019 |
| WO 2016075330 A1 | 19-05-2016 | EP 3218357 A1 | 20-09-2017 |
| | | US 2017313653 A1 | 02-11-2017 |
| | | WO 2016075330 A1 | 19-05-2016 |
| WO 2013152039 A1 | 10-10-2013 | US 2015079035 A1 | 19-03-2015 |
| | | US 2017233370 A1 | 17-08-2017 |
| | | US 2019135782 A1 | 09-05-2019 |
| | | WO 2013152039 A1 | 10-10-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013152039 A
- WO 9933795 A
- WO 9933815 A
- WO 9933793 A
- WO 9933792 A
- WO 2020113028 A1


### Non-patent literature cited in the description


- **SKOUTA R. et al.** *J. Am. Chem. Soc.*, 2014, vol. 136, 4551-4556
- **L. ELIEL** ; **S. H. WILEN** ; **L. N. MANDER**. Stereochemistry of Organic Compounds. Wiley- Interscience, 1994
- Biotransformation of Drugs. **GOODMAN** ; **GILMAN**. The Pharmacological Basis of Therapeutics. McGraw-Hill, Int. Ed., 1992, 13-15
- **DIXON et al.** *Cell*, 2012, vol. 149, 1060-1072
- **HENKE N. et al.** *Cell Death and Disease*, 2013, vol. 4, e470
- **LOUANDRE C. et al.** *Int. J. Cancer*, 2013, vol. 133, 1732
- **VAN COILLIE et al.** *Nat Comm*, 2022, vol. 13, 1046
- **STAHL** ; **WERMUTH**. Handbook of Pharmaceutical Salts: Properties, Selection, and Use. Wiley-VCH, 2002
- **K. R. MORRIS**. Polymorphism in Pharmaceutical Solids. Marcel Dekker, 1995
- **O. ALMARSSON** ; **M. J. ZAWOROTKO**. *Chem Commun*, 2004, vol. 17, 1889-1896
- **HALEBLIAN**. *J Pharm Sci*, August 1975, vol. 64 (8), 1269-1288
- **N. H. HARTSHORNE** ; **A. STUART**. Crystals and the Polarizing Microscope. 1970
- **SHAH et al.** *Cell Chem. Biol.*, 2019, vol. 26, 1594-1607